# EUROPEAN PATENT SPECIFICATION

(11) **EP 2 125 727 B1**
(45) Date of publication and mention of the grant of the patent: **21.11.2012**
(21) Application number: 07858162.6
(22) Date of filing: 27.12.2007
(51) Int. Cl.: C07D 211/54, C07D 401/06, A61K 31/44

(54) **STABLE S-NITROSOTHIOLS, METHOD OF SYNTHESIS AND USE**
STABILE S-NITROSOTHIOLE, SYNTHESEVERFAHREN UND VERWENDUNG
S-NITROSOTHIOLS STABLES, PROCÉDÉ DE SYNTHÈSE ET UTILISATION

(30) Priority: 28.12.2006 EP 06380340
(43) Date of publication of application: 02.12.2009
(73) Proprietor: LACER, S.A., 08025 Barcelona (ES)
(72) Inventor: REPOLLÉS MOLINER, José, E-08025 Barcelona (ES); PUBILL COY, Francisco, E-08025 Barcelona (ES); MOURELLE MANCINI, Marisabel, E-08025 Barcelona (ES); DEL CASTILLO NIETO, Juan Carlos, E-08025 Barcelona (ES); CABEZA LLORENTE, Lydia, E-08025 Barcelona (ES); MARTÍNEZ BONÍN, Juan, E-08025 Barcelona (ES); MODOLELL SALADRIGAS, Ana, E-08025 Barcelona (ES)
(74) Representative: ABG Patentes, S.L.
(86) International application number: PCT/EP2007/064565
(87) International publication number: WO 2008/080934

(56) References cited:
- EP-A- 1 157 987
- WO-A-03/086282

## Description

### FIELD OF THE INVENTION

The present invention relates to stable S-nitrosothiols derivatives useful for the preparation of medicaments for treatment of NO related diseases, and synthesis thereof.

### BACKGROUND OF THE INVENTION

It is well known that compounds capable of releasing nitrogen oxide (NO) into the organism exhibit in many cases some type of activity of the vascular system, for example vasodilating activity or inhibition of the aggregation of the platelets, which make them potentially useful for the treatment of different disorders related to dysfunctions of the circulatory system.

Further, it is described too that specific derivatives which contain an S-nitrosothiol group have, from a medical point of view, advantageous characteristics due to the fact that they are capable of releasing NO in the organism.

Radomski et al., Br. J. Pharmacol. (1992) 107, 745-749, describes that an S-nitrosoglutathione (GSNO) compound is capable of inhibiting the activity of the platelets.

Golino et al, Circulation Research, 71, No 6 (1992), describes that S-nitrosocysteine is capable of inhibiting the activity of the platelets, due to an anti-thrombosis effect.

Smith et al., Met. Find. Exp. Cline. Pharmacy. (1994), 16, 5, describes that the GSNO produces a strong relaxing effect of the arterioles.

WO95/12394 describes the use of S-nitroso adducts of peptides, among others the S-nitroso-N-acetylpenicillamine (SNAP), as protecting agents against vascular inflammation of traumatic origin.

WO95/07691 describes the use of different S-nitrosothiols, in particular the GSNO, in the treatment and prevention of the action of the platelets and the formation of thrombosis on damaged vascular surface.

WO03/086282 A2 describes a broad range of cyclic and heterocyclic organic nitro- and nitroso-compounds as nitric oxide donors.

WO93/09806 describes S-nitrosated proteins or amino acid residues, capable of releasing NO, which have a relaxing effect on the musculature and an inhibitory effect on platelet aggregation.

The S-nitrosothiol group is an unstable functional group and therefore the administration of active ingredients comprising the same posses many problems. The *in vivo* decomposition of the S-nitrosothiol functionality reduces the efficacy of some medicaments which show excellent *in vitro* activity. A further problem derived from the low stability of S-nitroso compounds is their short shelf stability.

EP-B1-1157987, in the name of the applicant, describes S-nitrosothiols derivatives of penicillamine or glutathione, which both have a potent vasodilating effect and a high inhibitory effect on the aggregation of the platelets of the formula 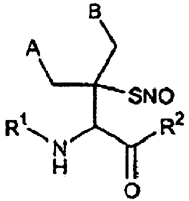 wherein A and B are phenyl groups or together form the rest -CH₂-Q-CH₂- constituting a ring of six units in which Q represents an atom of oxygen, of sulfur, or a group N-R₃, in which R₃ is hydrogen or an alkyl group C₁-C₄; R₁ is an acyl rest, which may be an aliphatic acyl group C₁-C₅ or a rest of glutamic acid bound via its non amino acid carboxyl; R₂ is a hydroxyl group or a glycine residue bound via a peptide bond. Although they show high efficacy, their usefulness is reduced due to the poor stability of the S-nitrosothiol functionality.

EP-B1-412699 describes S-nitrosothiols which correspond to following general formula: 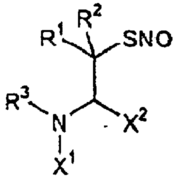 and its use as therapeutic agents against cardiovascular diseases, in particular as anti-hypertension (increased blood pressure) and as agents for the treatment of angina pectoris. According to EP-B1-412699 the S-nitrosothiol functional group may be stabilized by bulky R₁ and R₂ groups.

Thus, there is a need for providing stable S-nitrosothiol derivatives.

### SUMMARY OF THE INVENTION

The inventors have know surprisingly found that the provision of compounds similar to those described in EP-B1-1157987 but having an ester group instead of an acid or a glycine amide, provides stable S-nitrosothiol derivatives.

Thus, a first aspect of the present invention relates to S-nitrosothiol derivatives of formula (I) (compounds of the invention) as described below.

A second aspect of the invention relates to the synthesis of said S-nitrosothiol derivatives by nitrosation of the thiols of formula (III) as described below.

A further aspect of the invention is directed to the thiols of formula (III), and a method for their synthesis.

A further aspect of the invention relates to compounds of formula (IV), which are precursors of the thiols of formula (III) and therefore key intermediates in the synthesis of the S-nitrosothiols of formula (I).

A further aspect of the invention is directed to the use of the S-nitrosothiols of formula (I) for the preparation of medicament for the treatment and/or prophylaxis of a NO mediated condition.

A further aspect is directed to a pharmaceutical composition comprising a S-nitrosothiol of formula (I) and a pharmaceutically acceptable carrier.

### DETAILED DESCRIPTION OF THE INVENTION

### Compounds of formula (I)

A first aspect of the present invention relates to S-nitrosothiol derivatives of formula (I) 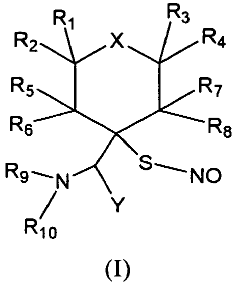 wherein
each of R₁, R₂, R₃, R₄, R₅, R₆, R₇ and R₈ are hydrogen;
R₉ is selected from the group consisting of hydrogen, C₁-C₆ alkyl and C₇-C₁₅ aralkyl;
R₁₀ is selected from the group consisting of hydrogen, C₁-C₆ alkyl, C₃-C₈ cycloalkyl, -C(=O)R₁₁ and -S(=O)₂R₁₁, wherein R₁₁ is selected from the group consisting of hydrogen, C₁-C₆ alkyl, C₂-C₆ alkenyl, C₃-C₈ cycloalkyl, C₂-C₆ alkynyl, C₆-C₁₀ aryl, C₇-C₁₀ aralkyl and 5- or 6-membered heterocyclyl;
Y is an alkoxycarbonyl of formula -C(=O)OR₁₂, wherein R₁₂ is selected from the group consisting of C₁-C₆ alkyl, C₂-C₆ alkenyl, C₃-C₈ cycloalkyl, C₂-C₆ alkynyl, C₆-C₁₀ aryl, C₇-C₁₀ aralkyl and 5- or 6-membered heterocyclyl; and X is selected from the group consisting of oxygen atom, sulphur atom, and - NR₁₃; wherein R₁₃ is selected from the group consisting of hydrogen, C₁-C₆ alkyl, C₂-C₆ alkenyl, C₃-C₈ cycloalkyl, C₂-C₆ alkynyl, C₆-C₁₀ aryl, C₇-C₁₀ aralkyl and 5- or 6-membered heterocyclyl;
or its diastereoisomers, enantiomers, salts or solvates thereof.

The invention also provides salts of compounds of the invention. For instance, pharmaceutically acceptable salts of compounds provided herein may be acid addition salts, base addition salts or metallic salts, and they can be synthesized from the parent compound which contains a basic or an acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, bisulphate, nitrate, phosphate, diacid phosphate and organic acid addition salts such as, for example, acetate, maleate, fumarate, citrate, lactate, salicilate, oxalate, succinate, tartrate, malate, mandelate, methanesulphonate (also referred to as mesilate) and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

It will be readily apparent to those skilled in the art that the scope of the present invention also encompasses salts which are not pharmaceutically acceptable as possible means for obtaining pharmaceutically acceptable salts.

The term "solvate" according to this invention is to be understood as meaning any form of the active compound according to the invention which has another molecule (most likely a polar solvent) attached to it via non-covalent bonding. Examples of solvates include hydrates and alcoholates, preferably C₁-C₆ alcoholates, e.g. methanolate.

It will be immediately apparent to the skilled person that the present invention encompasses all possible stereoisomers of the compounds described herein. An stereoisomer is understood by the skilled person as compounds made up of the same atoms bonded by the same sequence of bonds but having different three-dimensional structures which are not interchangeable, e.g. diastereoisomers or enantiomers. Separation methods of the different diastereoisomers or enantiomers by chemical and/or physical means are readily available to the skilled person. Examples of such methods are provided (see example 33). For example, an enantiomerically enriched mixture of enantiomers may be obtained at any stage of the synthesis by purification under chiral conditions, such as chiral supercritical fluid chromatography. Following the same methods pure enantiomers may also be isolated.

For the purposes of the present invention, "**enantiomerically enriched**" applied to a mixture of enantiomers refers to a mixture of enantiomers of a compound in which one of them is present in greater amounts than the other enantiomer. Therefore, enantiomerically enriched mixtures have an enantiomeric excess above 0% with regard to one of its enantiomers, preferably above 20%, preferably above 40%, preferably above 70%, more preferably above 80%, more preferably above 90% and more preferably above 95%.

An "**enantiomerically pure**" compound can be considered as a mixture of two enantiomers having enantiomeric excess above 95%, preferably above 98%, more preferably above 99%, more preferably above 99.5%.

According to a preferred embodiment, X is -NR₁₃, wherein R₁₃ is defined as above.

According to a preferred embodiment, R₉ is selected from the group consisting of hydrogen and C₁-C₆ alkyl.

According to a preferred embodiment, R₁₂ is a C₁-C₆ alkyl group.

According to a preferred embodiment, one of R₉ or R₁₀ is hydrogen.

According to a preferred embodiment, the compounds of the invention are selected from the group consisting of:
- Ethyl (R,S)-acetylamino-(1-methyl-4-S-nitrosomercapto-piperidin-4-yl)-acetate;
- (R,S)-4-(Acetylamino-ethoxycarbonyl-methyl)-1-methyl-4-S-nitrosomercaptopiperidinium chloride;
- Ethyl (R,S)-heptanoylamino-(1-methyl-4-S-nitrosomercapto-piperidin-4-yl)-acetate;
- (R,S)-4-(Heptanoylamino-ethoxycarbonyl-methyl)-1-methyl-4-S-nitrosomercapto-piperidinium chloride;
- Ethyl (R,S)-benzoylamino-(1-methyl-4-S-nitrosomercapto-piperidin-4-yl)-acetate;
- (R,S)-4-(Benzoylamino-ethoxycarbonyl-methyl)-l-methyl-4-S-nitrosomercaptopiperidinium chloride;
- Ethyl (R,S)-(4-chloro-benzoylamino)-(1-methyl-4-S-nitrosomercapto-piperidin-4-yl)-acetate;
- (R,S)-4-[(4-Chloro-benzoylamino)-ethoxycarbonyl-methyl)]-1-methyl-4-S-nitrosomercapto-piperidinium chloride;
- Ethyl (R,S)-(2-chloro-benzoylamino)-(1-methyl-4-S-nitrosomercapto-piperidin-4-yl)-acetate;
- (R,S)-4-[(2-Chloro-benzoylamino)-ethoxycarbonyl-methyl)]-1-methyl-4-S-nitrosomercapto-piperidinium chloride;
- Ethyl (R,S)-(1-methyl-4-S-nitrosomercapto-piperidin-4-yl)-[(pyridin-3-carbonyl)-amino]-acetate;
- (R,S)-4-{Ethoxycarbonyl-[(pyridin-3-carbonyl)-amino]-methyl}-1-methyl-4-S-nitrosomercapto-piperidinium chloride;
- Ethyl (R,S)-benzenesulfonylamino-(1-methyl-4-S-nitrosomercapto-piperidin-4-yl)-acetate;
- (R,S)-4-(Benzenesulfonylamino-ethoxycarbonyl-methyl)-1-methyl-4-S-nitrosomercapto-piperidinium chloride;
- (R,S)-4-(benzenesulfonylamino-ethoxycarbonyl-methyl)-l-methyl-4-S-nitrosomercapto-piperidinium acetate;
- (R,S)-4-(benzenesulfonylamino-ethoxycarbonyl-methyl)-1-methyl-4-S-nitrosomercapto-piperidinium mesylate;
- (R,S)-4-(benzenesulfonylamino-ethoxycarbonyl-methyl)-1-methyl-4-S-nitrosomercapto-piperidinium nitrate;
- (R,S)-4-(benzenesulfonylamino-ethoxycarbonyl-methyl)-1-methyl-4-S-nitrosomercapto-piperidinium sulfate;
- (R,S)-4-(benzenesulfonylamino-ethoxycarbonyl-methyl)-1-methyl-4-S-nitrosomercapto-piperidinium bisulfate;
- (R,S)-4-(benzenesulfonylamino-ethoxycarbonyl-methyl)-1-methyl-4-S-nitrosomercapto-piperidinium phosphate diacid;
- (R,S)-4-(benzenesulfonylamino-ethoxycarbonyl-methyl)-1-methyl-4-S-nitrosomercapto-piperidinium lactate;
- (R,S)-4-(benzenesulfonylamino-ethoxycarbonyl-methyl)-l-methyl-4-S-nitrosomercapto-piperidinium citrate;
- (R,S)-4-(benzenesulfonylamino-ethoxycarbonyl-methyl)-1-methyl-4-S-nitrosomercapto-piperidinium salicylate;
- Ethyl (R,S)-(1-methyl-4-S-nitrosomercapto-piperidin-4-yl)-(4-nitro-benzene sulfonylamino)-acetate;
- Ethyl (R,S)-(4-methyl-benzenesulfonylamino)-(1-methyl-4-S-nitrosomercaptopiperidin-4-yl)-acetate;
- Ethyl (R,S)-(2-chloro-benzenesulfonylamino)-(1-methyl-4-S-nitrosomercaptopiperidin-4-yl)-acetate;
- Ethyl (R,S)-(3-chloro-benzenesulfonylamino)-(1-methyl-4-S-nitrosomercaptopiperidin-4-yl)-acetate;
- Ethyl (R,S)-(4-chloro-benzenesulfonylamino)-(1-methyl-4-S-nitrosomercaptopiperidin-4-yl)-acetate;
- (R,S)-4-(4-Chloro-benzenesulfonylamino-ethoxycarbonyl-methyl)-1-methyl-4-S-nitrosomercapto-piperidinium chloride;
- (R,S)-4-(2-Fluoro-benzenesulfonylamino-ethoxycarbonyl-methyl)-1-methyl-4-S-nitrosomercapto-piperidinium chloride;
- Ethyl (R,S)-(4-fluoro-benzenesulfonylamino)-(1-methyl-4-S-nitrosomercaptopiperidin-4-yl)-acetate;
- (R,S)- 4-(4-Fluoro-benzenesulfonylamino-ethoxycarbonyl-methyl)-1-methyl-4-S-nitrosomercapto-piperidinium chloride;
- Ethyl (R,S)-(2,4-difluoro-benzenesulfonylamino)-(1-methyl-4-S-nitroso mercapto-piperidin-4-yl)-acetate;
- (R,S)-4-(2,4-Difluoro-benzenesulfonylamino-ethoxycarbonyl-methyl)-1-methyl-4-S-nitrosomercapto-piperidinium chloride;
- Ethyl (RS)-formylamino-(1-methyl-4-S-nitrosomercapto-piperidin-4-yl)-acetate;
- (R,S)-4-(Ethoxycarbonyl-formylamino-methyl)-1-methyl-4-S-nitrosomercaptopiperidinium chloride;
- Ethyl (R,S)-(1-methyl-4-S-nitrosomercapto-piperidin-4-yl)-(2,2,2-trifluoroacetyl amino)-acetate;
- Benzyl (R,S)-acetylamino-(1-methyl-4-S-nitrosomercapto-piperidin-4-yl)-acetate;
- Ethyl (R,S)-(acetyl-ethyl-amino)-(1-methyl-4-S-nitrosomercapto-piperidin-4-yl)-acetate;
- Ethyl (R,S)-acetylamino-(1-ethyl-4-S-nitrosomercapto-piperidin-4-yl)-acetate;
- (R,S)-4-(Acetylamino-ethyloxycarbonyl-methyl)-1-ethyl-4-S-nitrosomercaptopiperidinium chloride;
- Ethyl (R,S)-(1-ethyl-4-S-nitrosomercapto-piperidin-4-yl)-[(pyridin-3-carbonyl)-amino]-acetate;
- (R,S)-1-Ethyl-4-{ethoxycarbonyl-[(pyridin-3-carbonyl)-amino]-methyl}-4-S-nitrosomercapto-piperidinium chloride;
- Ethyl (R,S)-acetylamino-(1-benzyl-4-S-nitrosomercapto-piperidin-4-yl)-acetate;
- (R,S)-4-(Acetylamino-ethyloxycarbonyl-methyl)-1-benzyl-4-S-nitrosomercaptopiperidinium chloride;
- Ethyl (R,S)-(1-benzyl-4-S-nitrosomercapto-piperidin-4-yl)-[(pyridin-3-carbonyl)-amino]-acetate;
- (R,S)-1-Benzyl-4-{ethoxycarbonyl-[(pyridin-3-carbonyl)-amino]-methyl}-4-S-nitrosomercapto-piperidinium chloride;
- Ethyl (R,S)-(1-ethyl-4-S-nitrosomercapto-piperidin-4-yl)-formylamino-acetate;
- (R,S)-1-Ethyl-4-(ethyloxycarbonyl-formylamino-methyl)-4-S-nitrosomercaptopiperidinium chloride;
- Ethyl (R,S)-(1-benzyl-4-S-nitrosomercapto-piperidin-4-yl)-formylamino-acetate;
- (R,S)-,-Benzyl-4-(ethyloxycarbonyl-formylamino-methyl)- 4-S-nitrosomercapto-piperidinium chloride;
- Methyl (R,S)-acetylamino-(1-methyl-4-S-nitrosomercapto-piperidin-4-yl)-acetate;
- (R,S)-4-(Acetylamino-methyloxycarbonyl-methyl)-1-methyl-4-S-nitrosomercapto-piperidinium chloride;
- Methyl (R,S)-(1-methyl-4-S-nitrosomercapto-piperidin-4-yl)-[(pyridin-3-carbonyl)-amino]-acetate;
- Methyl (R,S)-benzenesulfonylamino-(1-methyl-4-S-nitrosomercapto-piperidin-4-yl)-acetate;
- (R,S)-4-(Benzenesulfonylamino-methoxycarbonyl-methyl)-1-methyl-4-S-nitrosomercapto-piperidinium chloride;
- Methyl (R,S)-formylamino-(1-methyl-4-S-nitrosomercapto-piperidin-4-yl)-acetate;
- Ethyl (R,S)-acetylamino-(4-nitrosomercapto-tetrahydro-thiopyran-4-yl)-acetate; and
- Ethyl (R,S)-benzenesulfonylamino-(4-nitrosomercapto-tetrahydro-thiopyran-4-yl)-acetate;
or enantiomers or solvates thereof.

According to a preferred embodiment, the compounds of the invention are selected from the group consisting of:
- Ethyl-(R)-benzenesulfonylamino-(1-methyl-4-S-nitroso mercapto-piperidin-4-yl)-acetate;
- Ethyl-(S)-benzenesulfonylamino-(1-methyl-4-S-nitrosomercapto-piperidin-4-yl)-acetate;
- (S)-4-(Benzenesulfonylamino-ethoxycarbonyl-methyl)-1-methyl-4-S-nitrosomercapto-piperidinium chloride; and
- (R)-4-(Benzenesulfonylamino-ethoxycarbonyl-methyl)-1-methyl-4-S-nitrosomercapto-piperidinium chloride.

### Synthesis of the compounds of formula (I) and intermediates of the same

A second aspect of the invention is directed to a method for the synthesis of a compound of the invention comprising nitrosation of the thiol functionality of a compound of formula (III) 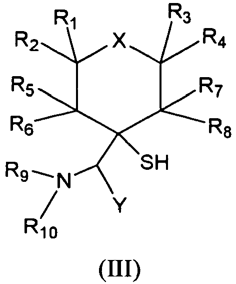 wherein
R₁, R₂, R₃, R₄, R₅, R_{b}, R₇, R₈, R₉, R₁₀, Y and X are as defined in formula (I).

Nitrosation is achieved with reagents known to those skilled in the art and which may be found in text books such as "Advanced Organic Chemistry, Reactions, Mechanism, and Structure" 5th Edition, Wiley-Interscience (see pages 699,779-780, 818 and references cited therein).

According to preferred embodiment, nitrosation comprises contacting said compound of formula (III) with a reagent selected from the group consisting of nitrous acid, alkylnitrites, NO gas, NOCI, NOBr, N₂O₃, N₂O₄ an BrCH₂NO₂.

For example, nitrosation agents may be obtained by placing sodium nitrite in aqueous or hydroalcoholic acid media, i.e. hydrochloric media, or by placing an alkylnitrite (i.e. tert-butyl or amyl nitrite) in neutral media, such as any suitable organic solvent, i.e. alcohols (ethanol, methanol,...), acetone, dichloromethane or tetrahydrofurane.

A further aspect of the invention is directed to said compounds of formula (III), or its diastereoisomers, enantiomers, salts or solvates thereof.

Compounds of formula (III) may be obtained by removal of the Z group from a compound of formula (IV) 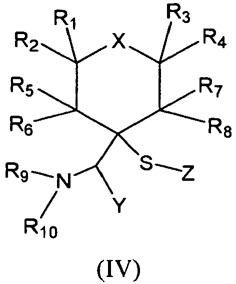 wherein
R₁, R₂, R₃, R₄, R₅, R₆, R₇, R₈, R₉, R₁₀, Y and X are as defined above for formula (I), and Z is selected from the group consisting of unsubstituted alkyl, alkenyl, C₇-C₂₀ aralkyl, -Si(R')₃, -C(=O)R', wherein R' is selected from the group consisting of alkyl, alkenyl, alkynyl, aryl and aralkyl.

In US 6,225,311 (Page 109) and WO 2000044709 A2 (page 293) the following compounds have been described wherein the Z group is 2-hidroxylethyl (substituted alkyl):

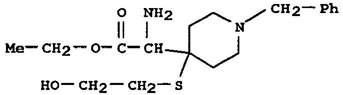

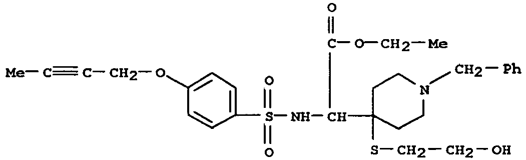

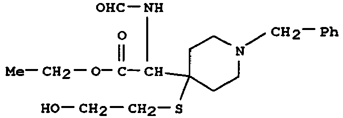

Z is usually selected so as to be easily cleaved at this stage of the synthesis. For example, thioethers, such as methyl thioether, tert-butyl thioether, benzyl thioether, p-methoxybenzyl thioether, 3,4-dimethoxybenzyl thioether, trityl thioether; allyl thioether; silyl derivatives of formula -Si(R')₃ such as trimethylsilyl (also represented as "TMS"), triethylsilyl, tert-butyldimethylsilyl (also represented as "TBDMSO"), tert-butyldiphenylsilyl, tri-isopropylsilyl, diethylisopropylsilyl, thexyldimethylsilyl ether, triphenylsilyl, di-tert-butylmethylsilyl; or thioesters such as acetate thioester, benzoate thioester; pivalate thioester; methoxyacetate thioester; chloroacetate thioester; levulinate thioester. Further conditions under which said groups may be removed, can be found in T.W. Greene and P.G.M. Wuts en "Protective Groups in Organic Synthesis", 3a Edicción, Wiley-Interscience, pages 454-493.

According to a preferred embodiment, Z is a C₇-C₂₀ aralkyl group (e.g. p-methoxybencyl) which may be removed under standard conditions (see T.W. Greene et al.), such as acidic conditions (e.g. trifluoroactic acid) or hydrogenolysis.

Thus, according to a preferred embodiment, the method for the synthesis of the compounds of the invention comprises:
a) the removal of the Z group from a compound of formula (IV) as defined above in order to obtain the compound of formula (III); and
b) the nitrosation of the thiol functionality of said compound of formula (III) obtained in step a).

Therefore, a further aspect of the invention is a method for the synthesis of said compound of formula (III) which comprises the removal of the Z group from a compound of formula (IV).

A further aspect of the invention is directed to the compounds of formula (IV) as described above, or its diastereoisomers, enantiomers, salts or solvates thereof.

The compounds of formula (IV) may be obtained by the addition of a compound of formula HS-Z, wherein Z is as defined above, in the presence of a base, to a compound of formula (V) 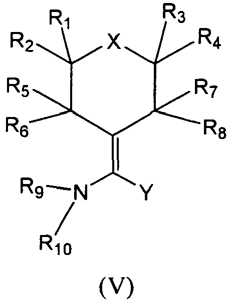 wherein
R₁, R₂, R₃, R₄, R₅, R₆, R₇, R₈, R₉, R₁₀, Y and X are as defined in formula (I), or its diastereoisomers, enantiomers, salts or solvates thereof

Conditions for this kind of reactions can be found in Nelson C.F. Yim et al., J. Org. Chem., 1988, 53, 4605-7 and C. Freeman Stanfield et al., Synthetic Communications, 1988, 18(5), 531-43.

Thus, according to a preferred embodiment, the method for the synthesis of the compound of formula (I), comprises
a) the addition of a compound of formula HS-Z, wherein Z is as defined above, in the presence of a base, to a compound of formula (V) as defined above; in order to obtain said compound of formula (IV);
b) the removal of the Z group form said compound of formula (IV) obtained in step a); to obtain a compound of formula (III); and
c) the nitrosation of the thiol functionality of said compound of formula (III) obtained in step b).

An alternative method for the synthesis of the compounds of formula (I) comprises
a) the addition of SH₂ to a compound of formula (V) 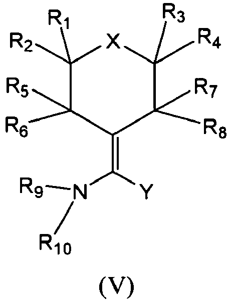 wherein
   R₁, R₂, R₃, R₄, R₅, R₆, R₇, R₈, R₉, R₁₀, Y and X are as defined in claim 1, to obtain a compound of formula (III); and
b) the nitrosation of the thiol functionality of said compound of formula (III) obtained in step a).

Thus, with this method it is possible to provide the compounds of formula (III) in a single step from the compounds of formula (V).

A further aspect of the invention is a method for the synthesis of said compounds of formula (IV) which comprises the addition of a compound of formula HS-Z, wherein Z is as defined above, in the presence of a base, to a compound of formula (V) as defined above.

As mentioned above, enantiomerically enriched or enantiomerically pure compounds of the invention may be obtained by chiral separation at any stage of the synthesis (i.e., chiral separation of compounds of formula (III), formula (IV) and/or formula (V)). According to a preferred embodiment, prior to nitrosation the compounds of formula (III) are subjected to chiral separation, preferably by chiral supercritical fluid separation, in order to obtain enantiomerically enriched mixtures or enantiomerically pure compounds of formula (III). Further nitrosation of said enantiomerically enriched mixtures or enantiomerically pure compounds of formula (III) provide enantiomerically enriched mixtures or enantiomerically pure compounds of the invention.

The compounds of formula (V) are readily available by reaction of ketones of formula (VI) 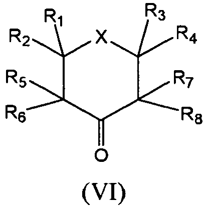 wherein R₁, R₂, R₃, R₄, R₅, R₆, R₇, R₈ and X are as defined in formula (I);
with alkylisocyanoacetates of formula (VII) 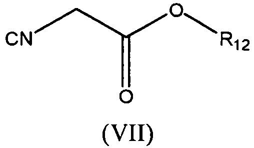 wherein R₁₂ is as defined in formula (I).

The conditions for this reactions may be found in DE 2801849 and Nunami Kenichi et al., J. Chem. Soc. Perkin Trans. 1, 1979, 9, 2224-9.

The resulting compounds of this reaction are compounds of formula (V) wherein R₉ is hydrogen and R₁₀ is a formyl group. Thus, if necessary, the appropriate functionality must be introduced in R₉ and R₁₀ This may be done by methods known to the skilled person at any stage of the synthesis of the compounds of the invention: prior to the formation of the compounds of formula (I), prior to the formation of the compounds of formula (III) or prior to the formation of the compounds (IV). The election of the precise stage at which to introduce or modify said R₉ and R₁₀ groups will depend on the compatibility of the different functional groups in the molecule. Said incompatibilities are a matter of common general knowledge or routine experimentation for the skilled person.

For example, it is possible to prepare a compound of formula (V) wherein R₉ is hydrogen and R₁₀ is a formyl group from a compound of formula (VI) and a compound of formula (VII) as described above, and then remove the formyl group. Then, a compound of formula (IV) (wherein R₉ and R₁₀ are hydrogen) may be prepared following the method described above. In further steps R₉ and R₁₀ groups may be introduced followed by removal of the Z group and then nitrosation. Alternatively, R₉ and R₁₀ may be introduced prior to the formation of the compound of formula (IV), i.e. after hydrolysis of the formyl group. The above mentioned alternatives are shown bellow in scheme I.

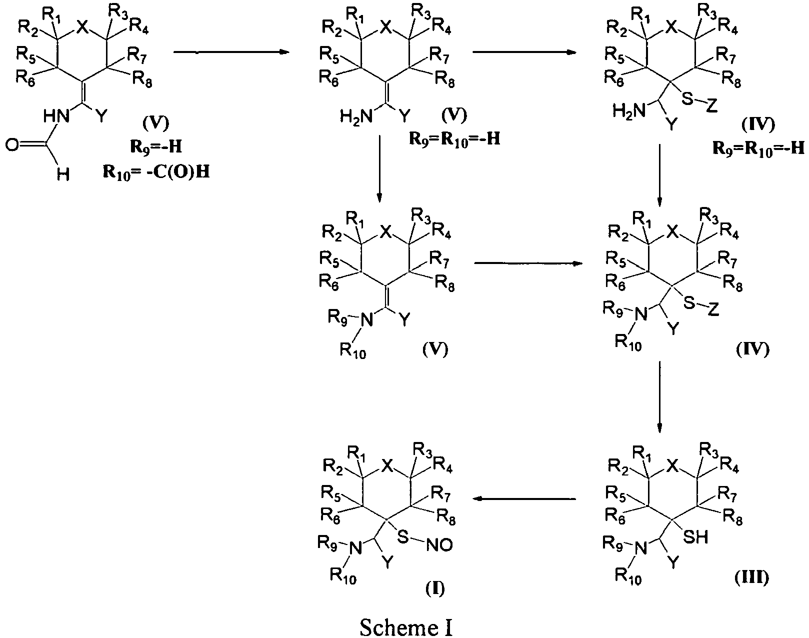

Another alternative is to prepare a compound of formula (V) wherein R₉ is hydrogen and R₁₀ is a formyl group form a compound of formula (VI) and a compound of formula (VII), transform said compound of formula (V) into a compound of formula (IV) (wherein R₉ is hydrogen and R₁₀ is formyl) and then remove the Z group to form a compound of formula (III) wherein R₉ is hydrogen and R₁₀ is formyl. Said compound of formula (III) may be used to prepare a compound of formula (I) wherein R₉ is hydrogen and R₁₀ is formyl. Alternatively, introduction of the R₉ and R₁₀ groups in said compound of formula (III), followed by nitrosation may yield a compound of formula (I). The above mentioned alternatives are shown bellow in scheme II

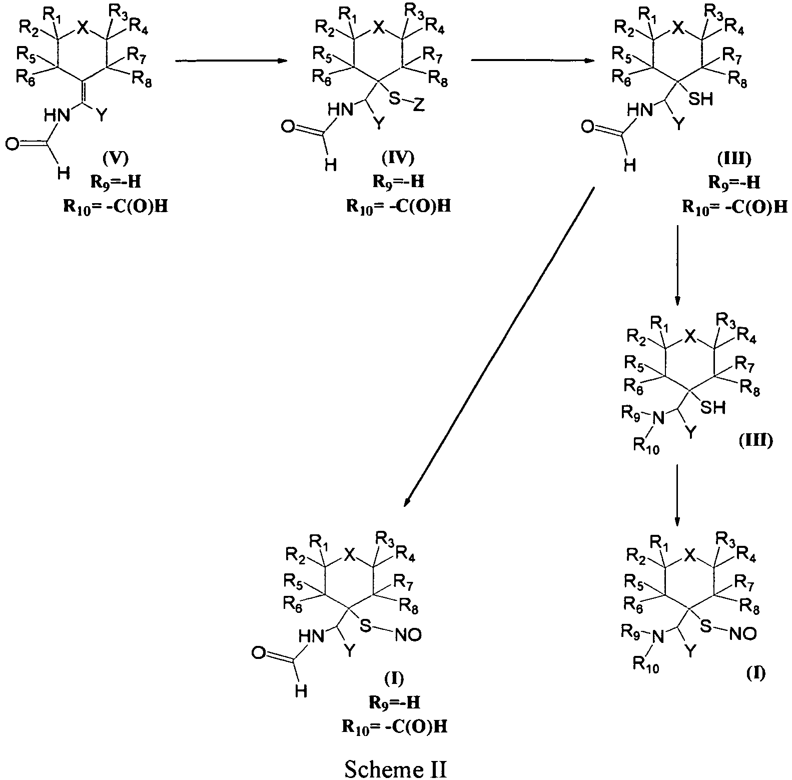

In a further embodiment it is also possible to introduce R₉ and R₁₀ in nonconsecutive steps at different points in the synthesis. Other possible combinations will be readily apparent to the skilled person.

### Uses of the compounds of the invention

A further aspect of the invention is directed to the compounds of the invention for use as a medicament.

A further aspect of the invention is a compound of the invention for use in the treatment and/or prophylaxis of a NO mediated conditions.

The term "treatment and/or prophylaxis" in the context of this specification means administration of a compound or formulation according to the invention to preserve health in a patient suffering or in risk of suffering a NO mediated condition. Said terms also include administration of a compound or formulation according to the invention to prevent, ameliorate or eliminate one or more symptoms associated with a NO mediated condition.

The compounds of the invention have shown increased stability with respect to those described in EP-B1-1157987 and comparable vasodilating activity and inhibitory effect on the aggregation of the platelets.

According to a preferred embodiment said NO mediated condition is platelet dysfunction, endocrine, metabolic, cardiovascular, inflammatory, genitourinary, digestive, dermatological, neuronal, central nervous system related, neurodegenerative diseases, mental disorders, cognitive disorders, respiratory or infectious conditions.

According to a further preferred embodiment, said NO mediated condition is hypertension, thrombosis, thromboembolic processes, vascular or trauma inflammation, complications associated to diabetes, ischemia-reperfusion, carotid endarterectomy and coronary bypass, graft rejections, in percutaneous coronary interventions, as vascular tone modulator, infant respiratory distress syndrome, asthma, pulmonary hypertension, bronchopulmonary dysplasia, cystic fibrosis, infectious diseases caused by bacteria, viruses and protozoa, Leishmaniasis, Trypanosomiasis, inhibition of AIDS virus replication, gastrointestinal tract motor diseases, inflammatory bowel disease, achalasia, diseases of the gall-bladder and the sphincter of Oddi, in cholangiopancreotography, liver failure, hepatic fibrosis, hepatic cirrhosis, portal hypertension, genitourinary tract diseases and erectile dysfunction, preeclampsia, endometrial hyperplasia, uterine smooth muscle proliferation, myometrium tumors, skin ulcers, ulcers related to diabetis, Alzheimer's disease, pain and fibromyalgias.

According to a further preferred embodiment said NO mediated condition is an ophthalmological disease caused or related to ocular hypertension, in particular glaucoma.

According to a further aspect, the present invention is directed to a pharmaceutical composition comprising a compound of the invention and a pharmaceutically acceptable carrier.

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions, also buffers, isotonic agents or agents capable increasing solubility. Suitable pharmaceutical carriers are described in "Remington's Pharmaceutical Sciences" by E.W. Martin or "Tratado de Farmacia Galénica", C. Faulí i Trillo, Luzán 5, S.A. de Ediciones, 1993.

The pharmaceutical composition of the invention may be administered in the form of different preparations. Non limiting examples are preparations for oral administration, e.g. tablets, capsules, syrups or suspensions; ophthalmological administration, e.g. solutions, ointments or creams; and parenteral administration, e.g. aqueous and non-aqueous sterile injection solutions or aqueous and non-aqueous sterile suspensions. Also, the pharmaceutical compositions of the invention may include topical compositions, e.g. creams, ointments or pastes, or transdermic preparations such as patches or plasters. The pharmaceutical composition of the invention may also be prepared for vaginal or for rectal administration, e.g. rectal gel or suppository.

Generally an effective administered amount of a compound used in the invention will depend on the relative efficacy of the compound chosen, the severity of the disorder being treated, or the age, weight or mode of administration. However, active compounds will typically be administered once or more times a day, for example 1, 2, 3 or 4 times daily, with typical total daily doses in the range of from 0.01 to 100 mg/kg/day.

The compounds used in the present invention may also be administered with other drugs to provide a combination therapy. The other drugs may form part of the same composition, or be provided as a separate composition for administration at the same time or at different time.

### Definitions

In the definitions of the compounds described herein the following terms have the meaning indicated:
"Alkyl" refers to a straight or branched hydrocarbon chain radical consisting of carbon and hydrogen atoms, containing no unsaturation, having one to twelve, preferably one to eight, more preferably one to six carbon atoms and which is attached to the rest of the molecule by a single bond, e. g., methyl, ethyl, n-propyl, i-propyl, n-butyl, t-butyl, n-pentyl, etc.
"Alkenyl" refers to a straight or branched hydrocarbon chain radical consisting of carbon and hydrogen atoms, containing at least one unsaturation, having two to twelve, preferably two to eight, more preferably two to six carbon atoms, and which is attached to the rest of the molecule by a single bond.
"Cycloalkyl" refers to a saturated carbocyclic ring having from three to eight, preferably three to six carbon atoms. Suitable cycloalkyl groups include, but are not limited to cycloalkyl groups such as cyclopropyl. cyclobutyl, cyclopentyl or cyclohexyl.
"Alkynyl" refers to a straight or branched hydrocarbon chain radical consisting of carbon and hydrogen atoms, containing at least one carbon-carbon triple bond, conjugated of not, having two to twelve, preferably two to eight, more preferably two to six carbon atoms, and which is attached to the rest of the molecule by a single bond, such as -CCH, -CH₂CCH, -CCCH₃, -CH₂CCCH₃, and which is attached to the rest of the molecule by a single bond.
"Aryl" refers to an aromatic hydrocarbon radical having six to ten carbon atoms such as phenyl or naphthyl.
"Aralkyl" refers to an aryl group linked to the rest of the molecule by an alkyl group such as benzyl and phenethyl.
"Heterocyclyl" refers to a stable 3- to 15- membered ring which consists of carbon atoms and from one to five heteroatoms selected from the group consisting of nitrogen, oxygen, and sulphur, preferably a 4-to 8-membered ring with one, two, three or four heteroatoms, more preferably a 5-or 6-membered ring with one, two or three heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quatemized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

Unless otherwise indicated, alkyl, cycloalkyl, alkenyl, alkynyl, aryl, aralkyl and heterocyclyl radicals may be optionally substituted by one, two or three substituents such as halo, alkyl, alkenyl, alkynyl, cycloalkyl, hydroxy, alkoxy, sulfoxy, OBenzyl, OBenzoyl, carboxy, cyano, carbonyl, acyl, alkoxycarbonyl, amino, imino and nitro.

The term "alkoxycarbonyl" refers to compounds of formula -C(=O)O-, wherein the Carbon terminus is attached to the molecule and the oxygen terminus is attached to a carbon atom to form an ester functionality, i.e. MOLECULE-C(=O)O- C Said carbon atom me be part of an alkyl, alkenyl, cycloalkyl, alkynyl, aryl, aralkyl or heterocyclyl group.

### Experimental section

The examples set forth in this description detail the suitable processes for obtaining several compounds which can be assigned to formula (I). In view of said examples, it is evident and direct for a person skilled in the art to obtain the compounds which are not explicitly exemplified, by means of applying modifications of the methods set forth, characteristic of the common general knowledge of the persons skilled in the art.

Consequently, the examples set forth below must not be interpreted as limiting the scope of the present invention, but rather as an additional and more detailed explanation which guides the person skilled in the art to a deeper understanding of the invention.

The following abbreviations are used in the following examples:

| | |
|---|---|
| AcOEt | Ethyl acetate |
| AcOH | Acetic acid |
| DMSO-d₆ | Hexadeuterodimethyl sulfoxide |
| EtOH | Ethanol |
| Et₂O | Diethyl ether |
| HPLC | high pressure liquid chromatography |
| IPA | Isopropyl alcohol |
| RT | Room temperature |
| THF | Tetrahydrofuran |
| TLC | Thin layer chromatography |

The nuclear magnetic resonance spectra have been carried out in a Varian Gemini-200 apparatus.

The ¹H -NMR spectra indicate the working frequency and the solvent used to make the spectrum. The position of the signals is indicated in δ (ppm), using the signal of the protons of the solvent as reference. The reference values are 7.24 ppm for chloroform and 2.49 ppm for deuterated dimethyl sulfoxide. Within brackets are indicated the number of protons corresponding to each signal measured by electronic integration and the type of signal using the following abbreviations: s (singlet), d (doublet), t (triplet), q (quadruplet), s.b. (signal broad). The signal assignation is indicated in some cases.

The ¹³C-NMR spectra indicate the working frequency and the solvent used to make the spectrum. The position of the signals is indicated in δ (ppm), using the central signal of the solvent as reference. The reference values are 77.00 ppm for chloroform and 39.50 ppm for deuterated dimethylsulfoxide.

### A) SYNTESIS OF THE RELEVANT COMPOUNDS

### Example 1.

### 1a).- Synthesis of Ethyl formylamino-(1-methyl-piperidin-4-ilydene)-acetate

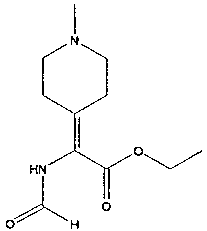

180 g (1.6 moles) of potassium tert-butoxide and 1L of dry THF are added to a 6L flask with 3 mouths, provided with condenser, thermometer and a calcium chloride tube. It is cooled with an ice bath and 181 ml (1.6 moles) of ethyl isocyanoacetate dissolved in 180 ml of dry THF are added dropwise, keeping the temperature below 15°C. After the addition, 187 ml (1.6 moles) of 1-methyl-4-piperidone dissolved in 187 ml of dry THF are added dropwise, keeping the temperature below 15°C. After the addition, it is allowed to reach room temperature and it is stirred for 30 minutes. 900 ml of a 1/1 AcOH/H₂O solution is added, controlling that the exothermic which occurs does not exceed 45°C. After the addition, the stirring is maintained for 1 hour. The THF is eliminated under vacuum and 1L of water is added. 350 g of sodium carbonate is added until reaching a pH between 8 and 9 and it is extracted with 5x600 ml of dichloromethane. The organic phase is dried and concentrated, obtaining 248 g of a dark brown solid which is recrystallized with 1.4 L of a 1/1 toluene/cyclohexane mixture to yield 167 g of a light brown solid (Yield: 46%).
**¹H -NMR (D₂O, 200 MHz):** 7.80 (s, 1H, CHO); 3.93 (q, 2H, OCH₂); 2.50-2.10 (m, 8H, piperidine); 1.93 (s, 3H, NCH₃); 0.95 (t, 3H, CH₃)
**¹³C-NMR (D₂O, 200 MHz):** 165.81 (C=O); 163.12 (C=O); 146.88 (C=C); 117.69 (C=C); 61.94 (OCH₂); 54.35 and 53.98 (NCH₂); 43.53 (NCH₃); 29.08 and 28.41 (CH); 12.76 (CH₃)

### 1b).- Synthesis of Ethyl (R,S)-formylamino-[1-methyl-4-(4-methoxybenzylsulfanyl)-piperidin-4-yl]-acetate

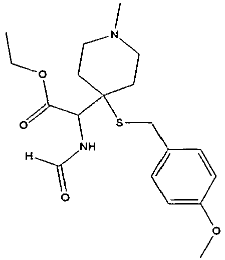

16.09 g of 55% sodium hydride in mineral oil (0.367 moles) and 300 ml of toluene are added in a 1L flask with 3 mouths, provided with a condenser and thermometer. It is cooled at 10°C and 56.8 ml (0.367 moles) of 4-methoxybenzylthiol dissolved in 200 ml of toluene are quickly added and it is stirred at this temperature for 5 minutes. 83.0 g (0.367 moles) of ethyl formylamino-(1-methyl-piperidin-4-ilydene)-acetate, product obtained in (1a), is added in portions, preventing the temperature for exceeding 35°C. After the addition, it is maintained at RT for 3 hours. 370 ml of 2N hydrochloric acid is added and the phases are separated. The aqueous phase is washed with 2x300ml of toluene to eliminate impurities. The aqueous phase is taken to pH 10-11 with 2N NaOH and it is extracted with 4x300 ml of dichloromethane, which once it is dried on sodium sulfate, is filtered and concentrated. 125.7 g of a completely pure amber oil is obtained by TLC (CH₂Cl₂/EtOH/NH₄OH) and NMR. Yield: 90%
**¹H-NMR (DMSO-d₆, 200 MHz):** 8.70 (d, J=9.2Hz; 1H, NH); 8.11 (s, 1H, CHO); 7.21 (d, J=8.6Hz, 2H); 6.86 (d, J=8.6Hz, 2H); 4.74 (d, J=9.2Hz, 1H, CH); 4.16 (q, 2H, OCH₂); 3.72 (s, 3H, OCH₃); 3.63 (s, 2H, SCH₂); 2.60-2.20 (m, 4H, piperidine); 2.16 (s, 3H, NCH₃); 1.90-1.50 (m, 4H, piperidine); 1.24 (t, 3H, CH₃)
**¹³C-NMR (DMSO-d₆, 200 MHz):** 169.50 (C=O); 161.23 (C=O); 158.30 (C-O); 130.26 (2C, CH); 128.67 (1C, C-CH₂); 113.85 (2C, CH); 60.81 (OCH₂); 57.01 (1C, CH); 55.04 (1C, OCH₃); 50.30 and 50.08 (3C, 2 NCH₂ and C4piperidine); 45.74 (NCH₃); 30.70 and 30.22 (3C, 2CH₂ and S-CH₂); 14.02 (1C, CH₃)

### 1c).- Synthesis of Ethyl (R,S)-amino-[1-methyl-4-(4-methoxy-benzylsulfanyl)piperidin-4-yl]-acetate

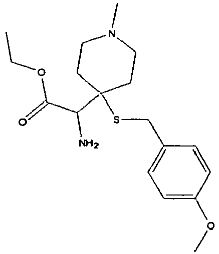

125.7 g (0.330 moles) of Ethyl (R,S)-formylamino-[1-methyl-4-(4-methoxybenzylsulfanyl)-piperidin-4-yl]-acetate and 300 ml of a 6M EtOH/HCl solution are added to a 1L flask and heated under reflux for 24 hours. It is concentrated to dryness and residue is dissolved in 700 ml of water and washed with 2x200 ml of AcOEt. The aqueous phase is basified with 30% NH₄OH and extracted with 3x300 ml of dichloromethane. The organic phase is dried, filtered and concentrated and 109.3 g (Yield: 94%) of a completely pure amber oil are obtained by TLC (CH₂Cl₂/EtOH/NH₄OH) and NMR
**¹H -NMR (DMSO-d₆, 200 MHz):** 7.21 (d, J=8.4Hz, 2H); 6.86 (d, J=8.4Hz, 2H); 4.12 (q, 2H, OCH₂); 4.10-3.90 (m, 1H, CH); 3.72 (s, 3H, OCH₃); 3.60 (s, 2H, SCH₂); 2.50 (s.b, 2H, NH₂); 2.40-2.20 (m, 2H, piperidine); 2.15 (s, 3H, NCH₃); 2.10-1.50 (m, 6H, piperidine); 1.23 (t, 3H, CH₃)
**¹³C-NMR (DMSO-d₆, 200 MHz):** 173.32 (C=O); 158.15 (C-O); 130.11 (2C, CH); 129.36 (1C, C-CH₂); 113.76 (2C, CH); 61.82 (1C, CH); 59.91 (OCH₂); 54.99 (1C, OCH₃); 51.23 and 50.60 ((3C, 2 NCH₂ and C4piperidine); 45.90 (NCH₃); 30.35, 30.21 and 30.11 (3C, 2CH₂ and S-CH₂); 14.09 (1C, CH₃)

### 1d).- Synthesis of Ethyl (R,S)-acetylamino-[1-methyl-4-(4-methoxybenzylsulfanyl)-piperidin-4-yl]-acetate

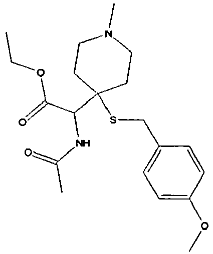

18 g (50 mmol) of Ethyl (R,S)-amino-[1-methyl-4-(4-methoxy-benzylsulfanyl)-piperidin-4-yl]-acetate, product obtained in 1c, and 300 ml of dry THF area added to a 1L flask. It is cooled at 0°C and 7.12 ml (50 mmol) of triethylamine are added. 3.64 ml (50 mmol) of acetyl chloride in 10 ml of dry THF are added dropwise. It is stirred for 2 hours at 0°C, it is concentrated to dryness and the residue is dissolved in 100 ml of dichloromethane and washed with 2x50 ml of a 5% sodium bicarbonate solution. The organic phase is dried, filtered and concentrated. 19.37g (Yield: 96%) of an amber oil which crystallizes by letting it be is obtained.
**¹H -NMR (CDCl₃, 200 MHz):** 7.21 (d, J=8.8Hz, 2H); 6.82 (d, J=8.8Hz, 2H); 6.32 (d, J=9Hz, NH); 4.78 (d, J=9Hz, 1H, CH); 4.21 (q, 2H, OCH₂); 3.77 (s, 3H, OCH₃); 3.61-3.58 (m, 2H, SCH₂); 2.70-2.40 (m, 4H, piperidine); 2.28 (s, 3H, NCH₃); 2.00 (s, 3H, COCH₃); 2.10-1.60 (m, 4H, piperidine); 1.30 (t, 3H, CH₃)
**¹³C-NMR (CDCl₃, 200 MHz):** 173.40 (C=O); 170.05 (C=O); 158.92 (C-O); 130.38 (2C, CH); 128.78 (1C, C-CH₂); 114.15 (2C, CH); 61.64 (OCH₂); 58.56 (1C, CH); 55.38 (1C, OCH₃); 50.91 ((3C, 2 NCH₂ and C4piperidine); 46.15 (NCH₃); 32.97, 32.05 and 31.27 (3C, 2CH₂ and S-CH₂); 23.41(1C, CO-CH₃); 14.27 (1C, CH₃)

### 1e).- Synthesis of (R,S)-4-(Acetylamino-ethoxycarbonyl-methyl)-4-mercapto-1methyl-piperidinium (II) trifluoroacetate

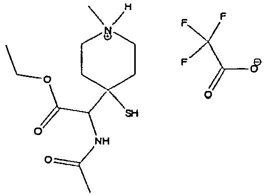

1.97 g (5 mmol) of the product obtained in 1d and 5 ml of trifluoroacetic acid are added to a 50 ml flask and heated under reflux for 20 hours. It is allowed to cool and 20 ml of water are added. It is washed with 3x50 ml of AcOEt. The aqueous phase is concentrated to dryness by azeotropically distilling the remains of water with IPA. The residue is treated with Et₂O and stirring and a white solid is obtained which is filtered and dried. 1.7 g are obtained (Yield: 88%)
**¹H -NMR (DMSO-d₆, 200 MHz):** 10.60-9.80(s.b., 1H, CO₂H); 8.50-8.10 (s.b., 1H, NH); 4.52 (s.b., 1H, CH); 4.10 (q, 2H, OCH₂); 3.50-2.90 (m, 5H, 4Hpiperidine and SH); 2.77 (s, 3H, NCH₃); 2.10-1.60 (m, 7H, COCH₃ and 4Hpiperidine); 1.85 (t, 3H, CH₃)
**¹³C-NMR (DMSO-d₆, 200 MHz):** 169.92 (C=O); 168.46 (C=O); 159.14 and 158.51 (CO₂H); 120.00 and 114.07 (CF3); 61.42 (1C, CH); 60.95 (OCH₂); 49.46 (2C, 2 NCH₂); 46.41 (1C, C4piperidine); 42.40 (NCH₃); 33.01 and 32.27 (2C, 2CH₂); 22.26 (1C, COCH₃); 14.01 (1C, CH₃)

### 1f).- Synthesis of Ethyl (R,S)-acetylamino-(1-methyl-4-S-nitrosomercaptopiperidin-4-yl)-acetate

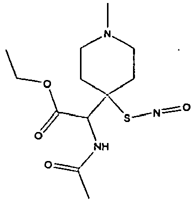

1.17 g (3 mmol) of (R,S)-4-(Acetylamino-ethoxycarbonyl-methyl)-4-mercapto-1-methyl-piperidinium trifluoroacetate (1e) are dissolved in 15 ml of 1N HCl, it is cooled externally with an ice bath for 5 min and 6 ml (6 mmol) of a 1M sodium nitrite solution are added. It is stirred at 0°C for 5 minutes and it is taken to pH 11-12 with 2N NaOH. It is extracted with 3x30 ml of dichloromethane. The organic phase is dried, filtered and concentrated under vacuum (186 mm/Hg) and at 16°C. 650 mg of a reddish-greenish solid foam are obtained. (Yield: 72%)
**¹H -NMR (DMSO-d₆, 200 MHz):** 8.57 (d, J=9Hz, NH); 5.35 (d, J=9Hz, 1H, CH); 4.03 (q, 2H, OCH₂); 2.80-2.00 (m, 8H,piperidine); 2.17 (s, 3H, NCH₃); 1.88 (s, 3H, COCH₃); 1.11 (t, 3H, CH₃)
¹³C**-NMR (DMSO-d₆, 200 MHz):** 169.87 (C=O); 169.25 (C=O); 61.16 (1C, C4piperidine); 61.00 (OCH₂); 59.31 (1C, CH); 50.74 and 50.61 (2C, 2NCH₂); 45.61 (NCH₃); 32.17 and 31.77 (2C, 2CH₂); 22.17 (1C, COCH₃); 13.81 (1C, CH₃)

### 1g).- Synthesis of (R,S)-4-(Acetylamino-ethoxycarbonyl-methyl)-1-methyl-4-S-nitrosomercapto-piperidinium chloride

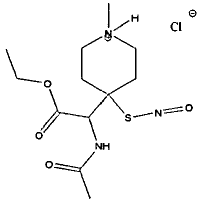

650 mg (2.14 mmol) of the product obtained in 1f) are dissolved in 50 ml of Et₂O and 177µL (2.14 mmol) of concentrated hydrochloric acid (12.076M) are added. A mixture of solid and oil is formed. 10 ml of EtOH are added until it is completely solubilized and it is concentrated to dryness, keeping the temperature under 20°C. The residue is treated with dry Et₂O and stirring and a reddish-green solid precipitates which is filtered and dried. 450mg (Yield: 62%) are thus obtained, with a 96.5% purity by HPLC.
**¹H -NMR (DMSO-d₆, 200 MHz):** 11.20 (s.b., HCl); 8.68 (d, J=9.6Hz, NH); 5.24 (d, J=9.6Hz, 1H, CH); 4.08 (q, 2H, OCH₂); 3.80-3.20 (m, 4H,piperidine); 3.47 (s, 3H, NCH₃); 3.10-2.60 (m, 4H,piperidine); 1.87 (s, 3H, COCH₃); 1.08 (t, 3H, CH₃)
**¹³C-NMR (DMSO-d₆, 200 MHz):** 169.99 (C=O); 168.59 (C=O); 61.44 (OCH₂); 59.75 (1C, C4piperidine); 59.25 (1C, CH); 49.16 and 49.00 (2C, 2NCH₂); 41.98 (NCH₃); 29.41 (2C, 2CH₂); 22.20 (1C, COCH₃); 13.84 (1C, CH₃)

Starting from Ethyl (R,S)-amino-[1-methyl-4-(4-methoxy-benzylsulfanyl)-piperidin-4-yl]-acetate, obtained in 1c and substituting the acetyl chloride used in 1d with the corresponding acid chloride or sulfonyl chloride and following a process similar to those described in Examples 1d to 1f, the following products are obtained:

### Example 2. Ethyl (R,S)-heptanoylamino-(1-methyl-4-S-nitrosomercapto-piperidin-4-yl)-acetate

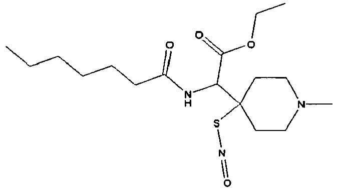

Yield last step: 66%. HPLC purity 98.8%
**¹H -NMR (DMSO-d₆, 200 MHz):** 8.54 (d, J=9.6Hz, NH); 5.36 (d, J=9.6Hz, 1H, CH); 4.04 (q, 2H, OCH₂); 3.04 (s.b., 2H, piperidine); 2.70-2.30 (m, 6H,piperidine); 2.43 (s, 3H, NCH₃); 2.16 (t, 2H, COCH₂); 1.43 (m, 2H, CH₂); 1.30-1.00 (m, 9H, 3CH₂ + OCH₂CH₃); 0.83 (t, 3H, CH₃)
**¹³C-NMR (DMSO-d₆, 200 MHz):** 172.98 (C=O); 169.05 (C=O); 61.12 (1C, C4piperidine); 60.25 (OCH₂); 59.02 (1C, CH); 50.14 and 50.01 (2C, 2NCH₂); 44.13 (NCH₃); 34.70 (1C, CO-CH₂), 31.11 and 30.91 (3C, 2CH₂piperidine +CH₂); 28.16 (1C, CH₂); 25.14 (1C, CH₂); 21.98 (1C, CH₂); 13.87 and 13.80 (2C, 2CH₃)

By a process similar to that described in 1g, the corresponding hydrochloride is obtained: (**R,S)-4-(Heptanoylamino-ethoxycarbonyl-methyl)-1-methyl-4-S-nitrosomercapto-piperidinium chloride**
**¹H -NMR (DMSO-d₆, 200 MHz):** 11.20 (s.b., HCl); 8.62 (d, NH); 5.24 (d, 1H, CH); 4.09 (q, 2H, OCH₂); 3.10-2.60 (m, 8Hpiperidine + NCH₃); 2.14 (t, 2H, COCH₂); 1.60-1.00 (m, 4CH₂ + OCH₂CH₃); 0.83 (s.b., 3H, CH₃)
**¹³C-NMR (DMSO-d₆, 200 MHz):** 173.01 (C=O); 168.66 (C=O); 61.42 (OCH₂); 59.63 (1C, CH); 59.22 (1C, C4piperidine); 49.19 and 49.03 (2C, 2NCH₂); 42.01 (NCH₃); 34.74 (1C, CO-CH₂), 30.94 (1C, CH₂); 29.52 and 29.33 (2C, 2CH₂piperidine); 28.19 (1C, CH₂); 25.09 (1C, CH₂); 21.99 (1C, CH₂); 13.91 and 13.85 (2C, 2CH₃)

### Example 3. Ethyl (R,S)-benzoylamino-(1-methyl-4-S-nitrosomercapto-piperidin-4-yl)-acetate

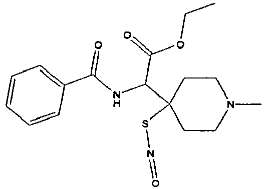

Yield last step: 5%. HPLC purity 99.3%
**¹H -NMR (DMSO-d₆, 200 MHz):** 8.88 (d, J=9Hz, NH); 7.80 (d, 2H); 7.55-7.40 (m, 3H); 5.61 (d, J=9Hz, 1H, CH); 4.08 (q, 2H, OCH₂); 3.43 (s.b., 2H, piperidine +D₂O); 2.86 (s.b., 2H, piperidine); 2.61 (s.b., 2H, piperidine); 2.27 (s.b., 5H, 2H,piperidine + NCH₃); 1.13 (t, 3H, CH₃)
**¹³C-NMR (DMSO-d₆, 200 MHz):** 168.93 (C=O); 167.40 (C=O); 133.48 (1C); 131.74 (1C); 128.21 (2C); 127.87 (2C); 61.18 (1C, C4piperidine); 61.07 (OCH₂); 60.17 (1C, CH); 50.63 and 50.45 (2C, 2NCH₂); 45.10 (NCH₃); 31.96 and 31.56 (2C, 2CH₂piperidine); 13.88 (1C, CH₃)

By a process similar to that described in 1g the corresponding hydrochloride is obtained: **(R,S)-4-(Benzoylamino-ethoxycarbonyl-methyl)-1-methyl-4-S-nitrosomercapto-piperidinium chloride**
**¹H -NMR (DMSO-d₆, 200 MHz):** 11.20 (s.b., HCl); 9.02 (d, NH); 7.83 (d, 2H); 7.55-7.40 (m, 3H); 5.53 (d, J=9Hz, 1H, CH); 4.11 (q, 2H, OCH₂); 3.50-3.35 (m, 2H, piperidine +D₂O) 3.20-2.80 (m, 6H, piperidine); 2.74 (s.b., NCH₃); 1.13 (t, 3H, CH₃)
**¹³C-NMR (DMSO-d₆, 200 MHz):** 168.43 (C=O); 167.51 (C=O); 133.41 (1C); 131.79 (1C); 128.20 (2C); 127.97 (2C); 61.50 (OCH₂); 60.29 (1C, CH); 59.31 (1C, C4piperidine); 49.27 and 49.07 (2C, 2NCH₂); 42.05 (NCH₃); 29.57 and 29.27 (2C, 2CH₂piperidine); 13.88 (1C, CH₃)

### Example 4. Ethyl (R,S)-(4-chloro-benzoylamino)-(1-methyl-4-S-nitrosomercaptopiperidin-4-yl)-acetate

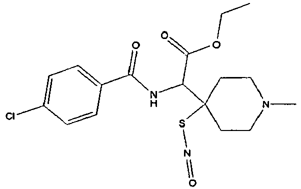

Yield last step:77%. HPLC purity 93.4%
**¹H -NMR (CDCl₃, 200 MHz):** 7.66 (d, 2H); 7.38 (d, 2H); 6.92 (d, J=9Hz, NH); 5.60 (d, J=9Hz, 1H, CH); 4.12 (q, 2H, OCH₂); 2.90-2.20 (m, 8H, piperidine); 2.32 (s.b., NCH₃); 1.20 (t, 3H, CH₃)
**¹³C-NMR (CDCl₃, 200 MHz):** 169.40 (C=O); 166.23 (C=O); 138.26 (1C); 131.75 (1C); 128.85 (2C); 128.49 (2C); 62.02 (OCH₂); 61.20 (1C, C4piperidine); 60.38 (1C, CH); 51.12 and 51.05 (2C, 2NCH₂); 45.83 (NCH₃); 33.67 (2C, 2CH₂piperidine); 13.88 (1C, CH₃)

By a process similar to that described in 1g the corresponding hydrochloride is obtained: **(R,S)-4-[(4-Chloro-benzoylamino)-ethoxycarbonyl-methyl)]-1-methyl-4-S-nitrosomercapto-piperidinium chloride**
**¹H -NMR (CDCl₃, 200 MHz):** 12.40 (s.b., HCl); 7.76 (d, 2H); 7.34 (s.b., 2Hphenyl + NH); 5.60 (d, 1H, CH); 4.12 (q, 2H, OCH₂); 3.90-2.60 (m, 8H piperidine + NCH₃); 1.20 (t, 3H, CH₃)
**¹³C-NMR (CDCl₃, 200 MHz):** 168.70 (C=O); 166.76 (C=O); 138.21 (1C); 131.41 (1C); 128.94 (2C); 128.68 (2C); 62.40 (OCH₂); 60.07 (1C, CH); 58.73 (1C, C4piperidine); 50.48 and 50.24 (2C, 2NCH₂); 43.33 (NCH₃); 29.82 (2C, 2CH₂piperidine); 13.80 (1C, CH₃)

### Example 5. Ethyl (R,S)-(2-chloro-benzoylamino)-(1-methyl-4-S-nitrosomercaptopiperidin-4-yl)-acetate

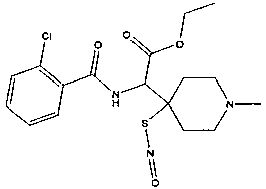

Yield last step: 91%. HPLC purity 91%
**¹H -NMR (DMSO-d₆, 200 MHz):** 9.32 (d, J=8.8Hz, NH); 7.47-7.31 (m, 4H); 5.56 (d, J=8.8Hz, 1H, CH); 4.10 (q, 2H, OCH₂); 3.20-2.50 (m, 8H, piperidine); 2.49 (s, NCH₃); 1.15 (t, 3H, CH₃)
**¹³C-NMR (DMSO-d₆, 200 MHz):** 168.46 (C=O); 166.98 (C=O); 135.96 (1C); 130.99 (1C); 129.94 (1C); 129.45 (1C); 128.97 (1C); 126.92 (1C); 61.24 (OCH₂); 60.21 (1C, C4piperidine); 59.79 (1C, CH); 50.21 and 50.06 (2C, 2NCH₂); 44.28 (NCH₃); 31.31 and 31.02 (2C, 2CH₂piperidine); 13.84 (1C, CH₃)

By a process similar to that described in 1g, the corresponding hydrochloride is obtained: **(R,S)-4-[(2-Chloro-benzoylamino)-ethoxycarbonyl-methyl)]-1-methyl-4-S-nitrosomercapto-piperidinium chloride**
**¹H -NMR (DMSO-d₆, 200 MHz):** 11.60 (s.b., HCl); 9.38 (d, J=8.8Hz, NH); 7.47-7.35 (m, 4H); 5.45 (d, J=8.8Hz, 1H, CH); 4.12 (q, 2H, OCH₂); 3.60-2.80 (m, 8H, piperidine); 2.72 (s, NCH₃); 1.15 (t, 3H, CH₃)
**¹³C-NMR (DMSO-d₆, 200 MHz):** 168.05 (C=O); 167.03 (C=O); 135.81 (1C); 131.05 (1C); 129.95 (1C); 129.44 (1C); 128.95 (1C); 126.91 (1C); 61.54 (OCH₂); 60.29 (1C, CH); 59.07 (1C, C4piperidine); 49.15 and 48.98 (2C, 2NCH₂); 42.05 (NCH₃); 29.46 (2C, 2CH₂piperidine); 13.86 (1C, CH₃)

### Example 6. Ethyl (R,S)-(1-methyl-4-S-nitrosomercapto-piperidin-4-yl)-[(pyridin-3-carbonyl)-amino]-acetate

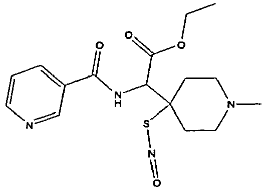

Yield last step: 76%. HPLC purity 95.2%
**¹H -NMR (CDCl₃, 200 MHz):** 8.96 (s.b., 1H); 8.74 (s.b., 1H); 8.04 (m, 1H); 7.39 (m, 1H); 6.99 (d, NH); 5.64 (d, 1H, CH); 4.14 (q, 2H, OCH₂); 3.00-2.20 (m, 8H, piperidine); 2.32 (s.b., NCH₃); 1.24 (t, 3H, CH₃)
**¹³⁰C-NMR (CDCl₃, 200 MHz):** 169.28 (C=O); 165.54 (C=O); 152.77 (1C); 148.17 (1C); 134.97 (1C); 129.19 (1C); 123.45 (1C); 62.16 (OCH₂); 61.20 (1C, C4piperidine); 60.51 (1C, CH); 51.11 (2C, 2NCH₂); 45.94 (NCH₃); 33.97 and 33.84 (2C, 2CH₂piperidine); 13.92 (1C, CH₃)

By a process similar to that described in 1g, the corresponding hydrochloride is obtained: **(R,S)-4-{Ethoxycarbonyl-[(pyridin-3-carbonyl)-amino]-methyl}-1-methyl-4-S-nitrosomercapto-piperidinium chloride**
**¹H -NMR (DMSO-d₆, 200 MHz):** 11.60 (s.b., HCl); 9.54 (d, NH); 9.11 (s.b., 1H); 8.84 (m, 1H); 8.47 (m, 1H); 7.72 (m, 1H); 5.53 (d, 1H, CH); 4.11 (q, 2H, OCH₂); 3.43 (s.b., 2H piperidine); 3.00 (s.b., 6H, piperidine); 2.73 (s.b., NCH₃); 1.15 (t, 3H, CH₃)
**¹³C-NMR (DMSO-d₆, 200 MHz):** 168.05 (C=O); 165.26 (C=O); 149.32 (1C); 146.38 (1C); 139.12 (1C); 130.24 (1C); 124.57 (1C); 61.61 (OCH₂); 60.35 (1C, CH); 59.22 (1C, C4piperidine); 49.15 and 48.99 (2C, 2NCH₂); 41.95 (NCH₃); 29.46 and 29.00 (2C, 2CH₂piperidine); 13.84 (1C, CH₃)

### Example 7. Ethyl (R,S)-benzenesulfonylamino-(1-methyl-4-S-nitrosomercaptopiperidin-4-yl)-acetate

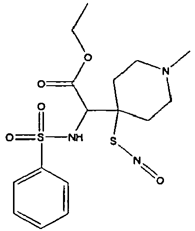

Yield last step: 99%. HPLC purity 96%
**¹H -NMR (DMSO-d₆, 200 MHz):** 8.75 (s.b., NH); 7.76-7.53 (m, 5H); 4.61 (s.b., 1H, CH); 3.58 (q, 2H, OCH₂); 2.80-2.00 (m, 8H, piperidine); 2.15 (s, NCH₃); 0.83 (t, 3H, CH₃)
**¹³C-NMR (DMSO-d₆, 200 MHz):** 168.12 (C=O); 140.36 (1C); 132.65 (1C); 128.99 (2C); 126.57 (2C); 63.58 (1C, CH); 60.86 (OCH₂ + C4piperidine); 50.63 and 50.56 (2C, 2NCH₂); 45.61 (NCH₃); 31.61 (2C, 2CH₂piperidine); 13.39 (1C, CH₃)

As way of example of different salts which may be used in the invention several salts of compound 7 have been prepared (examples 7a to 7m). The method followed is analogous to that of example 1g.

### Example 7a (R,S)-4-(Benzenesulfonylamino-ethoxycarbonyl-methyl)-1-methyl-4-S-nitrosomercapto-piperidinium chloride

1.66g (4.13 mmol) of the product ethyl **(R,S)-benzenesulfonylamino-(1-methyl-4-S-nitrosomercapto-piperidin-4-yl)-acetate** were dissolved in 50 ml of Et₂O and 5 ml of EtOH, and 5.9ml of a 0.7M solution of hydrochloric acid in ethanol (4.13 mmol) were added and a reddish-green solid precipitated, which was filtered and dried. 1.63g (Yield:91%), with an HPLC purity of 100%, were thus obtained
**¹H -NMR (DMSO-d₆, 200 MHz):** 11.40 (s.b., HCl); 8.85 (d, NH); 7.76-7.53 (m, 5H); 4.47 (d, 1H, CH); 3.67 (q, 2H, OCH₂); 3.50-2.60 (m, 8H, piperidine); 2.70 (s, NCH₃); 0.86 (t, 3H, CH₃)
**¹³C-NMR (DMSO-d₆, 200 MHz):** 167.47 (C=O); 140.17 (1C); 132.78 (1C); 129.05 (2C); 126.63 (2C); 64.21 (1C, CH); 61.35 (OCH₂); 58.92 (1C, C4piperidine); 49.08 and 48.86 (2C, 2NCH₂); 42.03 (NCH₃); 29.67 and 28.81 (2C, 2CH₂piperidine); 13.43 (1C, CH₃)

### Example 7b. (R,S)-4-(benzenesulfonylamino-ethoxycarbonyl-methyl)-1-methyl-4-S-nitrosomercapto-piperidinium chloride

2g (5 mmol) of the product ethyl **(R,S)-benzenesulfonylamino-(1-methyl-4-S-nitrosomercapto-piperidin-4-yl)-acetate** were dissolved in 10 ml of EtOH and 7.4ml of a 0.81M solution of hydrochloric acid in ethanol (6 mmol) were added and a reddish-green solid precipitated. It was cooled at 5°C for 5 minutes and it was filtered and dried. 1.63g (Yield:74.4%) of a product identical to that obtained in Example 7b and with an HPLC purity of 95.5% were thus obtained.

### Example 7c. (R,S)-4-(benzenesulfonylamino-ethoxycarbonyl-methyl)-1-methyl-4-S-nitrosomercapto-piperidinium chloride

2g (5 mmol) of the product ethyl **(R,S)-benzenesulfonylamino-(1-methyl-4-S-nitrosomercapto-piperidin-4-yl)-acetate** were dissolved in 20 ml of EtOH and 455µL of concentrated hydrochloric acid (36%) were added. It was externally cooled with an ice bath and a reddish-green solid precipitated, which was kept stirring at 5°C for 10 minutes and was then filtered and dried. 1.33g (Yield:60.7%) of a product identical to that obtained in Example 7b and with an HPLC purity of 97.3% were thus obtained.

The following salts of the product of **Example 7a** were obtained by following a process similar to that described in **Example 7b** and substituting hydrochloric acid with the corresponding organic or inorganic acid:

### Example 7d. (R,S)-4-(benzenesulfonylamino-ethoxycarbonyl-methyl)-1-methyl-4-S-nitrosomercapto-piperidinium acetate.

Starting from 2g (5mmol) of **ethyl (R,S)-benzenesulfonylamino-(1-methyl-4-S-nitroso mercapto-piperidin-4-yl)-acetate** and 285µL of glacial acetic acid, 0.84g of the product of the example with an HPLC purity of 92.2% were obtained.

A 2^{nd} fraction of 1.35g and an HPLC purity of 73.7% was obtained by concentrating the filtrate liquids.
**¹H -NMR (DMSO-d₆, 200 MHz):** 8.85 (s.b., NH); 7.76-7.53 (m, 5H); 4.61 (d, 1H, CH); 3.58 (q, 2H, OCH₂); 2.80-2.10 (m, 8H, piperidine); 2.16 (s, NCH₃); 1.88 (s, CH₃. acetic); 0.83 (t, 3H, CH₃)
**¹³C-NMR (DMSO-d₆, 200 MHz):** 172.05 (C=O); 168.13 (C=O); 140.39 (1C); 132.66 (1C); 129.01 (2C); 126.59 (2C); 63.61 (1C, CH); 60.91 and 60.86 (2C, OCH₂ and C4piperidine); 50.60 and 50.53 (2C, 2NCH₂); 45.54 (NCH₃); 31.59 (2C, 2CH₂piperidine); 21.09 (1C, CH₃); 13.41 (1C, CH₃)

### Example 7e. (R,S)-4-(benzenesulfonylamino-ethoxycarbonyl-methyl)-1-methyl-4-S-nitrosomercapto-piperidinium mesylate.

Starting from 2g (5mmol) of **ethyl (R,S)-Benzenesulfonylamino-(1-methyl-4-S-nitroso mercapto-piperidin-4-yl)-acetate** and 324 µL of methanesulfonic acid, 2.2g (Yield:88.4%) of the product of the example with an HPLC purity of the 88.6% were obtained.
**¹H -NMR (DMSO-d₆, 200 MHz):** 9.90-9.60 (s.b., SO₃H); 8.90 (d, NH); 7.76-7.53 (m, 5H); 4.55 (d, 1H, CH); 3.56 (q, 2H, OCH₂); 3.10-2.00 (m, 8H, piperidine); 2.79 (s, **NCH₃); 2.37 (s, 3H, CH₃SO₃H);** 0.83 (t, 3H, CH₃)
**¹³C-NMR (DMSO-d₆, 200 MHz):** 167.66 (C=O); 140.22 (1C); 132.87 (1C); 129.13 (2C); 126.65 (2C); 63.93 (1C, CH); 61.32 (OCH₂); 58.59 (1C, C4piperidine); 49.44 and 49.26 (2C, 2NCH₂); 42.44 (NCH₃); 39.77 (1C, CH₃SO₃H); 29.36 and 29.16 (2C, 2CH₂piperidine); 13.43 (1C, CH₃).

### Example 7f. (R,S)-4-(benzenesulfonylamino-ethoxycarbonyl-methyl)-1-methyl-4-S-nitrosomercapto-piperidinium nitrate.

Starting from 2g (5mmol) of **ethyl (R,S)-Benzenesulfonylamino-(1-methyl-4-S-nitroso mercapto-piperidin-4-yl)-acetate** and 380 µL of 60% nitric acid, 1.6g of the product of the example with an HPLC purity of 91.5% were obtained.
**¹H -NMR (DMSO-d₆, 200 MHz):** 9.80-9.50 (s.b., NO₃H); 8.90 (d, NH); 7.76-7.53 (m, 5H); 4.55 (d, 1H, CH); 3.56 (q, 2H, OCH₂); 3.10-2.40 (m, 8H, piperidine); 2.79 (s, NCH₃); 0.81 (t, 3H, CH₃)
**¹³C-NMR (DMSO-d₆, 200 MHz):** 167.65 (C=O); 140.22 (1C); 132.86 (1C); 129.11 (2C); 126.65 (2C); 63.84 (1C, CH); 61.29 (OCH₂); 58.51 (1C, C4piperidine); 49.45 and 49.30 (2C, 2NCH₂); 42.41 (NCH₃); 29.22 (2C, 2CH₂piperidine); 13.39 (1C, CH₃).

### Example 7g. (R,S)-4-(benzenesulfonylamino-ethoxycarbonyl-methyl)-1-methyl-4-S-nitrosomercapto-piperidinium sulfate.

Starting from 2g (5mmol) of **ethyl (R,S)-benzenesulfonylamino-(1-methyl-4-S-nitroso mercapto-piperidin-4-yl)-acetate** and 140 µL of 95% sulfuric acid, 2.2g of the product of the example with an HPLC purity of 84.7% were obtained.
**¹H -NMR (DMSO-d₆, 200 MHz):** 8.90-8.70 (s.b., NH); 7.80-7.50 (m, 5H); 4.59 (s.b., 1H, CH); 3.56 (q, 2H, OCH₂); 3.30-2.40 (m, 8H, piperidine); 2.53 (s, NCH₃); 0.82 (t, 3H, CH₃)
**¹³C-NMR (DMSO-d₆, 200 MHz):** 167.88 (C=O); 140.30 (1C); 132.78 (1C); 129.07 (2C); 126.64 (2C); 63.54 (1C, CH); 61.14 (OCH₂); 59.59 (1 C, C4piperidine); 49.86 and 49.73 (2C, 2NCH₂); 43.68 (NCH₃); 30.28 (2C, 2CH₂piperidine); 13.42 (1C, CH₃).

### Example 7h. (R,S)-4-(benzenesulfonylamino-ethoxycarbonyl-methyl)-1-methyl-4-S-nitrosomercapto-piperidinium bisulfate.

Starting from 2g (5mmol) of **ethyl (R,S)-benzenesulfonylamino-(1-methyl-4-S-nitroso mercapto-piperidin-4-yl)-acetate** and 280 µL of 95% sulfuric acid, 2.3g of the product of the example with HPLC purity of 89.7% were obtained.
**¹H -NMR (DMSO-d₆, 200 MHz):** 9.80-9.20 (s.b., H₂SO₄); 8.85 (d, NH); 7.80-7.50 (m, 5H); 4.56 (d, 1H, CH); 3.56 (q, 2H, OCH₂); 3.60-2.40 (m, 8H, piperidine); 2.80 (s, NCH₃); 0.82 (t, 3H, CH₃)
**¹³C-NMR (DMSO-d₆, 200 MHz):** 167.65 (C=O); 140.23 (1C); 132.86 (1C); 129.12 (2C); 126.65 (2C); 63.54 (1C, CH); 61.29 (OCH₂); 58.57 (1C, C4piperidine); 49.21 (2C, 2NCH₂); 43.32 (NCH₃); 29.24 (2C, 2CH₂piperidine); 13.41 (1C, CH₃).

### Example 7i. (R,S)-4-(benzenesulfonylamino-ethoxycarbonyl-methyl)-1-methyl-4-S-nitrosomercapto-piperidinium phosphate diacid.

Starting from 2g (5mmol) of **ethyl (R,S)-benzenesulfonylamino-(1-methyl-4-S-nitroso mercapto-piperidin-4-yl)-acetate** and 340 µL of 85% orthophosphoric acid, 2.3g of the product of the example with an HPLC purity of 89.8% were obtained.
**¹H -NMR (DMSO-d₆, 200 MHz):** 9.60-8.80 (s.b.,4H, 3H of H₃PO₄ and 1H of NH); 7.80-7.50 (m, 5H); 4.57 (d, 1H, CH); 3.60 (q, 2H, OCH₂); 3.20-2.40 (m, 8H, piperidine); 2.44 (s, NCH₃); 0.82 (t, 3H, CH₃)
**¹³C-NMR (DMSO-d₆, 200 MHz):** 167.93 (C=O); 140.34 (1C); 132.75 (1C); 129.07 (2C); 126.65 (2C); 63.57 (1C, CH); 61.14 (OCH₂); 59.91 (1C, C4piperidine); 49.75 and 49.64 (2C, 2NCH₂); 43.86 (NCH₃); 30.59 and 30.38 (2C, 2CH₂piperidine); 13.45 (1C, CH₃).

### Example 7j. (R,S)-4-(benzenesulfonylamino-ethoxycarbonyl-methyl)-1-methyl-4-S-nitrosomercapto-piperidinium lactate.

Starting from 2g (5mmol) of **ethyl (R,S)-benzenesulfonylamino-(1-methyl-4-S-nitroso mercapto-piperidin-4-yl)-acetate** and 406 µL of (±)lactic acid, 1.13g of the product of the example with an HPLC purity of 80.5% were obtained.
**¹H -NMR (DMSO-d₆, 200 MHz):** 8.90-8.20 (s.b., NH); 7.80-7.50 (m, 5H); 4.60 (s.b., 1H, CH); 3.95 (m, 1H, CH); 3.60 (q, 2H, OCH₂); 3.00-2.00 (m, 8H, piperidine); 2.29 (s, NCH₃); 1.30-1.00 (m, 3H, CH₃); 0.82 (t, 3H, CH₃)
**¹³C-NMR (DMSO-d₆, 200 MHz):** 176.47 (C=O); 168.01 (C=O); 140.35 (1C); 132.69 (1C); 129.02 (2C); 126.59 (2C); 65.83 (1C, CH); 63.57 (1C, CH); 60.98 (OCH₂); 60.39 (1C, C4piperidine); 50.16 (2C, 2NCH₂); 44.77 (NCH₃); 31.04 (2C, 2CH₂piperidine); 20.50 (1C, CH₃); 13.45 (1C, CH₃).

### Example 7k. (R,S)-4-(benzenesulfonylamino-ethoxycarbonyl-methyl)-1-methyl-4-S-nitrosomercapto-piperidinium citrate.

Starting from 2g (5mmol) of **ethyl (R,S)-benzenesulfonylamino-(1-methyl-4-S-nitroso mercapto-piperidin-4-yl)-acetate** and 0.96g of citric acid, 1.69g of the product of the example with an HPLC purity of 96.0% were obtained.
**¹H -NMR (DMSO-d₆, 200 MHz):** 10.40-9.20 (s.b., 3H, CO₂H); 8.90-8.60 (s.b., NH); 7.80-7.50 (m, 5H); 4.61 (s.b., 1H, CH); 3.59 (m, 2H, OCH₂); 3.30-2.20 (m, 15H, 8H, piperidine, 3H of NCH₃ and 4H CH₂ of citric acid); 0.81 (t, 3H, CH₃)
**¹³C-NMR (DMSO-d₆, 200 MHz):** 176.06 (C=O); 171.37 (2C=O); 167.86 (C=O); 140.30 (1C); 132.78 (1C); 129.08 (2C); 126.62 (2C); 71.763 (1C, C-OH); 63.47 (1C, CH); 61.12 (OCH₂); 59.51 (1C, C4piperidine); 49.81 and 49.70 (2C, 2NCH₂); 43.68 and 43.57 (2C, 2CH₂); 40.76 (NCH₃); 30.17 (2C, 2CH₂piperidine); 13.41 (1C, CH₃).

### Example 7l. (R,S)-4-(benzenesulfonylamino-ethoxycarbonyl-methyl)-1-methyl-4-S-nitrosomercapto-piperidinium salicylate.

Starting from 2g (5mmol) of **ethyl (R,S)-benzenesulfonylamino-(1-methyl-4-S-nitroso mercapto-piperidin-4-yl)-acetate** and 0.69g of salicylic acid, 1.32g of the product of the example with an HPLC purity of 90.4% were obtained.
**¹H -NMR (DMSO-d₆, 200 MHz):** 8.90-8.70 (s.b., NH); 7.80-7.50 (m, 6H); 7.30-7.20 (m, 1H); 6.80-6.60 (m, 2H); 4.61 (s.b., 1H, CH); 3.59 (m, 2H, OCH₂); 3.60-2.50 (m, 8H, piperidine); 2.70(s, 3H, NCH₃); 0.82 (t, 3H, CH₃)
**¹³C-NMR (DMSO-d₆, 200 MHz):** 172.89 (C=O); 167.72 (C=O); 161.95 (1C, C-OH, salicylic); 140.22 (1C); 132.78 (1C); 132.70 (1C, salicylic); 130.26 (1C, salicylic); 129.06 (2C); 126.64 (2C); 118.26 (1C, salicylic); 117.18 (1C, salicylic); 116.13 (1C, salicylic); 63.56 (1C, CH); 61.22 (OCH₂); 59.23 (1C, C4piperidine); 49.24 and 49.10 (2C, 2NCH₂); 42.57 (NCH₃); 29.84 and 29.10 (2C, 2CH₂piperidine); 13.40 (1C, CH₃).

### Example 8. Ethyl (R,S)-(1-methyl-4-S-nitrosomercapto-piperidin-4-yl)-(4-nitro-benzenesulfonylamino)-acetate

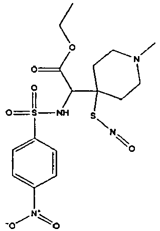

Yield last step: 46%. HPLC purity: 96.2%
**¹H -NMR (DMSO-d₆, 200 MHz):** 8.39 (d, J=8.8Hz, 2H); 8.01 (d, J=8.8Hz, 2H); 4.68 (s, 1H, CH); 3.66 (q, 2H, OCH₂); 3.00-2.20 (m, 8H, piperidine); 2.32 (s, NCH₃); 0.83 (t, 3H, CH₃).
**¹³C-NMR (DMSO-d₆, 200 MHz):** 167.79 (C=O); 149.68 (1C); 145.89 (1C); 128.29 (2C); 124.38 (2C); 63.75 (1C, CH); 61.26 (OCH₂); 60.27 (1C; C4piperidine); 50.12 and 50.01 (2C, 2NCH₂); 44.46 (NCH₃); 31.14 and 30.96 (2C, 2CH₂piperidine); 13.40 (1C, CH₃).

### Example 9. Ethyl (R,S)-(1-methyl-4-S-nitrosomercapto-piperidin-4-yl)-(4-methoxy-benzenesulfonylamino)-acetate

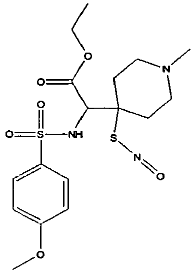

Yield last step: 92%. HPLC purity 97.7%
**¹H -NMR (DMSO-d₆, 200 MHz):** 8.55 (s.b., 1H, NH); 7.67 (d, J=7.8Hz, 2H); 7.05 (d, J=7.8Hz, 2H); 4.57 (s, 1H, CH); 3.80 (s, 3H, OCH₃); 3.62 (q, 2H, OCH₂); 2.80-2.00 (m, 8H, piperidine); 2.14 (s, NCH₃); 0.86 (t, 3H, CH₃).
**¹³C-NMR (DMSO-d₆, 200 MHz):** 168.37 (C=O); 162.42 (1C, C-O); 132.04 (1C, C-SO₂); 128.89 (2C); 114.09 (2C); 63.58 (1C, CH); 60.95 and 60.87 (OCH₂ + C4piperidine); 55.67 (1C, OCH₃); 50.70 and 50.64 (2C, 2NCH₂); 45.64 (NCH₃); 31.69 (2C, 2CH₂piperidine); 13.41 (1C, CH₃).

### Example 10. Ethyl (R,S)-(4-methyl-benzenesulfonylamino)-(1-methyl-4-S-nitrosomercapto-piperidin-4-yl)-acetate

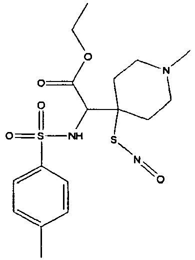

Yield last step: 93%. HPLC purity 94.8%
**¹H -NMR (DMSO-d₆, 200 MHz):** 8.65 (s.b., 1H, NH); 7.63 (d, J=8Hz, 2H); 7.34 (d, J=8Hz, 2H); 4.59 (s, 1H, CH); 3.62 (q, 2H, OCH₂); 2.80-2.00 (m, 8H, piperidine); 2.35 (s, 3H, CH₃); 2.14 (s, NCH₃); 0.84 (t, 3H, CH₃)
**¹³C-NMR (DMSO-d₆, 200 MHz):** 168.20 (C=O); 142.99 (1C, C-C); 137.50 (1C, C-SO₂); 129.36 (2C); 126.65 (2C); 63.53 (1C, CH); 60.88 and 60.86 (OCH₂ + C4piperidine); 50.63 and 50.56 (2C, 2NCH₂); 45.58 (NCH₃); 31.58 (2C, 2CH₂piperidine); 20.91 (1C, CH₃); 13.33 (1C, CH₃)

### Example 11. Ethyl (R,S)-(2-chloro-benzenesulfonylamino)-(1-methyl-4-S-nitrosomercapto-piperidin-4-yl)-acetate

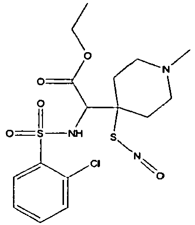

Yield last step: 80%. HPLC purity: 89.3 %
**¹H -NMR (DMSO-d₆, 200 MHz):** 8.90 (s.b., NH); 7.92 (d, 1H); 7.64-7.42 (m, 3H); 4.69 (s, 1H, CH); 3.80 (q, 2H, OCH₂); 2.80-2.00 (m, 8H, piperidine); 2.17 (s, NCH₃); 0.94 (t, 3H, CH₃).
**¹³C-NMR (DMSO-d₆, 200 MHz):** 168.23 (C=O); 137.37 (1C, C-Cl); 134.38 (1C, C-SO₂), 131.68 (1C); 130.90 (1C); 130.80 (1C); 127.58 (1C); 63.78 (1C, CH); 61.14 (OCH₂); 60.94 (1C, C4piperidine); 50.62 and 50.52 (2C, 2NCH₂); 45.52 (NCH₃); 31.56 (2C, 2CH₂piperidine); 13.52 (1C, CH₃).

### Example 12. Ethyl (R,S)-(3-chloro-benzenesulfonylamino)-(1-methyl-4-S-nitrosomercapto-piperidin-4-yl)-acetate

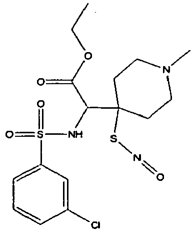

Yield last step: 76%. HPLC purity 99.9%
**¹H -NMR (DMSO-d₆, 200 MHz):** 7.76-7.53 (m, 4H); 4.63 (s.b., 1H, CH); 3.63 (q, 2H, OCH₂); 2.80-2.20 (m, 8H, piperidine); 2.25 (s, NCH₃); 0.86 (t, 3H, CH₃)
**¹³C-NMR (DMSO-d₆, 200 MHz):** 167.93 (C=O); 142.25 (1C); 133.69 (1C); 132.65 (1C); 131.15 (1C); 126.26 (1C); 125.27 (1C); 63.71 (1C, CH); 61.14 (OCH₂); 60.57 (C4piperidine); 50.34 and 50.25 (2C, 2NCH₂); 45.98 (NCH₃); 31.44 and 31.21 (2C, 2CH₂piperidine); 13.40 (1C, CH₃)

### Example 13. Ethyl (R,S)-(4-chloro-benzenesulfonylamino)-(1-methyl-4-S-nitrosomercapto-piperidin-4-yl)-acetate

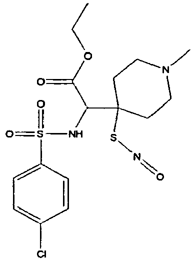

Yield last step: 88%. HPLC purity 95.8%
**¹H -NMR (DMSO-d₆, 200 MHz):** 7.76 (d, J=9Hz, 2H); 7.62 (d, J=9Hz, 2H); 4.63 (s, 1H, CH); 3.66 (q, 2H, OCH₂); 2.80-2.00 (m, 8H, piperidine); 2.16 (s, NCH₃); 0.86 (t, 3H, CH₃)
**¹³C-NMR (DMSO-d₆, 200 MHz):** 168.09 (C=O); 139.28 (1C, C-C1); 137.65 (1C, C-SO₂); 129.11 (2C); 128.60 (2C); 63.67 (1C, CH); 61.00 (OCH₂); 60.79 (1C, C4piperidine); 50.62 and 50.55 (2C, 2NCH₂); 45.53 (NCH₃); 31.72 (2C, 2CH₂piperidine); 13.36 (1C, CH₃)

By a process similar to that described in 1g, the corresponding hydrochloride is obtained: **(R,S)-4-(4-Chloro-benzenesulfonylamino-ethoxycarbonyl-methyl)-1-methyl-4-S-nitrosomercapto-piperidinium chloride**
**¹H -NMR (DMSO-d₆, 200 MHz):** 8.90 (s.b., 1H, NH); 7.76 (d, J=8Hz, 2H); 7.65 (d, J=8Hz, 2H); 4.58 (s.b., 1H, CH); 3.70 (q, 2H, OCH₂); 3.40-2.60 (m, 8H, piperidine); 2.70 (s, NCH₃); 0.88 (t, 3H, CH₃)
**¹³C-NMR (DMSO-d₆, 200 MHz):** 167.51 (C=O); 139.08 (1C, C-Cl); 137.71 (1C, C-SO₂); 129.19 (2C); 128.63 (2C); 63.00 (1C, CH); 61.42 (OCH₂); 58.92 (1C, C4piperidine); 49.04 and 48.88 (2C, 2NCH₂); 42.05 (NCH₃); 29.61 and 29.11 (2C, 2CH₂piperidine); 13.36 (1C, CH₃)

### Example 14. (R,S)-4-(2-Fluoro-benzenesulfonylamino)--(1-methyl-4-S-nitrosomercapto-piperidin-4-yl)-acetate

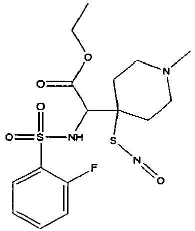

By a process similar to that described in 1g, the corresponding hydrochloride is obtained: **(R,S)-4-(2-Fluoro-benzenesulfonylamino-ethoxycarbonyl-methyl)-1-methyl-4-S-nitrosomercapto-piperidinium chloride**
Yield last step: 89%. HPLC purity 95.4%
**¹H -NMR (DMSO-d₆, 200 MHz):** 9.10 (s.b., NH); 7.80-7.65 (m, 2H); 7.44-7.30 (m, 2H); 4.68 (s, 1H, CH); 3.80 (q, 2H, OCH₂); 3.10 (s.b., 2H, piperidine); 2.75-2.40 (m, 6H, piperidine); 2.48 (s, NCH₃); 0.94 (t, 3H, CH₃)
**¹³C-NMR (DMSO-d₆, 200 MHz):** 167.88 (C=O); 160.60 and 155.54 (1C, C-F); 135.81 and 135.64 (1C, C-SO₂); 129.90 (1C); 128.11 and 127.83 (1C); 124.86 and 124.78 (1C); 117.29 and 116.87 (1C); 63.86 (1C, CH); 61.38 (OCH₂); 59.85 (1C, C4piperidine); 49.63 (2C, 2NCH₂); 43.47 (NCH₃); 30.48 and 30.21 (2C, 2CH₂piperidine); 13.50 (1C, CH₃)

### Example 15. Ethyl (R,S)-(4-fluoro-benzenesulfonylamino)-(1-methyl-4-S-nitrosomercapto-piperidin-4-yl)-acetate

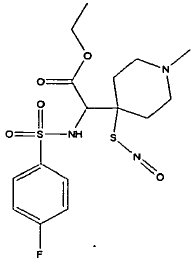

Yield last step: 95%. HPLC purity 99.4%
**¹H -NMR (DMSO-d₆, 200 MHz):** 8.80 (s.b., NH); 7.84-7.77 (m, 2H); 7.44-7.36 (m, 2H); 4.61 (s, 1H, CH); 3.66 (q, 2H, OCH₂); 2.75-2.00 (m, 8H, piperidine); 2.15 (s, NCH₃); 0.86 (t, 3H, CH₃)
**¹³C-NMR (DMSO-d₆, 200 MHz):** 168.15 (C=O); 166.76 and 161.77 (1C, C-F); 136.83 and 136.76 (1C, C-SO₂); 129.80 and 129.61 (2C); 116.37 and 115.92 (2C); 63.62 (1C, CH); 60.99 (OCH₂); 60.88 (1C, C4piperidine); 50.64 and 50.56 (2C, 2NCH₂); 45.61 (NCH₃); 31.73 and 31.65 (2C, 2CH₂piperidine); 13.42 (1C, CH₃)

By a process similar to that described in 1g, the corresponding hydrochloride is obtained: **(R,S)- 4-(4-Fluoro-benzenesulfonylamino-ethoxycarbonyl-methyl)-1-methyl-4-S-nitrosomercapto-piperidinium chloride**
**¹H -NMR (DMSO-d₆, 200 MHz):** 8.80 (s.b., NH); 7.81 (s.b., 2H); 7.40 (s.b., 2H); 4.56 (s.b., 1H, CH); 3.69 (q, 2H, OCH₂); 3.60-2.60 (m, 8H, piperidine); 2.68 (s, NCH₃); 0.88 (t, 3H, CH₃)
**¹³C-NMR (DMSO-d₆, 200 MHz):** 167.01 (C=O); 166.85 and 161.86 (1C, C-F); 136.70 and 136.64 (1C, C-SO₂); 129.91 and 129.72 (2C); 116.46 and 116.01 (2C); 63.66 (1C, CH); 61.44 (OCH₂); 59.02 (1C, C4piperidine); 49.10 and 48.92 (2C, 2NCH₂); 42.12 (NCH₃); 29.71 and 29.16 (2C, 2CH₂piperidine); 13.46 (1C, CH₃)

### Example 16. Ethyl (R,S)-(2,4-difluoro-benzenesulfonylamino)-(1-methyl-4-S-nitroso mercapto-piperidin-4-yl)-acetate

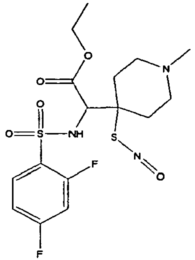

Yield last step: 52%. HPLC purity 98.4%
**¹H -NMR (DMSO-d₆, 200 MHz):** 7.78 (m, 1H); 7.42 (m, 1H); 7.18 (m, 1H); 4.64 (s, 1H, CH); 3.76 (q, 2H, OCH₂); 2.75-2.00 (m, 8H, piperidine); 2.15 (s, NCH₃); 0.95 (t, 3H, CH₃)
**¹³C-NMR (DMSO-d₆, 200 MHz):** 168.97 (C=O); 168.76 and 161.56 (1C, C-F); 161.30 and 156.45 (1C, C-F); 131.96 and 131.74 (1C, CH); 126.33 and 126.26 (1C, C-SO₂); 111.97 and 111.54 (1C, CH); 106.04, 105.51 and 104.99 (1C, C-H); 64.28 (1C, CH); 61.47 (OCH₂); 60.86 (1C, C4piperidine); 50.84 and 50.76 (2C, 2NCH₂); 45.71 (NCH₃); 31.87 and 31.81 (2C, 2CH₂piperidine); 13.57 (1C, CH₃)

By a process similar to that described in 1g the corresponding hydrochloride is obtained: **(R,S)-4-(2,4-Difluoro-benzenesulfonylamino-ethoxycarbonyl-methyl)-1-methyl-4-S-nitrosomercapto-piperidinium chloride**
**¹H -NMR (DMSO-d₆, 200 MHz):** 11.50 (s.b. 1H, HCl); 9.20 (s.b., NH); 7.82 (m, 1H); 7.52 (m, 1H); 7.24 (m, 1H); 4.60 (s, 1H, CH); 3.90 (q, 2H, OCH₂); 3.50-2.60 (m, 8H, piperidine); 2.71 (s, NCH₃); 0.99 (t, 3H, CH₃)
**¹³C-NMR (DMSO-d₆, 200 MHz):** 167.62 (C=O); 167.84; 167.62; 162.79 and 162.55 (1C, C-F); 161.60; 161.34; 156.49 and 156.23 (1C, C-F); 132.17 and 131.96 (1C, CH); 124.92 and 124.57 (1C, C-SO₂); 112.38 and 112.00 (1C, CH); 106.34; 105.83 and 105.31 (1C, C-H); 64.37 (1C, CH); 61.76 (OCH₂); 59.07 (1C, C4piperidine); 49.15 and 48.91 (2C, 2NCH₂); 42.01 (NCH₃); 29.87 and 28.94 (2C, 2CH₂piperidine); 13.57 (1C, CH₃)

### Example 17.

### a) Synthesis of (R,S)-4-(Ethoxycarbonyl-formylamino-methyl)-4-mercapto-1-methyl-piperidinium trifluoroacetate

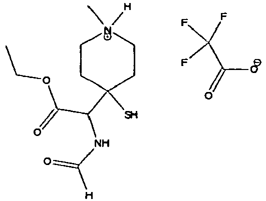

The product of the title is obtained starting from the product obtained in example 1b and proceeding as described in example 1e.
**¹H -NMR (DMSO-d₆, 200 MHz):** 10.20-9.80(s.b., 1H, CO₂H); 8.80-8.40 (s.b., 1H, NH); 8.13 (s, 1H, CHO); 4.60 (d, 1H, CH); 4.14 (q, 2H, OCH₂); 3.50-3.10 (m, 5H, 4Hpiperidine and SH); 2.79 (s, 3H, NCH₃); 2.10-1.70 (m, 4Hpiperidine); 1.21 (t, 3H, CH₃)
**¹³C-NMR (DMSO-d₆, 200 MHz):** 168.46 (C=O); 161.44 (C=O); 158.99 and 158.27 (CO₂H); 118.84 and 113.03 (CF3); 62.07 (1C, CH); 61.19 (OCH₂); 49.53 and 49.38 (2C, 2 NCH₂); 46.46 (1C, C4piperidine); 42.44 (NCH₃); 33.12 and 32.50 (2C, 2CH₂); 13.98 (1C, CH₃)

### b) Synthesis of Ethyl (R,S)-formylamino-(1-methyl-4-S-nitrosomercapto-piperidin-4-yl)-acetate

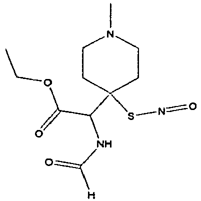

The product of the title is obtained starting from (R,S)-4-(Ethoxycarbonyl-formylamino-methyl)-4-mercapto-1-methyl-piperidinium trifluoroacetate and proceeding as described in example 1f.
**¹H -NMR (DMSO-d₆, 200 MHz):** 8.90 (d, NH); 8.09 (s, 1H, CHO); 5.35 (d, J=9.6Hz, 1H, CH); 4.05 (q, 2H, OCH₂); 2.80-2.00 (m, 8H,piperidine); 2.17 (s, 3H, NCH₃); 1.15 (t, 3H, CH₃)
**¹³C-NMR (DMSO-d₆, 200 MHz):** 168.77 (C=O); 161.38 (C=O); 61.22 (1C, C4piperidine); 60.98 (OCH₂); 57.94 (1C, CH); 50.72 and 50.62 (2C, 2NCH₂); 45.64 (NCH₃); 32.31 and 32.14 (2C, 2CH₂); 13.80 (1C, CH₃)

By a process similar to that described in 1g, the corresponding hydrochloride is obtained: **(R,S)-4-(Ethoxycarbonyl-formylamino-methyl)-1-methyl-4-S-nitrosomercapto-piperidinium chloride**
**¹³C-NMR (DMSO-d₆, 200 MHz):** 168.16 (C=O); 161.53 (C=O); 61.67 (OCH₂); 59.11(1C, CH); 58.48 (1C, C4piperidine); 48.94 (2C, 2NCH₂); 41.97 (NCH₃); 29.73 and 29.37 (2C, 2CH₂); 13.81 (1C, CH₃)

### Example 18.

### a) Synthesis of (R,S)-4-[Ethoxycarbonyl-(2,2.2-trifluoro-acetylamino)-methyl]-4-mercapto-1-methyl-piperidinium trifluoroacetate

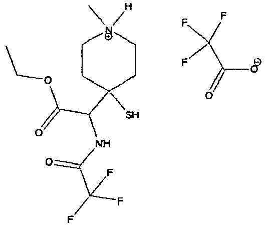

The product of the title is obtained starting from the product obtained in Example 1c and proceeding as described in Example 1e.
**¹H -NMR (DMSO-d₆, 200 MHz):** 9.84 (d, 1H, NH); 4.72 (d, 1H, CH); 4.17 (q, 2H, OCH₂); 3.70-3.10 (m, 5H, 4Hpiperidine and SH); 2.76 (s, 3H, NCH₃); 2.20-1.80 (m, 4Hpiperidine); 1.21 (t, 3H, CH₃)

### b) Synthesis of Ethyl (R,S)-(1-methyl-4-S-nitrosomercapto-piperidin-4-yl)-(2,2,2-trifluoro-acetylamino)-acetate

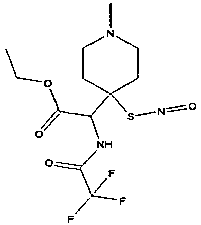

The product of the title is obtained starting from (R,S)-4-[Ethoxycarbonyl-(2,2,2-trifluoro-acetylamino)-methyl]-4-mercapto-1-methyl-piperidinium trifluoroacetate and proceeding as described in Example 1f.
Yield: 84% HPLC purity 95%
**¹H -NMR (CDCl₃, 200 MHz):** 7.10 (d, NH); 5.37 (d, 1H, CH); 4.15 (q, 2H, OCH₂); 2.90-2.20 (m, 8H,piperidine); 2.33 (s, 3H, NCH₃); 1.24 (t, 3H, CH₃)
**¹³C-NMR (CDCl₃, 200 MHz):** 167.89 (C=O); 159.00 (C=O); 118.42 and 112.69 (CF₃); 62.61 (OCH₂); 60.83(1C, C4piperidine); 60.54 (1C, CH); 50.99 (2C, 2NCH₂); 45.91 (NCH₃); 33.85 and 33.75 (2C, 2CH₂); 13.87 (1C, CH₃)

By a process similar to that described in 1g, the corresponding hydrochloride is obtained:
**¹H -NMR (CDCl₃, 200 MHz):** 12,20 (HCl); 7.94 (d, NH); 5.50 (d, 1H, CH); 4.22 (q, 2H, OCH₂); 3.90-2.20 (m, 8H,piperidine); 2.86 (s, 3H, NCH₃); 1.24 (t, 3H, CH₃)

### Example 19.

### a) Synthesis of Benzyl amino-[1-methyl-4-(4-methoxy-benzylsulfanyl)-piperidin-4-yl]-acetate

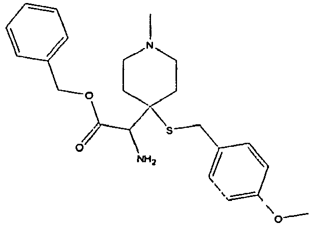

The product of the title is obtained starting from the product obtained in Example 1c by treatment with benzyl alcohol at 100°C.
**¹H -NMR (DMSO-d₆, 200 MHz):** 7.41-7.33 (m,5H); 7.10 (d, J=8.8Hz, 2H); 6.79 (d, J=8.8Hz, 2H); 5.14 (s, 2H, OCH₂Ph); 3.70 (s, 3H, OCH₃); 3.60-3.40 (m, CH + SCH₂); 2.50 (s.b., 2H, NH₂); 2.40-2.20 (m, 2H, piperidine); 2.15 (s, 3H, NCH₃); 2.10-1.50 (m, 6H, piperidine).
**¹³C-NMR (DMSO-d₆, 200 MHz):** 173.34 (C=O); 158.13 (C-O); 135.90 (1C, C-CH₂O); 130.15 (2C); 129.28 (1C C-CH₂S); 128.41-128.07 (5C); 113.73 (2C, CH); 65.67 (OCH₂); 61.93 (1C, CH); 54.99 (1C, OCH₃); 51.20 and 50.58 (3C, 2NCH₂ and C4piperidine); 45.86 (NCH₃); 30.29 and 30.15 (3C, 2CH₂ and S-CH₂)

### b) Synthesis of Benzyl (R,S)-acetylamino-(1-methyl-4-S-nitrosomercaptopiperidin-4-yl)-acetate

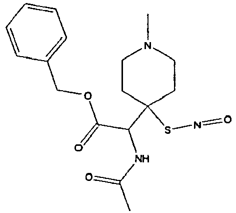

The product of the title is obtained starting from the product obtained in 19a and following the processes described in 1d to 1f.
**¹H -NMR (CDCl₃, 200 MHz):** 7.34-7.21 (m, 5H); 6.64 (d, NH); 5.50 (d, 1H, CH); 5.02 (s, 2H, OCH₂); 2.90-2.20 (m, 8H, piperidine); 2.36 (s, NCH₃); 2.08 (s, 3H, COCH₃)
**¹³C-NMR (CDCl₃, 200 MHz):** 170.23 (CO); 169.41 (C=O); 134.46 (1C); 128.49 (5C); 67.49 (OCH₂); 60.43 (C4piperidine); 59.78 (1C, CH); 51.02 and 50.87 (2C, 2NCH₂); 45.49 (NCH₃); 33.07 and 32.59 (2C, 2CH₂piperidine); 23.03 (1C, CH₃)

### Example 20.

### a) Synthesis of Ethyl amino-[1-methyl-4-(4-methoxy-benzylsufanil)-piperidin-4-yl]-acetate

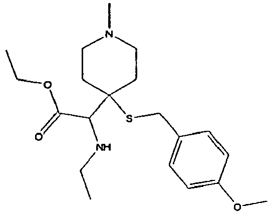

Starting from 10 g (28.37 mmol) of the product obtained in Example 1c by reductive amination with acetaldehyde (1.4 g; 31.2 mmol) and sodium cyanoborohydride (1.8 g; 30 mmol) in ethanol and after subjecting the crude reaction product to column chromatography on silica gel and eluting with dichloromethane, 5.4 g of the product of the title are obtained after concentrating to dryness.
**¹H -NMR (DMSO-d₆, 200 MHz):** 7.20 (d, J=8.4Hz, 2H); 6.85 (d, J=8.8Hz, 2H); 4.15 (q, 2H, OCH₂); 3.72 (s, 3H, OCH₃); 3.62 (d, 2H, SCH₂); 3.36-3.20 (m, 2H, NH + CH); 2.60-2.20 (m, 6H, 4Hpiperidine + NCH₂); 2.14 (s, 3H, NCH₃); 2.10-1.50 (m, 4H, piperidine); 1.23 (t, 3H, CH₃); 1.00 (t, 3H, CH₃)
**¹³C-NMR (DMSO-d₆, 200 MHz):** 173.21 (C=O); 158.17 (C-O); 130.09 (2C, CH); 129.31 (1C, C-CH₂); 113.77 (2C, CH); 68.72 (1C, CH); 59.91 (OCH₂); 55.00 (1C, OCH₃); 50.54 and 50.20 (3C, 2NCH₂ and C4piperidine); 45.92 (NCH₃); 42.48 (HN-CH₂); 30.77; 30.43 and 30.28 (3C, 2CH₂ and S-CH₂); 15.21(1C, CH₃); 14.20 (1C, CH₃)

### b) Synthesis of Ethyl (acetyl-ethyl-amino)-[1-methyl-4-(4-methoxy-benzylsulfanyl)-piperidin-4-yl]-acetate

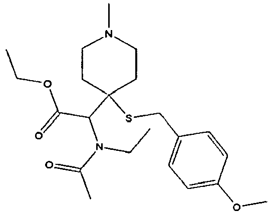

3.8 g (10 mmol) of the product obtained in a) are subjected to reflux in 50 ml of acetic anhydride for 16 hours. It is concentrated to dryness and the residue is dissolved in 50 ml of water and basified with 2N NaOH. It is extracted with 3x100 ml of dichloromethane. The extracts are dried and concentrated and the residue is chromatographed on silica gel. 1.7 g of the product of the title is obtained by eluting with 90/10/1% CH₂Cl₂/EtOH/NH₄OH
**¹H -NMR (DMSO-d₆, 200 MHz):** 7.20 (d, J=8.4Hz, 2H); 6.86 (d, J=8.8Hz, 2H); 5.02 (s.b., 1H, CH); 4.10 (m, 2H, OCH₂ + NH); 3.72 (s, 3H, OCH₃); 3.62 (s.b., 2H, SCH₂); 3.44 (q, 2H, NCH₂); 2.60-2.20 (m, 4H, 4Hpiperidine); 2.14 (s, 3H, NCH₃); 2.10 (s, 3H, COCH₃); 2.00-1.50 (m, 4H, piperidine); 1.20 (t, 3H, CH₃); 1.05 (t, 3H, CH₃)
¹³C-NMR (DMSO-d₆, 200 MHz): 171.29 (C=O); 168.54 (C=O); 158.25 (C-O); 130.18 (2C, CH); 128.96 (1C, C-CH₂); 113.85 (2C, CH); 60.62 (1C, CH); 60.27 (OCH₂); 55.02 (1C, OCH₃); 52.56 (1C, C4piperidine); 50.54 and 50.39 (2C, 2NCH₂); 45.76 (NCH₃); 42.57 (HN-CH₂); 30.73 and 30.57 (3C, 2CH₂ and S-CH₂); 21.39 (1C, COCH₃); 14.65 (1C, CH₃); 13.88 (1C, CH₃)

### c) Synthesis of Ethyl (R,S)-(acetyl-ethyl-amino)-(1-methyl-4-S-nitrosomercaptopiperidin-4-yl)-acetate

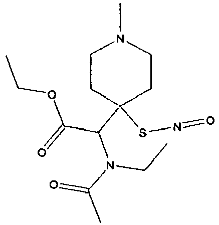

The product of the title is obtained starting from the product obtained in b) and following the processes described in 1e and 1f.
**¹H -NMR (CDCl₃, 200 MHz):** 5.28 (s.b., 1H, CH); 4.10 (q, 2H, OCH₂); 3.80-3.20 (m, 2H, NCH₂); 3.10-2.20 (m, 8H,piperidine); 2.39 (s, 3H, NCH₃); 2.17 (s, 3H, COCH₃); 1.28-1.14 (m, 6H, 2CH₃)
**¹³C-NMR (CDCl₃, 200 MHz):** 172.14 (C=O); 168.12 (C=O); 64.97 (1C, CH); 62.29 (1C, C4piperidine); 61.13 (OCH₂); 51.35 and 50.92 (2C, 2NCH₂); 50.25 (1C, NCH₂); 45.33 (NCH₃); 32.97 and 31.23 (2C, 2CH₂); 21.57 (1C, COCH₃); 14.33 (1C, CH₃); 13.89 (1C, CH₃)

The following products are obtained by following a process similar to that described in example 1 but substituting 1-methyl-4-piperidone with 1-ethyl-4-piperidone or 1-benzyl-4-piperidone in step a):

### Example 21. Synthesis of Ethyl (R,S)-acetylamino-(1-ethyl-4-S-nitrosomercaptopiperidin-4-yl)-acetate

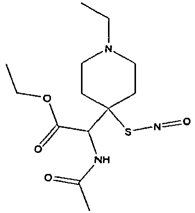

Yield last step: 51% ; HPLC purity: 89.3%
**¹H -NMR (DMSO-d₆, 200 MHz):** 8.59 (d, J=9.2Hz, NH); 5.36 (d, J=9.2Hz, 1H, CH); 4.05 (q, 2H, OCH₂); 3.00-2.10 (m, 10H, 8H, piperidine + NCH₂); 1.88 (s, 3H, COCH₃); 1.14-0.98 (m, 6H, 2CH₃)
**¹³C-NMR (DMSO-d₆, 200 MHz):** 169.68 (C=O); 169.18 (C=O); 61.45 (1C, C4piperidine); 61.02 (OCH₂); 59.23 (1C, CH); 51.36 (1C, NCH₂); 48.22 and 48.07 (2C, 2NCH₂); 31.93 and 31.42 (2C, 2CH₂); 22.18 (1C, COCH₃); 13.79 (1C, CH₃); 11.53 (1C, CH₃);

By a process similar to that described in 1g the corresponding hydrochloride is obtained: **(R,S)-(acetylamino-ethoxycarbonyl-methyl)-1-ethyl-4-S-nitrosomercaptopiperidinium chloride**
**¹H -NMR (DMSO-d₆, 200 MHz):** 11.30 (HCl); 8.66 (d, NH); 5.22 (d, J=9.2Hz, 1H, CH); 4.05 (q, 2H, OCH₂); 3.60-2.60 (m, 10H, 8H,piperidine + NCH₂); 1.86 (s, 3H, COCH₃); 1.14-0.98 (m, 6H, 2CH₃)
**¹³C-NMR (DMSO-d₆, 200 MHz):** 169.97 (C=O); 168.60 (C=O); 61.43 (1C); 59.74 (2C, OCH₂+CH); 50.57 (1C, NCH₂); 47.09 and 46.84 (2C, 2NCH₂); 29.28 and 29.22 (2C, 2CH₂); 22.20 (1C, COCH₃); 13.83 (1C, CH₃); 8.85 (1C, CH₃);

### Example 22. Synthesis of Ethyl (R,S)-(1-ethyl-4-S-nitrosomercapto-piperidin-4-yl)-[(pyridin-3-carbonyl)-am ino]-acetate

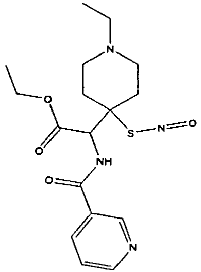

Yield last step: 76% ; HPLC purity: 91.3%

By a process similar to that described in 1g, the corresponding hydrochloride is obtained: **(R,S)-1-Ethyl-4-{ethoxycarbonyl-[(pyridin-3-carbonyl)-amino]-methyl}-4-S-nitrosomercapto-piperidinium chloride**
**¹H -NMR (DMSO-d₆, 200 MHz):** 11.30 (s.b., HCl); 9.39 (d., J=9Hz, NH); 8.98 (s, 1H, H2pyridine); 8.73 (d, J=6Hz, 1H, H6pyridine); 8.24 (d, J=7.2 Hz, 1H, H4pyridine); 7.53 (dd, J=6Hz, J₂=7.2Hz, 1H, H5pyridine); 5.52 (d, J=9Hz, 1H, CH); 4.11 (q, 2H, OCH₂); 3.80-2.60 (m, 10H, 8H piperidine +NCH₂); 1.27-1.02 (m, 6H, 2CH₃)
**¹³C-NMR (DMSO-d₆, 200 MHz):** 168.17 (C=O); 166.02 (C=O); 151.61 (1C); 148.35 (1C); 136.54 (1C); 129.42 (1C); 123.58 (1C); 61.56 (OCH₂); 60.31 (1C, CH); 59.78 (1C, C4piperidine); 50.58 (1C, NCH₂); 47.13 and 46.84 (2C, 2NCH₂); 29.37 and 28.98 (2C, 2CH₂piperidine); 13.84 (1C, CH₃); 8.87 (1C, CH₃)

### Example 23. Synthesis of Ethyl (R,S)-acetylamino-(1-benzyl-4-S-nitrosomercaptopiperidin-4-yl)-acetate

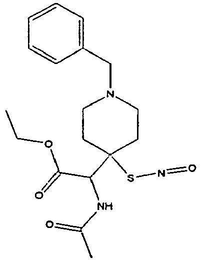

Yield last step: 86%; HPLC purity: 99.4%
**¹H -NMR (DMSO-d₆, 200 MHz):** 8.55 (d, J=9.2Hz, NH); 7.30-7.23 (m, 5H, phenyl); 5.36 (d, J=9.2Hz, 1H, CH); 4.01 (q, 2H, OCH₂); 3.35 (s, 2H, CH₂); 2.80-2.10 (m, 8H, 8Hpiperidine); 1.88 (s, 3H, COCH₃); 1.08 (t, 3H, CH₃)
**¹³C-NMR (DMSO-d₆, 200 MHz):** 169.93 (C=O); 169.28 (C=O); 138.05 (1C); 128.84 (2C); 128.21 (2C); 126.94 (1C); 61.88 (1C, C4piperidine); 61.72 (OCH₂); 61.00 (1C, NCH₂Ph); 59.30 (1C, CH); 48.68 and 48.55 (2C, 2NCH₂); 32.43 and 31.90 (2C, 2CH₂); 22.22 (1C, COCH₃); 13.80 (1C, CH₃)

By a process similar to that described in 1g, the corresponding hydrochloride is obtained: **(R,S)-4-(Acetylamino-ethyloxycarbonyl-methyl)-1-benzyl-4-S-nitrosomercapto-piperidinium chloride**
**¹H -NMR (DMSO-d₆, 200 MHz):** 11.60 (s.b., HCl); 8.66 (d, J=9.2Hz, NH); 7.63-7.37 (m, 5H, phenyl); 5.22 (d, J=9.2Hz, 1H, CH); 4.29 (d, J=4.6Hz, 2H, CH₂Ph); 4.05 (q, 2H, OCH₂); 3.40-2.60 (m, 8H, 8Hpiperidine); 1.85 (s, 3H, COCH₃); 1.08 (t, 3H, CH₃)
**¹³C-NMR (DMSO-d₆, 200 MHz):** 169.95 (C=O); 168.58 (C=O); 131.44 (2C); 129.70 (1C); 129.38 (1C); 128.68 (2C); 61.39 (OCH₂); 59.82 (1C, CH); 59.72 (1C, C4piperidine); 58.55 (1C, NCH₂Ph); 47.37 and 47.13 (2C, 2NCH₂); 29.05 (2C, 2CH₂); 22.20 (1C, COCH₃); 13.78 (1C, CH₃)

### Example 24. Synthesis of Ethyl (R,S)-(1-benzyl-4-S-nitrosomercapto-piperidin-4-yl)-[(pyridin-3-carbonyl)-amino]-acetate

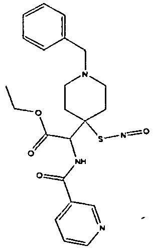

By a process similar to that described in 1g, the corresponding hydrochloride is obtained: **(R,S)-1-Benzyl-4-{ethoxycarbonyl-[(pyridin-3-carbonyl)-amino]-methyl}-4-S-nitrosomercapto-piperidinium chloride**
**¹H-NMR (DMSO-d₆, 200 MHz):** 11.60 (s.b., HCl); 9.37 (d., J=9Hz, NH); 8.97 (s, 1H, H2pyridine); 8.72 (d, J=4.4Hz, 1H, H6pyridine); 8.24 (d, J=7.8 Hz, 1H, H4pyridine); 7.60-7.42 (m, 6H, H5pyridine +5HPh); 5.52 (d, J=9Hz, 1H, CH); 4.31 (s.b., 2H, CH₂Ph); 4.09 (q, 2H, OCH₂); 3.80-2.80 (m, 8H, 8Hpiperidine); 1.07 (t, 3H, CH₃)

**¹³C-NMR (DMSO-d₆, 200 MHz):** 168.16 (C=O); 166.01 (C=O); 151.54 (1C); 148.33 (1C); 136.61 (1C); 131.46 (2C); 129.71 (1C); 129.44 (2C); 128.71 (2C); 123.59 (1C); 61.55 (OCH₂); 60.28 (1C, CH); 59.80 (1C, C4piperidine); 58.61 (1C, NCH₂); 47.36 and 47.20 (2C, 2NCH₂); 29.27 and 28.81 (2C, 2CH₂piperidine); 13.82 (1C, CH₃).

The following products are obtained by a process similar to that described in example 17 but starting from the corresponding N-ethyl or N-benzylpiperidine compound:

### Example 25. Synthesis of Ethyl (R,S)-(1-ethyl-4-S-nitrosomercapto-piperidin-4-yl)-formylamino-acetate

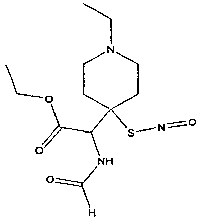

Yield last step: 82%; HPLC purity: 96.2%
**¹H -NMR (DMSO-d₆, 200 MHz):** 8.89 (d, J=9.4Hz, NH); 8.08 (s, 1H, CHO); 5.36 (d, J=9.4Hz, 1H, CH); 4.03 (q, 2H, OCH₂); 2.90-2.00 (m, 10H, 8H,piperidine + NCH₂); 1.06 (t, 3H, CH₃); 0.97 (t, 3H, CH₃)
**¹³C-NMR (DMSO-d₆, 200 MHz):** 168.76 (C=O); 161.38 (C=O); 61.52 (1C, C4piperidine); 61.20 (OCH₂); 57.81 (1C, CH); 51.43 (1C, NCH₂); 48.33 and 48.21 (2C, 2NCH₂); 32.34 and 32.07 (2C, 2CH₂); 13.78 (1C, CH₃); 11.91 (1C, CH₃);

### Example 26. Synthesis of Ethyl (R,S)-(1-benzyl-4-S-nitrosomercapto-piperidin-4-yl)-formylamino-acetate

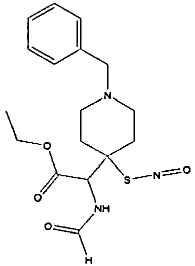

By a process similar to that described in 1g, the corresponding hydrochloride is obtained: **(R,S)-1-Benzyl-4-(ethyloxycarbonyl-formylamino-methyl)-4-S-nitrosomercapto-piperidinium chloride**
**¹H -NMR (DMSO-d₆, 200 MHz):** 11.60 (s.b., HCl); 8.98 (d, J=9.2Hz, NH); 8.06 (s, 1H, CHO); 7.60-7.39 (m, 5H, phenyl); 5.22 (d, J=9.2Hz, 1H, CH); 4.29 (d, J=4.4Hz, 2H, CH₂Ph); 4.07 (q, 2H, OCH₂); 3.40-2.60 (m, 8H, 8Hpiperidine); 1.09 (t, 3H, CH₃)
**¹³C-NMR (DMSO-d₆, 200 MHz):** 169.95 (C=O); 168.58 (C=O); 131.44 (2C); 129.70 (1C); 129.38 (1C); 128.68 (2C); 61.39 (OCH₂); 59.82 (1C, CH); 59.72 (1C, C4piperidine); 58.55 (1C, NCH₂Ph); 47.37 and 47.13 (2C, 2NCH₂); 29.05 (2C, 2CH₂); 22.20 (1C, COCH₃); 13.78 (1C, CH₃)

The following products are obtained by following a process similar to that described in example 1 but substituting ethyl isocyanoacetate with methyl isocyanoacetate in step a):

### Example 27. Synthesis of Methyl (R,S)-acetylamino-(1-methyl-4-S-nitrosomercapto-piperidin-4-yl)-acetate

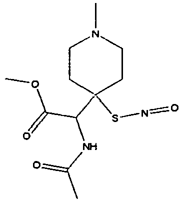

Yield last step: 50%; HPLC purity: 90.2%
**¹H -NMR (DMSO-d₆, 200 MHz):** 8.59 (d, J=9.2Hz, NH); 5.37 (d, J=9.2Hz, 1H, CH); 3.57 (s, 3H, OCH₃); 2.80-2.00 (m, 8H,piperidine); 2.17 (s, 3H, NCH₃); 1.88 (s, 3H, COCH₃);
**¹³C-NMR (DMSO-d₆, 200 MHz):** 169.80 (2C, 2C=O); 61.14 (1C, C4piperidine); 59.33 (1C, CH); 52.02 (OCH₃); 50.74 and 50.61 (2C, 2NCH₂); 45.62 (NCH₃); 32.13 and 31.84 (2C, 2CH₂); 22.14 (1C, COCH₃)

By a process similar to that described in 1g, the corresponding hydrochloride is obtained: **(R,S)-4-(Acetylamino-methyloxycarbonyl-methyl)-1-methyl-4-S-nitrosomercapto-piperidinium chloride**
**¹H -NMR (DMSO-d₆, 200 MHz):** 11.48 (s.b., HCl); 8.71 (d, J=9.2Hz, NH); 5.25 (d, J=9.2Hz, 1H, CH); 3.62 (s, 3H, OCH₃); 3.60-2.70 (m, 11H, 8Hpiperidine + NCH₃); 1.86 (s, 3H, COCH₃);
**¹³C-NMR (DMSO-d₆, 200 MHz):** 169.96 (1C, C=O); 169.08 (1C, C=O); 59.76 (1C, CH); 59.20 (1C, C4piperidine); 52.42 (OCH₃); 49.15 and 48.99 (2C, 2NCH₂); 41.95 (NCH₃); 29.42 and 29.22 (2C, 2CH₂); 22.18 (1C, COCH₃)

### Example 28. Synthesis of Methyl (R,S)-(1-methyl-4-S-nitrosomercapto-piperidin-4-yl)-[(pyridin-3-carbonyl)-amino]-acetate

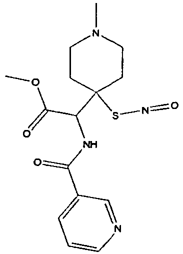

**¹H -NMR (DMSO-d₆, 200 MHz):** 9.20 (d., J=9Hz, NH); 8.91 (s, 1H, H2pyridine); 8.71 (d, J=6.2Hz, 1H, H6pyridine); 8.13 (m,, 1H, H4pyridine); 7.48 (m, 1H, H5pyridine); 5.64 (d, J=9Hz, 1H, CH); 3.63 (s, 3H, OCH₃); 2.80-2.10 (m, 8H, 8Hpiperidine); 2.17(s, 3H, NCH₃)
**¹³C-NMR (DMSO-d₆, 200 MHz):** 168.29 (C=O); 164.04 (C=O); 152.21 (1C); 148.82 (1C); 135.62 (1C); 128.16 (1C); 123.27 (1C); 61.38 (1C, C4piperidine); 60.24 (1C, CH); 52.29(OCH₃); 50.80 and 50.63 (2C, 2NCH₂); 45.63 (1C, NCH₃); 32.22 and 32.04 (2C, 2CH₂piperidine)

### Example 29. Methyl (R,S)-benzenesulfonylamino-(1-methyl-4-S-nitrosomercaptopiperidin-4-yl)-acetate

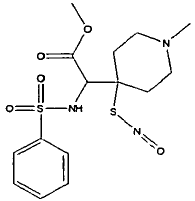

Yield last step: 50%; HPLC purity: 95.6%
**¹H -NMR (CDCl₃, 200 MHz):** 7.82-7.44 (m, 5H); 4.53 (s, 1H, CH); 3.25 (s, 3H, OCH₃); 2.80-2.20 (m, 8H, piperidine); 2.29 (s, NCH₃)
**¹³C-NMR (CDCl₃, 200 MHz):** 169.02 (C=O); 139.17 (1C); 132.98 (1C); 129.03 (2C); 127.38 (2C); 64.18 (1C, CH); 60.12 (1C, C4piperidine); 52.30 (1C, OCH₃); 51.02 and 50.91 (2C, 2NCH₂); 45.85 (NCH₃); 33.61 and 33.10 (2C, 2CH₂piperidine)

By a process similar to that described in 1g, the corresponding hydrochloride is obtained: **(R,S)-4-(Benzenesulfonylamino-methoxycarbonyl-methyl)-1-methyl-4-S-nitrosomercapto-piperidinium chloride**
**¹H -NMR (DMSO-d₆, 200 MHz):** 11.47 (s.b., 1H, HCl); 8.86 (d, J=10.4Hz, 1H, NH); 7.83-7.51 (m, 5H); 4.51 (d, J=10.4Hz, 1H, CH); 3.22 (s, 3H, OCH₃); 3.60-2.70 (m, 11H, 8H piperidine + NCH₃)
**¹³C-NMR (DMSO-d₆, 200 MHz):** 167.98 (C=O); 139.97 (1C); 132.76 (1C); 129.02 (2C); 126.59 (2C); 64.22 (1C, CH); 58.84 (1C, C4piperidine); 52.18 (1C, OCH₃); 49.08 and 48.85 (2C, 2NCH₂); 41.98 (NCH₃); 29.69 and 28.60 (2C, 2CH₂piperidine)

The following product is obtained by a process similar to that described in example 17 but starting from the corresponding compound with methyl ester instead of ethyl ester:

### Example 30. Synthesis of Methyl (R,S)-formylamino-(1-methyl-4-S-nitrosomercapto-piperidin-4-yl)-acetate

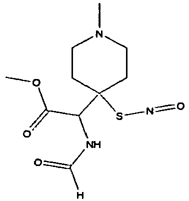

**¹H -NMR (DMSO-d₆, 200 MHz):** 8.91 (d, J=9.6Hz, NH); 8.08 (s, 1H, CHO); 5.38 (d, J=9.6Hz, 1H, CH); 3.60 (s, 3H, OCH₃); 2.90-2.10 (m, 11H, 8Hpiperidine + NCH₃)
**¹³C-NMR (DMSO-d₆, 200 MHz):** 168.47 (C=O); 161.19 (C=O); 60.88 (1C, C4piperidine); 57.85 (1C, CH); 54.88 (1C, OCH₃); 50.66 and 50.55 (2C, 2NCH₂); 45.48 (NCH₃); 32.13 (2C, 2CH₂piperidine)

### Example 31. Synthesis of Ethyl (R,S)-acetylamino-(4-nitrosomercapto-tetrahydrothiopyran-4-yl)-acetate

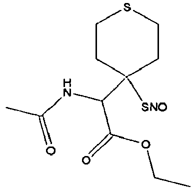

The product of the example is obtained by following a process similar to that described in examples 1a-1f and starting from the 4-tetrahydrothiopyranone.
**¹H -NMR (CDCl₃, 200 MHz):** 6.44 (d, NH); 5.42 (d, J=9.6Hz, 1H, CH); 4.10 (q, 2H, OCH₂); 3.20-2.40 (m, 8H, thiopyran); 2.13 (s, 3H, COCH₃); 1.17 (t, 3H, CH₃)
**¹³C-NMR (CDCl₃, 200 MH₂):** 170.13 (C=O); 169.32 (C=O); 62.36 (1C, C4thiopyran); 61.81 (1C, OCH₂); 59.58 (1C, CH); 34.78 and 34.38 (2C, 2CH₂); 23.62 and 23.56 (2C, 2CH₂); 22.99 (1C, COCH₃); 13.77 (1C, CH₃)

### Example 32. Synthesis of Ethyl (R,S)-benzenesulfonylamino-(4-nitrosomercaptotetrahydro-thiopyran-4-yl)-acetate

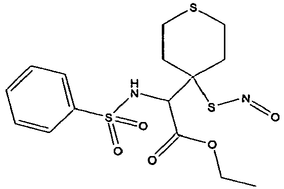

**¹³C-NMR (CDCl₃, 200 MHz):** 168.30 (C=O); 139.07 (1C); 132.90 (1C); 128.86 (2C); 127.07 (2C); 63.93 (1C, CH); 61.79 (1C, C4thiopyran); 61.50 (1C, OCH₂); 34.22 (2C, 2CH₂); 23.39 (2C, 2CH₂); 13.39 (1C, CH₃)

### Example 33:

### Synthesis of Ethyl-(R)-benzenesulfonylamino-(1-methyl-4-S-nitrosomercaptopiperidin-4-yl)-acetate and Ethyl-(S)-benzenesulfonylamino-(1-methyl-4-S-nitrosomercapto-piperidin-4-yl)-acetate.

### Example33a (R,S)-4-(benzenesulfonylamino-ethoxycarbonyl-methyl)4-mercapto-1-methyl-piperidinium trifluoroacetate

Starting from Ethyl (R,S)-amino-[1-methyl-4-(4-methoxy-benzylsulfanyl)-piperidin-4-yl]-acetate, obtained in 1c and substituting the acetyl chloride used in 1d with benzenesulfonyl chloride and following a process similar to those described in Examples 1d to 1e, the product of the example is obtained.
**¹H -NMR (DMSO-d₆, 200 MHz):** 10.00 (s.b., CO₂H); 8.68 (d., NH); 7.81-7.53 (m, 5H); 3.90 (d., 1H, CH); 3.69 (q, 2H, OCH₂); 3.60-1.60 (m, 8H, piperidine); 2.78 (s, NCH₃); 0.89 (t, 3H, CH₃)
**¹³C-NMR (DMSO-d₆, 200 MHz):** 167.89 (C=O); 159.90; 159.19; 158.48 and 157.77 (q, 1C, CO₂H); 140.49 (1C); 132.70 (1C); 129.04 (2C); 126.64 (2C); 124.73; 118.92; 103.10 and 107.29 (q, CF₃); 65.26 (1C, CH); 60.76 (OCH₂); 49.19 (2C, 2NCH₂); 46.30 (1C); 42.38 (NCH₃); 32.63 and 31.51 (2C, 2CH₂piperidine); 13.42 (1C, CH₃)

### Example33b (R)-4-(benzenesulfonylamino-ethoxycarbonyl-methyl)4-mercapto-1-methyl-piperidine and (S)-4-(benzenesulfonylamino-ethoxy carbonyl-methyl)4-mercapto-1-methyl-piperidine.

17 g of (R,S)- 4-(benzenesulfonylamino-ethoxycarbonyl-methyl)4-mercapto-1-methylpiperidinium trifluoroacetate was separated and isolated by Chiral Supercritical Fluid Chromatography (SFC) in the following conditions:
Column: 250 x 50mm CHIRALPAK^{R} AD-H
Mobil Phase: CO2/EtOH+ 1% DEA 80/20
Flow rate: 200mL/min
Detection UV 230nm
Temperature: 35°C

We obtained 2.58g of enantiomer E1 and 5.15g of enantiomer E2 of mercapto derivative.

These enantiomers were analyzed by the following HPLC method:
Column: 250 x 4,6 mm CHIRALPAK^{R} AD-H
Mobil phase: n-Heptane/Ethanol/Diethylamine 80/20/0.1
Flow rate: 1ml/min
Detection: UV 230nm
Temperature: 25°C

| | **Thiol Enantiomer E1** | **Thiol Enantiomer E2** |
|---|---|---|
| Retention time (min) | 11.8 | 18.6 |
| Quantity | 2.58g | 5.15g |
| Chemical purity (area% at 230nm) | > 99.0% | > 82.7% |
| Enantiomeric excess (%) | > 99.5% | 96.3% |

### Enantiomer E1:

**¹H -NMR (DMSO-d₆, 200 MHz):** 7.81-7.53 (m, 5H); 3.85 (s., 1H, CH); 3.69 (q, 2H, OCH₂); 2.60-1.40 (m, 8H, piperidine); 2.12 (s, NCH₃); 0.90 (t, 3H, CH₃)
**¹³C-NMR (DMSO-d₆, 200 MHz):** 168.35 (C=O); 140.67 (1C); 132.49 (1C); 128.94 (2C); 126.57 (2C); 65.49 (1C, CH); 60.45 (OCH₂); 50.59 and 50.48 (2C, 2NCH₂); 48.16 (1C); 45.75 (NCH₃); 35.34 and 34.42 (2C, 2CH₂piperidine); 13.61 (1C, CH₃)

### Example33c Ethyl-(R)-benzenesulfonylamino-(1-methyl-4-S-nitroso mercaptopiperidin-4-yl)-acetate and Ethyl-(S)-benzenesulfonylamino-(1-methyl-4-S-nitrosomercapto-piperidin-4-yl)-acetate and its corresponding hydrocloride salt were obtained by a process similar to that described in 7 and 7a.

The S-nitroso compound were analyised by the same HPLC method:

| | **S-nitroso Enantiomer E1** | **S-nitroso Enantiomer E2** |
|---|---|---|
| Retention time (min) | 8.5 | 13,4 |
| Chemical purity (area% at 230nm) | 93.5% | 98.6% |
| Enantiomeric excess (%) | 100% | 99.4% |

The ¹H- RMN and ¹³C-RMN of each enantiomer are the same to the ¹H- RMN and ¹³C-RMN described for the racemic mixtures in 7 and 7a

### Example 34. Stability study.

The stability of the S-nitrothiol compounds of the invention was determined by placing the compounds at 5°C and 25°C in a closed borosilicate glass vial. After 1, 2, 3, 4, and 6 months, the compounds were analysed by HPLC and detected at 201 and 345 nm.

Thus, compound of example 1g was stable for 2.5 months at 25°C and more than 10 months at 4°C; compound of example 7 as hydrochloride salt was stable at least 6 months at 25°C and more than 6 months at 4°C.

Therefore, it is clear that the compounds of the invention present a much better stability than the prior art S-nitrothiol compounds like GSNO (stable 1 day at 25°C), or N-acetyl-2-amino-2[4-(4-S-nitrosomercapto-1-methylpiperidin)]acetic acid [example 2 of EP 1157987] (stable 2 days at 25°C).

Note: it has been considered that a compound is no longer stable when it looses more than 5% of its initial purity value.

### B) PHARMACOLOGY

The activity of the products was assessed in experimental animals in accordance with the European Community Standards on the Care and Use of Laboratory Animals and approved by the Animal Care and Use Committee of local authorities. Two experimental models to test antithrombotic activity in rats were used: Arterio-Venous Shunt and Ferric chloride arterial thrombosis model. Additionally, the effect of the products on the systemic blood pressure was investigated.

### Example 35. Rat Arterial Thrombosis Model.

Fasted male Wistar rats (300-320 g; Janvier, Le Genest St. Isle, France) were anesthetized with sodium pentobarbital (60 mg/kg i.p.) and thermoregulated by use of blankets (Biosis homeothermic blanket control unit; Biosis, Spain). A segment (approximately 1-cm long) of the left carotid artery was exposed and fitted at the distal end with an appropriately sized Doppler flow probe. Thrombosis was induced by applying a 70% ferric chloride solution embedded patch onto the artery. Blood flow velocity was measured using a Doppler flowmeter (Transonic Systems, San Diego, CA) and data recorded using a system for acquisition of data (Acqk). Blood flow was recorded for 60 min post-lesion. When the flow declined to zero, the time in minutes to thrombus formation was noted. The protocol followed was originally described by Feuerstein GZ, et al Artherioscler. Thromb. Vasc. Biol. 1999, 19: 2554-2562 and modified by Kurz KD, et al Thromb. Res. 1990, 60:269-280.

The number of animals presenting no occlusion 30 min post-lesion were also noted. Products were dissolved in saline and were given as an i.v. infusion for 30 minutes. ED₅₀ for each product was calculated and results are shown in Table 1

**Table 1**

| Compound | ED₅₀ (µg/kg, i.v.) |
|---|---|
| GSNO | 18.1 |
| N-acetyl-2-amino-2[4-(4-S-nitrosomercapto-1-methylpiperidin)]acetic acid [example 2 of EP 1157987] | 4.1 |
| Example 1g | 9.1 |
| Example 7 (hydrochloride salt) | 56.4 |

| | |
|---|---|
| Anti-thrombotic effect of compounds compared with reference compound in rat arterial thrombosis model after i.v. administration for 30 minutes. Values shown are the mean values with 95% confidence limits. | |

### Example 36. Rat Arterio-Venous Shunt-Silk Thread Model.

Fasted male Wistar rats (300-320 g; Janvier, Le Genest St. Isle, France) were anesthetized with sodium pentobarbitone (60 mg/kg i.p.). An arterio-venous (AV) shunt was prepared according to the technique of Umetsu and Sanai (1978). Two 12 cm-long polyethylene tubes (0.85- and 1.27-mm i.d. and o.d., respectively) linked to a central part (6 cm-long; 1.14-mm i.d.) containing a 5-cm cotton thread and filled with heparised (25 u/ml) saline solution were placed between the right carotid artery and the left jugular vein. All products were given i.v through the jugular vein for 60 minutes. Shunt was placed 45 minutes after the initiation of the perfusion with the testing substances and removed at the end of the perfusion i.e. after 15 min of blood circulation. Cotton thread supporting the thrombus was extracted. The wet weight of the thrombus was determined and the % of anti-thrombotic protection was calculated and shown in Table 2.

**Table 2**

| Compound | Dose *(µg*/*kg, i.v.)* | Antithrombotic protection (%) |
|---|---|---|
| GSNO | 4.0 | 58 |
| N-acetyl-2-amino-2[4-(4-S-nitrosomercapto-1--methylpiperidin)]acetic acid [example 2 of EP 1157987] | 1.7 | 61 |
| Example 1g | 2.0 | 54 |
| Example 7 (hydrochloride salt) | 10.4 | 60 |

| | | |
|---|---|---|
| *Anti-thrombotic effect of compounds compared with reference compound in rat arterio-venous shunt model after i.v. administration.* | | |

### Example 37. Effect of nitrosothiols on systemic blood pressure.

Fasted male Wistar rats (300-320 g; Janvier, Le Genest St. Isle, France) were anesthetized with sodium pentobarbitone (60 mg/kg i.p.). The effect of treatments on systemic blood pressure was tested. Products dissolved in saline solution and were infused intravenously for 30 minutes at 0.5 ml/h. Dose of product given was 100 times the antithrombotic ED50 determined in the arterial thrombosis model. Basal blood pressure was measured before initiation of the treatment and was recorded throughout the treatment period using a pressure transducer connected to the carotid artery. The change in blood pressure was expressed as a % of change vs. basal values.

**Table 3**

| *Compound DE₅₀x100 (µg*/*kg, i.v.)* | ***Mean arterial pressure (mm Hg)*** | | ***Change* (%)** |
|---|---|---|---|
| | **Basal** | **After 30 min infusion** | |
| GSNO | 112.2±6.2 | 98.8±4.1 | - 12.0 |
| N-acetyl-2-amino-2[4-(4-S-nitrosomercapto-1-methylpiperidin)]acetic acid [example 2 of EP 1157987] | 99.6±2.1 | 95.1±2.4 | - 4.5 |
| Example 1g | 115.7 ± 8.3 | 113.6 ± 9.2 | - 1.8 |
| Example7 (hydrochloride salt) | 117.9 ± 10.2 | 118.8 ±11.1 | + 0.8 |

| | | | |
|---|---|---|---|
| *Effect of compounds on blood pressure after i.v.* administration | | | |

### Example 38. Effect of nitrosothiols on alfa-chymotrypsin-induced glaucoma

The method used was that described by Gabriele Campana, Claudio Bucolo, Giovanna Murari and Santi Spampinato in Pharmacol. Exp Therap Vol. 303, Issue 3, 1086-1094, December 2002

Animals were injected with intra muscular injection of dexamethasone at the dose rate of 10 mg/Kg body weight, to avoid immediate inflammation. Animals were anesthetized with ketamine (50 mg/kg IV) in combination with Dizepam.

Xylocaine (4%) was used for local anesthesia topically. A cannula attached to reservoir was inserted into the anterior chamber with the help of a 30 gauge needle to provide a hydrostatic pressure of 25 mmHg during injection of alpha-chymotrypsin. Then a second appropriately shaped 30 gauge needle was introduced near the pupil. Freshly prepared 150 units of alpha chymotrypsin prepared in 0.1 ml of sterile saline was irrigated through the cannula into the posterior chamber. Care was taken to prevent the contact of alpha chymotrypsin with corneal stroma. Both cannulas were carefully removed without significant loss of aqueous humor. Immediately after surgery the eye treated with Sofracort (Corticosteroid) to reduce the chances of fungal or microbial infection.

All the animals were kept under observation for 5 days and after these five days the intraocular pressure (IOP) was measured daily with a Schiontz type indentation tonometer using 5.5 gm weights and by compilation of readings the maximum period required to achieve a stable increase in IOP was determined. It was found that 2 - 3 weeks were sufficient to achieve a stable increase in IOP. IOP was measured after 15 days for 3 consecutive days, every morning (at same time) to assure stable IOP. The rejection criteria in our study was the removal of those rabbits from the study which showed IOP< 30 mmHg.

### Treatment:

All the animals were closely observed for the development of glaucoma. Thirty animals showing the symptoms of glaucoma and with the intra ocular pressure more than 30 mmHg were selected for the present investigation.

Compound (R,S)-4-(Acetylamino-ethoxycarbonyl-methyl)-1-methyl-4-S-nitroso-mercapto-piperidinium chloride (example 1g) was administered to a group of rabbits at a concentration of 0.05% by weight [0.05g in 100 mL of saline serum] by 3 drops instillation. For comparative data, the compound Timolol (Timoftol®) widely used for the treatment of glaucoma, was also administered to another group of rabbits at a concentration of 0.5% by weight.

The intraocular pressure measurements are included in the table 2 in mm Hg taken at 0, 1 and 2 h after 3 drops instillation.

| **Group** | **Treatment** | **0 hr** | **1 hr** | **2 hr** |
|---|---|---|---|---|
| Gr I | **Example 1g** | 34.33 ± 0.68 | 24.72 ± 0.47 | 23.86 ± 0.32 |
| Gr II | **Timolol 0.5% (Timoftol**® **0.5%)** | 34.45 ± 0.58 | 24.77 ± 0.26 | 24.54 ± 0.26 |
| control | **Normal saline** | 33.76 ± 0.54 | 33.21 ± 0.34 | 32.37 ± 0.31 |

These results point out that compound of example 1g of the present invention significantly reduces the intraocular pressure showing similar results to those obtained with a conventional compound even when using 10-fold lower concentrations.

## Claims

1. A compound of formula (I) 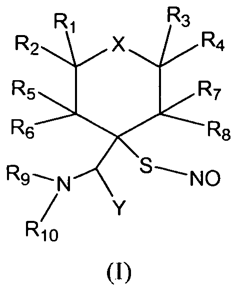 wherein
each of R₁, R₂, R₃, R₄, R₅, R₆, R₇ and R₈ are hydrogen;
R₉ is selected from the group consisting of hydrogen, C₁-C₆ alkyl and C₇-C₁₅ aralkyl;
R₁₀ is selected from the group consisting of hydrogen, C₁-C₆ alkyl, C₃-C₈ cycloalkyl, -C(=O)R₁₁ and -S(=O)₂R₁₁, wherein R₁₁ is selected from the group consisting of hydrogen, C₁-C₆ alkyl, C₂-C₆ alkenyl, C₃-C₈ cycloalkyl; C₂-C₆ alkynyl, C₆-C₁₀ aryl, C₇-C₁₀ aralkyl and 5- or 6-membered heterocyclyl;
Y represents a radical of formula -C(=O)OR₁₂, wherein R₁₂ is selected from the group consisting of C₁-C₆ alkyl, C₂-C₆ alkenyl, C₃-C₈ cycloalkyl, C₂-C₆ alkynyl, C₆-C₁₀ aryl, C₇-C₁₀ aralkyl and 5- or 6-membered heterocyclyl; and
X is selected from the group consisting of oxygen atom, sulphur atom, and - NR₁₃; wherein R₁₃ is selected from the group consisting of hydrogen, C₁-C₆ alkyl, C₂-C₆ alkenyl, C₃-C₈ cycloalkyl, C₂-C₆ alkynyl, C₆-C₁₀ aryl, C₇-C₁₀ aralkyl and 5- or 6-membered heterocyclyl;
or its diastereoisomers, enantiomers, salts or solvates thereof.

2. - Compound according to claim 1, wherein X is -NR₁₃, wherein R₁₃ is defined as in claim 1.

3. - Compound according to any of the previous claims, wherein R₉ is selected from the group consisting of hydrogen and C₁-C₆ alkyl.

4. - Compound according to any of the previous claims, wherein R₁₂ is a C₁-C₆ alkyl group.

5. - Compound according to any of the previous claims, wherein one of R₉ or R₁₀ is hydrogen.

6. - Compound according to claim 1 selected from the group consisting of:
- Ethyl (R,S)-acetylamino-(1-methyl-4-S-nitrosomercapto-piperidin-4-yl)-acetate;
- (R,S)-4-(Acetylamino-ethoxycarbonyl-methyl)-1-methyl-4-S-nitrosomercaptopiperidinium chloride;
- Ethyl (R,S)-heptanoylamino-(1-methyl-4-S-nitrosomercapto-piperidin-4-yl)-acetate;
- (R,S)-4-(Heptanoylamino-ethoxycarbonyl-methyl)-1-methyl-4-S-nitrosomercapto-piperidinium chloride;
- Ethyl (R,S)-benzoylamino-(1-methyl-4-S-nitrosomercapto-piperidin-4-yl)-acetate;
- (R,S)-4-(Benzoylamino-ethoxycarbonyl-methyl)-1-methyl-4-S-nitrosomercaptopiperidinium chloride;
- Ethyl (R,S)-(4-chloro-benzoylamino)-(1-methyl-4-S-nitrosomercapto-piperidin-4-yl)-acetate;
- (R,S)-4-[(4-Chloro-benzoylamino)-ethoxycarbonyl-methyl)]-1-methyl-4-S-nitrosomercapto-piperidinium chloride;
- Ethyl (R,S)-(2-chloro-benzoylamino)-(1-methyl-4-S-nitrosomercapto-piperidin-4-yl)-acetate;
- (R,S)-4-[(2-Chloro-benzoylamino)-ethoxycarbonyl-methyl)]-1-methyl-4-S-nitrosomercapto-piperidinium chloride;
- Ethyl (R,S)-(1-methyl-4-S-nitrosomercapto-piperidin-4-yl)-[(pyridin-3-carbonyl)-amino]-acetate;
- (R,S)-4-{Ethoxycarbonyl-[(pyridin-3-carbonyl)-amino]-methyl}-1-methyl-4-S-nitrosomercapto-piperidinium chloride;
- Ethyl (R,S)-benzenesulfonylamino-(1-methyl-4-S-nitrosomercapto-piperidin-4-yl)-acetate;
- (R,S)-4-(Benzenesulfonylamino-ethoxycarbonyl-methyl)-1-methyl-4-S-nitrosomercapto-piperidinium chloride;
- (R,S)-4-(benzenesulfonylamino-ethoxycarbonyl-methyl)-1-methyl-4-S-nitrosomercapto-piperidinium acetate;
- (R,S)-4-(benzenesulfonylamino-ethoxycarbonyl-methyl)-1-methyl-4-S-nitrosomercapto-piperidinium mesylate;
- (R,S)-4-(benzenesulfonylamino-ethoxycarbonyl-methyl)-1-methyl-4-S-nitrosomercapto-piperidinium nitrate;
- (R,S)-4-(benzenesulfonylamino-ethoxycarbonyl-methyl)-1-methyl-4-S-nitrosomercapto-piperidinium sulfate;
- (R,S)-4-(benzenesulfonylamino-ethoxycarbonyl-methyl)-1-methyl-4-S-nitrosomercapto-piperidinium bisulfate;
- (R,S)-4-(benzenesulfonylamino-ethoxycarbonyl-methyl)-1-methyl-4-S-nitrosomercapto-piperidinium phosphate diacid;
- (R,S)-4-(benzenesulfonylamino-ethoxycarbonyl-methyl)-1-methyl-4-S-nitrosomercapto-piperidinium lactate;
- (R,S)-4-(benzenesulfonylamino-ethoxycarbonyl-methyl)-1-methyl-4-S-nitrosomercapto-piperidinium citrate;
- (R,S)-4-(benzenesulfonylamino-ethoxycarbonyl-methyl)-1-methyl-4-S-nitrosomercapto-piperidinium salicylate;
- Ethyl (R,S)-(1-methyl-4-S-nitrosomercapto-piperidin-4-yl)-(4-nitro-benzene sulfonylamino)-acetate;
- Ethyl (R,S)-(4-methyl-benzenesulfonylamino)-(1-methyl-4-S-nitrosomercaptopiperidin-4-yl)-acetate;
- Ethyl (R,S)-(2-chloro-benzenesulfonylamino)-(1-methyl-4-S-nitrosomercaptopiperidin-4-yl)-acetate;
- Ethyl (R,S)-(3-chloro-benzenesulfonylamino)-(1-methyl-4-S-nitrosomercaptopiperidin-4-yl)-acetate;
- Ethyl (R,S)-(4-chloro-benzenesulfonylamino)-(1-methyl-4-S-nitrosomercaptopiperidin-4-yl)-acetate;
- (R,S)-4-(4-Chloro-benzenesulfonylamino-ethoxycarbonyl-methyl)-1-methyl-4-S-nitrosomercapto-piperidinium chloride;
- (R,S)-4-(2-Fluoro-benzenesulfonylamino-ethoxycarbonyl-methyl)-1-methyl-4-S-nitrosomercapto-piperidinium chloride;
- Ethyl (R,S)-(4-fluoro-benzenesulfonylamino)-(1-methyl-4-S-nitrosomercaptopiperidin-4-yl)-acetate;
- (R,S)- 4-(4-Fluoro-benzenesulfonylamino-ethoxycarbonyl-methyl)-1-methyl-4-S-nitrosomercapto-piperidinium chloride;
- Ethyl (R,S)-(2,4-difluoro-benzenesulfonylamino)-(1-methyl-4-S-nitroso mercapto-piperidin-4-yl)-acetate;
- (R,S)-4-(2,4-Difluoro-benzenesulfonylamino-ethoxycarbonyl-methyl)-1-methyl-4-S-nitrosomercapto-piperidinium chloride;
- Ethyl (R,S)-formylamino-(1-methyl-4-S-nitrosomercapto-piperidin-4-yl)-acetate;
- (R,S)-4-(Ethoxycarbonyl-formylamino-methyl)-1-methyl-4-S-nitrosomercaptopiperidinium chloride;
- Ethyl (R,S)-(1-methyl-4-S-nitrosomercapto-piperidin-4-yl)-(2,2,2-trifluoroacetylamino)-acetate;
- Benzyl (R,S)-acetylamino-(1-methyl-4-S-nitrosomercapto-piperidin-4-yl)-acetate;
- Ethyl (R,S)-(acetyl-ethyl-amino)-(1-methyl-4-S-nitrosomercapto-piperidin-4-yl)-acetate;
- Ethyl (R,S)-acetylamino-(1-ethyl-4-S-nitrosomercapto-piperidin-4-yl)-acetate;
- (R,S)-4-(Acetylamino-ethyloxycarbonyl-methyl)-1-ethyl-4-S-nitrosomercaptopiperidinium chloride;
- Ethyl (R,S)-(1-ethyl-4-S-nitrosomercapto-piperidin-4-yl)-[(pyridin-3-carbonyl)-amino]-acetate;
- (R,S)-1-Ethyl-4-{ethoxycarbonyl-[(pyridin-3-carbonyl)-amino]-methyl}-4-S-nitrosomercapto-piperidinium chloride;
- Ethyl (R,S)-acetylamino-(1-benzyl-4-S-nitrosomercapto-piperidin-4-yl)-acetate;
- (R,S)-4-(Acetylamino-ethyloxycarbonyl-methyl)-1-benzyl-4-S-nitrosomercaptopiperidinium chloride;
- Ethyl (R,S)-(1-benzyl-4-S-nitrosomercapto-piperidin-4-yl)-[(pyridin-3-carbonyl)-amino]-acetate;
- (R,S)-1-Benzyl-4-{ethoxycarbonyl-[(pyridin-3-carbonyl)-amino]-methyl}-4-S-nitrosomercapto-piperidinium chloride;
- Ethyl (R,S)-(1-ethyl-4-S-nitrosomercapto-piperidin-4-yl)-formylamino-acetate;
- (R,S)-1-Ethyl-4-(ethyloxycarbonyl-formylamino-methyl)-4-S-nitrosomercaptopiperidinium chloride;
- Ethyl (R,S)-(1-benzyl-4-S-nitrosomercapto-piperidin-4-yl)-formylamino-acetate;
- (R,S)-1-Benzyl-4-(ethyloxycarbonyl-formylamino-methyl)- 4-S-nitrosomercapto-piperidinium chloride;
- Methyl (R,S)-acetylamino-(1-methyl-4-S-nitrosomercapto-piperidin-4-yl)-acetate;
- (R,S)-4-(Acetylamino-methyloxycarbonyl-methyl)-1-methyl-4-S-nitrosomercapto-piperidinium chloride;
- Methyl (R,S)-(1-methyl-4-S-nitrosomercapto-piperidin-4-yl)-[(pyridin-3-carbonyl)-amino]-acetate;
- Methyl (R,S)-benzenesulfonylamino-(1-methyl-4-S-nitrosomercapto-piperidin-4-yl)-acetate;
- (R,S)-4-(Benzenesulfonylamino-methoxycarbonyl-methyl)-1-methyl-4-S-nitrosomercapto-piperidinium chloride;
- Methyl (R,S)-formylamino-(1-methyl-4-S-nitrosomercapto-piperidin-4-yl)-acetate;
- Ethyl (R,S)-acetylamino-(4-nitrosomercapto-tetrahydro-thiopyran-4-yl)-acetate; and
- Ethyl (R,S)-benzenesulfonylamino-(4-nitrosomercapto-tetrahydro-thiopyran-4-yl)-acetate;
or enantiomers or solvates thereof.

7. - Compound according to claim 6 selected from the group consisting of:
- Ethyl-(R)-benzenesulfonylamino-(1-methyl-4-S-nitroso mercapto-piperidin-4-yl)-acetate;
- Ethyl-(S)-benzenesulfonylamino-(1-methyl-4-S-nitrosomercapto-piperidin-4-yl)-acetate;
- (S)-4-(Benzenesulfonylamino-ethoxycarbonyl-methyl)-1-methyl-4-S-nitrosomercapto-piperidinium chloride; and
- (R)-4-(Benzenesulfonylamino-ethoxycarbonyl-methyl)-1-methyl-4-S-nitrosomercapto-piperidinium chloride.

8. - Method for the synthesis of a compound of formula (I) as defined in any of claims 1-7 comprising nitrosation of the thiol functionality of a compound of formula (III) 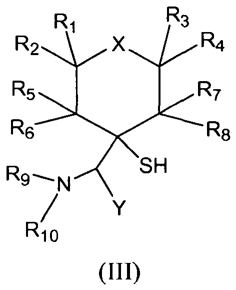 wherein
R₁, R₂, R₃, R₄, R₅, R₆, R₇, R₈, R₉, R₁₀, Y and X are as defined in claim 1.

9. - Method according to claim 8 comprising the following steps:
a) the removal of the Z group from a compound of formula (IV) 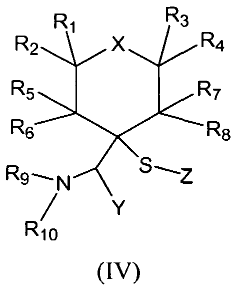 wherein
R₁, R₂, R₃, R₄, R₅, R₆, R₇, R₈, R₉, R₁₀, Y and X are as defined in claim 1, and Z is selected from the group consisting of unsubstituted alkyl, alkenyl, C₇-C₂₀ aralkyl, -Si(R')₃, -C(=O)R', wherein R' is selected from the group consisting of alkyl, alkenyl, alkynyl, aryl and aralkyl;
to obtain a compound of formula (III) as defined in claim 8; and
b) the nitrosation of the thiol functionality of said compound of formula (III) obtained in step a).

10. - Method according to any of claims 8 and 9 wherein at least one of the compounds of formula (III) and/or formula (IV) are subjected to a chiral separation.

11. - Compound of formula (III) 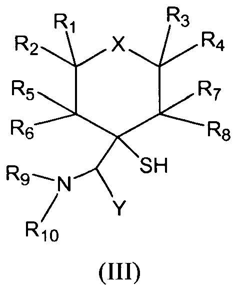 wherein
R₁, R₂, R₃, R₄, R₅, R₆, R₇, R₈, R₉, R₁₀, Y and X are as defined in claim 1;
or its diastereoisomers, enantiomers, salts or solvates thereof.

12. - Compound of formula (IV) 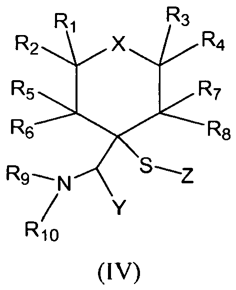 wherein
R₁, R₂, R₃, R₄, R₅, R₆, R₇, R₈, R₉, R₁₀, Y and X are as defined in claim 1, and Z is selected from the group consisting of unsubstituted alkyl, substituted or unsubstituted alkenyl, C₇-C₂₀ aralkyl, -Si(R')₃, -C(=O)R', wherein R' is selected from the group consisting of substituted or unsubstituted alkyl, substituted or unsubstituted alkenyl, substituted or unsubstituted alkynyl, substituted or unsubstituted aryl and substituted or unsubstituted aralkyl;
or its diastereoisomers, enantiomers, salts or solvates thereof.

13. - A compound as defined in any of claims 1-7 for use as a medicament.

14. - Pharmaceutical composition comprising a compound as defined in any of claims 1-7 and a pharmaceutically acceptable carrier.

15. - A compound as defined in any of claims 1-7 for use in the treatment and/or prophylaxis of a NO mediated conditions.

16. - A compound as defined in any of claims 1-7 for use in the treatment and/or prophylaxis of a condition selected from the group consisting of platelet dysfunction, endocrine, metabolic, cardiovascular, inflammatory, genitourinary, digestive, dermatological, neuronal, central nervous system related, neurodegenerative diseases, mental disorders, cognitive disorders, ophthalmological disease, respiratory and infectious conditions; hypertension, thrombosis, thromboembolic processes, vascular or trauma inflammation, complications associated to diabetes, ischemia-reperfusion, carotid endarterectomy and coronary bypass, graft rejections, in percutaneous coronary interventions, as vascular tone modulator, infant respiratory distress syndrome, asthma, pulmonary hypertension, bronchopulmonary dysplasia, cystic fibrosis, infectious diseases caused by bacteria, viruses and protozoa, Leishmaniasis, Trypanosomiasis, inhibition of AIDS virus replication, gastrointestinal tract motor diseases, inflammatory bowel disease, achalasia, diseases of the gall-bladder and the sphincter of Oddi, in cholangiopancreotography, liver failure, hepatic fibrosis, hepatic cirrhosis, portal hypertension, genitourinary tract diseases and erectile dysfunction, preeclampsia, endometrial hyperplasia, uterine smooth muscle proliferation, myometrium tumors, skin ulcers, ulcers related to diabetes, glaucoma, Alzheimer's disease, pain and fibromyalgias.

## Patentansprüche

1. Verbindung der Formel (I): 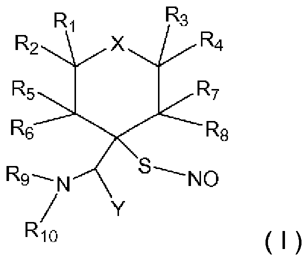 worin:
jedes von R₁, R₂, R₃, R₄, R₅, R₆, R₇ und R₈ Wasserstoff ist;
R₉ aus der Gruppe bestehend aus Wasserstoff, C₁₋₆-Alkyl und C₇₋₁₅-Aralkyl ausgewählt ist;
R₁₀ aus der Gruppe bestehend aus Wasserstoff, C₁₋₆-Alkyl, C₃₋₈-Cycloalkyl, -C(=O)R₁₁ und -S(=O)₂R₁₁, worin R₁₁ aus der Gruppe bestehend aus Wasserstoff, C₁₋₆-Alkyl, C₂₋₆-Alkenyl und C₃₋₈-Cycloalkyl; C₂₋₆-Alkinyl, C₆₋₁₀-Aryl, C₇₋₁₀-Aralkyl und 5- oder 6-gliedrigem Heterocyclyl ausgewählt ist;
Y einen Rest der Formel -C(=O)OR₁₂ darstellt, worin R₁₂ aus der Gruppe bestehend aus C₁₋₆-Alkyl, C₂₋₆-Alkenyl, C₃₋₈-Cycloalkyl, C₂₋₆-Alkinyl, C₆₋₁₀-Aryl, C₇₋₁₀-Aralkyl und 5- oder 6-gliedrigem Heterocyclyl ausgewählt ist; und
X aus der Gruppe bestehend aus einem Sauerstoffatom, Schwefelatom und -NR₁₃ ausgewählt ist, worin R₁₃ aus der Gruppe bestehend aus Wasserstoff, C₁₋₆-Alkyl, C₂₋₆-Alkenyl, C₃₋₈-Cycloalkyl, C₂₋₆-Alkinyl, C₆₋₁₀-Aryl, C₇₋₁₀-Aralkyl und 5- oder 6-gliedrigem Heterocyclyl ausgewählt ist;
oder Diastereoisomere, Enantiomere, Salze oder Solvate davon.

2. Verbindung gemäss Anspruch 1, worin X -NR₁₃ ist, worin R₁₃ wie in Anspruch 1 definiert ist.

3. Verbindung gemäss irgendeinem der vorhergehenden Ansprüche, worin R₉ aus der Gruppe bestehend aus Wasserstoff und C₁₋₆-Alkyl ausgewählt ist.

4. Verbindung gemäss irgendeinem der vorhergehenden Ansprüche, worin R₁₂ eine C₁₋₆-Alkylgruppe ist.

5. Verbindung gemäss irgendeinem der vorhergehenden Ansprüche, worin eines von R₉ oder R₁₀ Wasserstoff ist.

6. Verbindung gemäss Anspruch 1, ausgewählt aus der Gruppe bestehend aus:
Ethyl-(R,S)-acetylamino-(1-methyl-4-S-nitrosomercaptopiperidin-4-yl)-acetat;
(R,S)-4-(Acetylamino-ethoxycarbonyl-methyl)-1-methyl-4-S-nitrosomercapto-piperidiniumchlorid;
Ethyl-(R,S)-heptanoylamino-(1-methyl-4-S-nitrosomercapto-piperidin-4-yl)-acetat;
(R,S)-4-(Heptanoylamino-ethoxycarbonyl-methyl)-1-methyl-4-S-nitrosomercapto-piperidiniumchlorid;
Ethyl-(R,S)-benzoylamino-(1-methyl-4-S-nitrosomercapto-piperidin-4-yl)-acetat;
(R,S)-4-(Benzoylamino-ethoxycarbonyl-methyl)-1-methyl-4-S-nitrosomercapto-piperidiniumchlorid;
Ethyl-(R,S)-(4-chlor-benzoylamino)-(1-methyl-4-S-nitrosomercapto-piperidin-4-yl)-acetat;
(R,S)-4-[(4-Chlor-benzoylamino)-ethoxycarbonylmethyl)]-1-methyl-4-S-nitrosomercapto-piperidiniumchlorid;
Ethyl-(R,S)-(2-chlor-benzoylamino)-(1-methyl-4-S-nitrosomercapto-piperidin-4-yl)-acetat;
(R,S)-4-[(2-Chlor-benzoylamino)-ethoxycarbonylmethyl)]-1-methyl-4-S-nitrosomercapto-piperidiniumchlorid;
Ethyl-(R,S)-(1-methyl-4-S-nitrosomercapto-piperidin-4-yl)-[(pyridin-3-carbonyl)-amino]-acetat;
(R,S)-4-{Ethoxycarbonyl-[(pyridin-3-carbonyl)-amino]-methyl)-1-methyl-4-S-nitrosomercapto-piperidiniumchlorid;
Ethyl-(R,S)-benzolsulfonylamino-(1-methyl-4-S-nitrosomercapto-piperidin-4-yl)-acetat;
(R,S)-4-(Benzolsulfonylamino-ethoxycarbonyl-methyl)-1-methyl-4-S-nitrosomercapto-piperidiniumchlorid;
(R,S)-4-(Benzolsulfonylamino-ethoxycarbonyl-methyl)-1-methyl-4-S-nitrosomercapto-piperidiniumacetat;
(R,S)-4-(Benzolsulfonylamino-ethoxycarbonyl-methyl)-1-methyl-4-S-nitrosomercapto-piperidiniummesylat;
(R,S)-4-(Benzolsulfonylamino-ethoxycarbonyl-methyl)-1-methyl-4-S-nitrosomercapto-piperidiniumnitrat;
(R,S)-4-(Benzolsulfonylamino-ethoxycarbonyl-methyl)-1-methyl-4-S-nitrosomercapto-piperidiniumsulfat;
(R,S)-4-(Benzolsulfonylamino-ethoxycarbonyl-methyl)-1-methyl-4-S-nitrosomercapto-piperidiniumbisulfat;
(R,S)-4-(Benzolsulfonylamino-ethoxycarbonyl-methyl)-1-methyl-4-S-nitrosomercapto-piperidiniumphosphatdisäure;
(R,S)-4-(Benzolsulfonylamino-ethoxycarbonyl-methyl)-1-methyl-4-S-nitrosomercapto-piperidiniumlactat;
(R,S)-4-(Benzolsulfonylamino-ethoxycarbonyl-methyl)-1-methyl-4-S-nitrosomercapto-piperidiniumcitrat;
(R,S)-4-(Benzolsulfonylamino-ethoxycarbonyl-methyl)-1-methyl-4-S-nitrosomercapto-piperidiniumsalicylat;
Ethyl-(R,S)-(1-methyl-4-S-nitrosomercapto-piperidin-4-yl)-(4-nitro-benzolsulfonylamino)-acetat;
Ethyl-(R,S)-(4-methyl-benzolsulfonylamino)-(1-methyl-4-S-nitrosomercapto-piperidin-4-yl)-acetat;
Ethyl-(R,S)-(2-chlor-benzolsulfonylamino)-(1-methyl-4-S-nitrosomercapto-piperidin-4-yl)-acetat;
Ethyl-(R,S)-(3-chlor-benzolsulfonylamino)-(1-methyl-4-S-nitrosomercapto-piperidin-4-yl)-acetat;
Ethyl-(R,S)-(4-chlor-benzolsulfonylamino)-(1-methyl-4-S-nitrosomercapto-piperidin-4-yl)-acetat;
(R,S)-4-(4-Chlor-benzolsulfonylamino-ethoxycarbonylmethyl)-1-methyl-4-S-nitrosomercapto-piperidiniumchlorid;
(R,S)-4-(2-Fluor-benzolsulfonylamino-ethoxycarbonylmethyl)-1-methyl-4-S-nitrosomercapto-piperidiniumchlorid;
Ethyl-(R,S)-(4-fluor-benzolsulfonylamino)-(1-methyl-4-S-nitrosomercapto-piperidin-4-yl)-acetat;
(R,S)-4-(4-Fluor-benzolsulfonylamino-ethoxycarbonylmethyl)-1-methyl-4-S-nitrosomercapto-piperidiniumchlorid;
Ethyl-(R,S)-(2,4-difluor-benzolsulfonylamino)-(1-methyl-4-S-nitrosomercapto-piperidin-4-yl)-acetat;
(R,S)-4-(2,4-Difluor-benzolsulfonylaminoethoxycarbonyl-methyl)-1-methyl-4-S-nitrosomercaptopiperidiniumchlorid;
Ethyl-(R,S)-formylamino-(1-methyl-4-S-nitrosomercaptopiperidin-4-yl)-acetat;
(R,S)-4-(Ethoxycarbonyl-formylamino-methyl)-1-methyl-4-S-nitrosomercapto-piperidiniumchlorid;
Ethyl-(R,S)-(1-methyl-4-S-nitrosomercapto-piperidin-4-yl)-(2,2,2-trifluor-acetylamino)-acetat;
Benzyl-(R,S)-acetylamino-(1-methyl-4-S-nitrosomercapto-piperidin-4-yl)-acetat;
Ethyl-(R,S)-(acetyl-ethyl-amino)-(1-methyl-4-S-nitrosomercapto-piperidin-4-yl)-acetat;
Ethyl-(R,S)-acetylamino-(1-ethyl-4-S-nitrosomercaptopiperidin-4-yl)-acetat;
(R,S)-4-(Acetylamino-ethyloxycarbonyl-methyl)-1-ethyl-4-S-nitrosomercapto-piperidiniumchlorid;
Ethyl-(R,S)-(1-ethyl-4-S-nitrosomercapto-piperidin-4-yl)-[(pyridin-3-carbonyl)-amino]-acetat;
(R,S)-1-Ethyl-4-{ethoxycarbonyl-[(pyridin-3-carbonyl)-amino]-methyl}-4-S-nitrosomercaptopiperidiniumchlorid;
Ethyl-(R,S)-acetylamino-(1-benzyl-4-S-nitrosomercaptopiperidin-4-yl)-acetat;
(R,S)-4-(Acetylamino-ethyloxycarbonyl-methyl)-1-benzyl-4-S-nitrosomercapto-piperidiniumchlorid;
Ethyl-(R,S)-(1-benzyl-4-S-nitrosomercapto-piperidin-4-yl)-[(pyridin-3-carbonyl)-amino]-acetat;
(R,S)-1-Benzyl-4-{ethoxycarbonyl-[(pyridin-3-carbonyl)-amino]-methyl}-4-S-nitrosomercaptopiperidiniumchlorid;
Ethyl-(R,S)-(1-ethyl-4-S-nitrosomercapto-piperidin-4-yl)-formylamino-acetat;
(R,S)-1-Ethyl-4-(ethyloxycarbonyl-formylamino-methyl)-4-S-nitrosomercapto-piperidiniumchlorid;
Ethyl-(R,S)-(1-benzyl-4-S-nitrosomercapto-piperidin-4-yl)-formylamino-acetat;
(R,S)-1-Benzyl-4-(ethyloxycarbonyl-formylaminomethyl)-4-S-nitrosomercapto-piperidiniumchlorid;
Methyl-(R,S)-acetylamino-(1-methyl-4-S-nitrosomercapto-piperidin-4-yl)-acetat;
(R,S)-4-(Acetylamino-methyloxycarbonyl-methyl)-1-methyl-4-S-nitrosomercapto-piperidiniumchlorid;
Methyl-(R,S)-(1-methyl-4-S-nitrosomercapto-piperidin-4-yl)-[(pyridin-3-carbonyl)-amino]-acetat;
Methyl-(R,S)-benzolsulfonylamino-(1-methyl-4-S-nitrosomercapto-piperidin-4-yl)-acetat;
(R,S)-4-(Benzolsulfonylamino-methoxycarbonyl-methyl)-1-methyl-4-S-nitrosomercapto-piperidiniumchlorid;
Methyl-(R,S)-formylamino-(1-methyl-4-S-nitrosomercapto-piperidin-4-yl)-acetat;
Ethyl-(R,S)-acetylamino-(4-nitrosomercapto-tetrahydrothiopyran-4-yl)-acetat; und
Ethyl-(R,S)-benzolsulfonylamino-(4-nitrosomercaptotetrahydro-thiopyran-4-yl)-acetat;
oder Enantiomere oder Solvate davon.

7. Verbindung gemäss Anspruch 6, ausgewählt aus der Gruppe bestehend aus:
Ethyl-(R)-benzolsulfonylamino-(1-methyl-4-S-nitrosomercapto-piperidin-4-yl)-acetat;
Ethyl-(S)-benzolsulfonylamino-(1-methyl-4-S-nitrosomercapto-piperidin-4-yl)-acetat;
(S)-4-(Benzolsulfonylamino-ethoxycarbonyl-methyl)-1-methyl-4-S-nitrosomercapto-piperidiniumchlorid; und
(R)-4-(Benzolsulfonylamino-ethoxycarbonyl-methyl)-1-methyl-4-S-nitrosomercapto-piperidiniumchlorid.

8. Verfahren zur Synthese einer Verbindung der Formel (I) gemäss irgendeinem der Ansprüche 1 bis 7, umfassend die Nitrosierung der Thiolfunktionalität einer Verbindung der Formel (III): 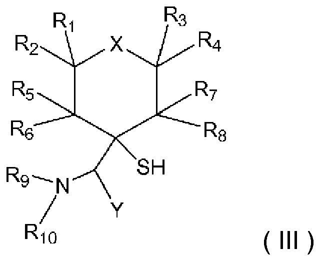 worin R₁, R₂, R₃, R₄, R₅, R₆, R₇, R₈, R₉, R₁₀, Y und X wie in Anspruch 1 definiert sind.

9. Verfahren gemäss Anspruch 8, umfassend die nachstehenden Schritte:
(a) Entfernen der Z-Gruppe von einer Verbindung der Formel (IV): 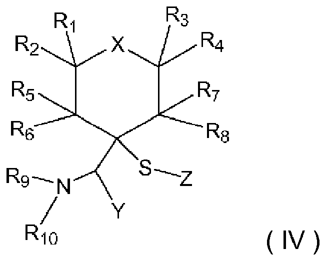 worin R₁, R₂, R₃, R₄, R₅, R₆, R₇, R₈, R₉, R₁₀, Y und X wie in Anspruch 1 definiert sind und Z aus der Gruppe bestehend aus unsubstituiertem Alkyl, Alkenyl, C₇₋₂₀-Aralkyl, -Si(R')₃ und -C(=O)R' ausgewählt ist, worin R' aus der Gruppe bestehend aus Alkyl, Alkenyl, Alkinyl, Aryl und Aralkyl ausgewählt ist;
um eine Verbindung der Formel (III) gemäss Anspruch 8 zu erhalten; und
(b) Nitrosierung der Thiolfunktionalität der in Schritt (a) erhaltenen Verbindung der Formel (III).

10. Verfahren gemäss irgendeinem der Ansprüche 8 und 9, wobei mindestens eine der Verbindungen der Formel (III) und/oder der Formel (IV) einer chiralen Trennung unterworfen wird.

11. Verbindung der Formel (III): 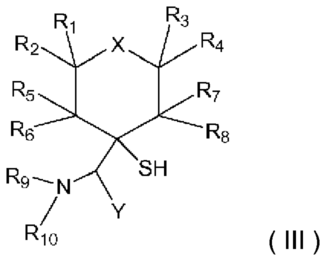 worin R₁, R₂, R₃, R₄, R₅, R₆, R₇, R₈, R₉, R₁₀, Y und X wie in Anspruch 1 definiert sind;
oder Diastereoisomere, Enantiomere, Salze oder Solvate davon.

12. Verbindung der Formel (IV): 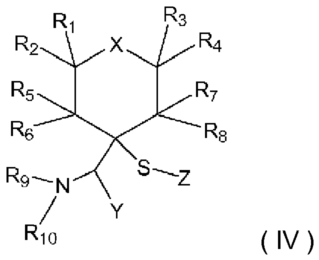 worin R₁, R₂, R₃, R₄, R₅, R₆, R₇, R₈, R₉, R₁₀, Y und X wie in Anspruch 1 definiert sind und Z aus der Gruppe bestehend aus unsubstituiertem Alkyl, substituiertem oder unsubstituiertem Alkenyl, C₇₋₂₀-Aralkyl, -Si(R')₃ und -C(=O)R' ausgewählt ist, worin R' aus der Gruppe bestehend aus substituiertem oder unsubstituiertem Alkyl, substituiertem oder unsubstituiertem Alkenyl, substituiertem oder unsubstituiertem Alkinyl, substituiertem oder unsubstituiertem Aryl und substituiertem oder unsubstituiertem Aralkyl ausgewählt ist;
oder Diastereoisomere, Enantiomere, Salze oder Solvate davon.

13. Verbindung gemäss irgendeinem der Ansprüche 1 bis 7 zur Verwendung als Medikament.

14. Pharmazeutische Zusammensetzung, umfassend eine Verbindung gemäss irgendeinem der Ansprüche 1 bis 7 und einen pharmazeutisch annehmbaren Träger.

15. Verbindung gemäss irgendeinem der Ansprüche 1 bis 7 zur Verwendung bei der Behandlung und/oder Prophylaxe von durch NO vermittelten Zuständen.

16. Verbindung gemäss irgendeinem der Ansprüche 1 bis 7 zur Verwendung bei der Behandlung und/oder Prophylaxe von Zuständen, ausgewählt aus der Gruppe bestehend aus einer Thrombozyten-Funktionsstörung, endokrinen, Stoffwechsel-, kardiovaskulären, entzündlichen, Urogenital-, Verdauungs-, dermatologischen, neuronalen, mit dem Zentralnervensystem verbundenen, neurodegenerativen Erkrankungen, psychischen Störungen, kognitiven Störungen, ophthalmologischen Krankheiten, Atemwegs- und Infektionskrankheiten; Bluthochdruck, Thrombose, thromboembolischen Prozessen, vaskulären oder Trauma-Entzündungen, Komplikationen im Zusammenhang mit Diabetes, Ischämie-Reperfusion, Karotisendarteriektomie und Koronar-Bypass, Abstossungsreaktionen, in der perkutanen Koronarintervention, als Gefässtonusmodulator, Atemnotsyndrom bei Säuglingen, Asthma, Lungenhochdruck, bronchopulmonaler Dysplasie, Mukoviszidose, durch Bakterien, Viren und Protozoen verursachten Erkrankungen, Leishmaniose, Trypanosomiasis, Inhibierung von AIDS-Virus-Replikation, Bewegungserkrankungen des Gastrointestinaltrakts, entzündlicher Darmerkrankung, Achalasie, Erkrankungen der Gallenblase und des Schliessmuskels von Oddi, bei Cholangiopancreotographie, Leberversagen, Leberfibrose, Leberzirrhose, Pfortaderhochdruck, Erkrankungen des Urogenitaltrakts und Erektionsstörungen, Präeklampsie, Endometriumhyperplasie, Proliferation des glatten Gebärmuttermuskels, Myometrium-Tumoren, Hautgeschwüren, Geschwüren im Zusammenhang mit Diabetes, Glaukom, Alzheimer-Krankheit, Schmerz und Fibromyalgie.

## Revendications

1. Composé de formule (I) 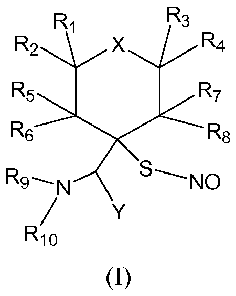 dans laquelle
chacun de R₁, R₂, R₃, R₄, R₅, R₆, R₇ et R₈ est un hydrogène ;
R₉ est sélectionné dans le groupe constitué de l'hydrogène, d'un alkyle en C₁-C₆ et d'un aralkyle en C₇-C₁₅ ;
R₁₀ est sélectionné dans le groupe constitué de l'hydrogène, d'un alkyle en C₁ -C₆, d'un cycloalkyle en C₃-C₈, de -C(=O)R₁₁ et de -S(=O)₂R₁₁, dans lequel R₁₁ est sélectionné dans le groupe constitué de l'hydrogène, d'un alkyle en C₁-C₆, d'un alcényle C₂-C₆, d'un cycloalkyle en C₃-C₈ ; d'un alcynyle en C₂-C₆, d'un aryle en C₆-C₁₀, d'un aralkyle en C₇-C₁₀ et d'un hétérocyclyle à 5 ou 6 chaînons ;
Y représente un radical de formule -C(=O)OR₁₂, dans laquelle R₁₂ est sélectionné dans le groupe constitué d'un alkyle en C₁-C₆, d'un alcényle en C₂-C₆, d'un cycloalkyle en C₃-C₈, d'un alcynyle en C₂-C₆, d'un aryle en C₆-C₁₀, d'un aralkyle en C₇-C₁₀ et d'un hétérocyclyle à 5 ou 6 chaînons ; et
X est sélectionné dans le groupe constitué d'un atome d'oxygène, d'atome de soufre, et de -NR₁₃ ; dans lequel R₁₃ est sélectionné dans le groupe constitué de l'hydrogène, d'un alkyle en C₁-C₆, d'un alcényle en C₂-C₆, d'un cycloalkyle en C₃-C₈, d'un alcynyle en C₂-C₆, d'un aryle en C₆-C₁₀, d'une aralkyle en C₇-C₁₀ et d'un hétérocyclyle à 5 ou 6 chaînons ;
ou ses diastéréoisomères, énantiomères, sels ou les solvates de ceux-ci.

2. - Composé selon la revendication 1, dans lequel X est -NR₁₃, dans lequel R₁₃ est tel que défini dans la revendication 1.

3. - Composé selon l'une quelconque des revendications précédentes, dans lequel R₉ est sélectionné dans le groupe constitué de l'hydrogène et d'un alkyle en C₁-C₆.

4. - Composé selon l'une quelconque des revendications précédentes, dans lequel R₁₂ est un groupe alkyle en C₁-C₆.

5. - Composé selon l'une quelconque des revendications précédentes, dans lequel l'un de R₉ ou R₁₀ est un hydrogène.

6. - Composé selon la revendication 1 sélectionné dans le groupe constitué :
- du (R,S)-acétylamino-(1-méthyl-4-S-nitrosomercapto-pipéridin-4-yl)-acétate d'éthyle ;
- du chlorure de (R,S)-4-(acétylamino-éthoxycarbonyl-méthyl)-1-méthyl-4-S-nitrosomercapto-pipéridinium ;
- du (R,S)-heptanoylamino-(1-méthyl-4-S-nitrosomercapto-pipéridin-4-yl)-acétate d'éthyle ;
- du chlorure de (R,S)-4-(heptanoylamino-éthoxycarbonyl-méthyl)-1-méthyl-4-S-nitrosomercapto-pipéridinium ;
- du (R,S)-benzoylamino-(1-méthyl-4-S-nitrosomercapto-pipéridin-4-yl)-acétate d'éthyle ;
- du chlorure de (R,S)-4-(benzoylamino-éthoxycarbonyl-méthyl)-1-méthyl-4-S-nitrosomercapto-pipéridinium ;
- du (R,S)-(4-chloro-benzoylamino)-(1-méthyl-4-S-nitrosomercapto-pipéridin-4-yl)-acétate d'éthyle ;
- du chlorure de (R,S)-4-[(4-chloro-benzoylamino)-éthoxycarbonyl-méthyl)]-1-méthyl-4-S-nitrosomercapto-pipéridinium ;
- du (R,S)-(2-chloro-benzoylamino)-(1-méthyl-4-S-nitrosomercapto-pipéridin-4-yl)-acétate d'éthyle ;
- du chlorure de (R,S)-4-[(2-chloro-benzoylamino)-éthoxycarbonyl-méthyl)]-1-méthyl-4-S-nitrosomercapto-pipéridinium ;
- du (R,S)-(1-méthyl-4-S-nitrosomercapto-pipéridin-4-yl)-[(pyridin-3-carbonyl)-amino]-acétate d'éthyle ;
- du chlorure de (R,S)-4-{éthoxycarbonyl-[(pyridin-3-carbonyl)-amino]-méthyl}-1-méthyl-4-S-nitrosomercapto-pipéridinium ;
- du (R,S)-benzènesulfonylamino-(1-méthyl-4-S-nitrosomercapto-pipéridin-4-yl)-acétate d'éthyle ;
- du chlorure de (R,S)-4-(benzènesulfonylamino-éthoxycarbonyl-méthyl)-1-méthyl-4-S-nitrosomercapto-pipéridinium ;
- de l'acétate de (R,S)-4-(benzènesulfonylamino-éthoxycarbonyl-méthyl)-1-méthyl-4-S-nitrosomercapto-pipéridinium ;
- du mésylate de (R,S)-4-(benzènesulfonylamino-éthoxycarbonyl-méthyl)-1-méthyl-4-S-nitrosomercapto-pipéridinium ;
- du nitrate de (R,S)-4-(benzènesulfonylamino-éthoxycarbonyl-méthyl)-1-méthyl-4-S-nitrosomercapto-pipéridinium ;
- du sulfate de (R,S)-4-(benzènesulfonylamino-éthoxycarbonyl-méthyl)-1-méthyl-4-S-nitrosomercapto-pipéridinium ;
- du bisulfate de (R,S)-4-(benzènesulfonylamino-éthoxycarbonyl-méthyl)-1-méthyl-4-S-nitrosomercapto-pipéridinium ;
- du phosphate diacide de (R,S)-4-(benzènesulfonylamino-éthoxycarbonylméthyl)-1-méthyl-4-S-nitrosomercapto-pipéridinium ;
- du lactate de (R,S)-4-(benzènesulfonylamino-éthoxycarbonyl-méthyl)-1-méthyl-4-S-nitrosomercapto-pipéridinium ;
- du citrate de (R,S)-4-(benzènesulfonylamino-éthoxycarbonyl-méthyl)-1-méthyl-4-S-nitrosomercapto-pipéridinium ;
- du salicylate de (R,S)-4-(benzènesulfonylamino-éthoxycarbonyl-méthyl)-1-méthyl-4-S-nitrosomercapto-pipéridinium ;
- du (R,S)-(1-méthyl-4-S-nitrosomercapto-pipéridin-4-yl)-(4-nitrobenzènesulfonylamino)-acétate d'éthyle ;
- du (R,S)-(4-méthyl-benzènesulfonylamino)-(1-méthyl-4-S-nitrosomercaptopipéridin-4-yl)-acétate d'éthyle ;
- du (R,S)-(2-chloro-benzènesulfonylamino)-(1-méthyl-4-S-nitrosomercaptopipéridin-4-yl)-acétate d'éthyle ;
- du (R,S)-(3-chloro-benzènesulfonylamino)-(1-méthyl-4-S-nitrosomercaptopipéridin-4-yl)-acétate d'éthyle ;
- du (R,S)-(4-chloro-benzènesulfonylamino)-(1-méthyl-4-S-nitrosomercaptopipéridin-4-yl)-acétate d'éthyle ;
- du chlorure de (R,S)-4-(4-chloro-benzènesulfonylamino-éthoxycarbonylméthyl)-1-méthyl-4-S-nitrosomercapto-pipéridinium ;
- du chlorure de (R,S)-4-(2-fluoro-benzènesulfonylamino-éthoxycarbonylméthyl)-1-méthyl-4-S-nitrosomercapto-pipéridinium ;
- du (R,S)-(4-fluoro-benzènesulfonylamino)-(1-méthyl-4-S-nitrosomercaptopipéridin-4-yl)-acétate d'éthyle ;
- du chlorure de (R,S)-4-(4-fluoro-benzènesulfonylamino-éthoxycarbonylméthyl)-1-méthyl-4-S-nitrosomercapto-pipéridinium ;
- du (R,S)-(2,4-difluoro-benzènesulfonylamino)-(1-méthyl-4-S-nitrosomercaptopipéridin-4-yl)-acétate d'éthyle ;
- du chlorure de (R,S)-4-(2,4-difluoro-benzènesulfonylamino-éthoxycarbonylméthyl)-1-méthyl-4-S-nitrosomercapto-pipéridinium ;
- du (R,S)-formylamino-(1-méthyl-4-S-nitrosomercapto-pipéridin-4-yl)-acétate d'éthyle ;
- du chlorure de (R,S)-4-(éthoxycarbonyl-formylamino-méthyl)-1-méthyl-4-S-nitrosomercapto-pipéridinium ;
- du (R,S)-(1-méthyl-4-S-nitrosomercapto-pipéridin-4-yl)-(2,2,2-trifluoroacétylamino)-acétate d'éthyle ;
- du (R,S)-acétylamino-(1-méthyl-4-S-nitrosomercapto-pipéridin-4-yl)-acétate de benzyle ;
- du (R,S)-(acétyl-éthyl-amino)-(1-méthyl-4-S-nitrosomercapto-pipéridin-4-yl)-acétate d'éthyle ;
- du (R,S)-acétylamino-(1-éthyl-4-S-nitrosomercapto-pipéridin-4-yl)-acétate d'éthyle ;
- du chlorure de (R,S)-4-(acétylamino-éthyloxycarbonyl-méthyl)-1-éthyl-4-S-nitrosomercapto-pipéridinium ;
- du (R,S)-(1-éthyl-4-S-nitrosomercapto-pipéridin-4-yl)-[(pyridin-3-carbonyl)-amino]-acétate d'éthyle ;
- du chlorure de (R,S)-1-éthyl-4-{éthoxycarbonyl-[(pyridin-3-carbonyl)-amino]-méthyl}-4-S-nitrosomercapto-pipéridinium ;
- du (R,S)-acétylamino-(1-benzyl-4-S-nitrosomercapto-pipéridin-4-yl)-acétate d'éthyle ;
- du chlorure de (R,S)-4-(acétylamino-éthyloxycarbonyl-méthyl)-1-benzyl-4-S-nitrosomercapto-pipéridinium ;
- du (R,S)-(1-benzyl-4-S-nitrosomercapto-pipéridin-4-yl)-[(pyridin-3-carbonyl)-amino]-acétate d'éthyle ;
- du chlorure de (R,S)-1-benzyl-4-{éthoxycarbonyl-[(pyridin-3-carbonyl)-amino]-méthyl}-4-S-nitrosomercapto-pipéridinium ;
- du (R,S)-(1-éthyl-4-S-nitrosomercapto-pipéridin-4-yl)-formylamino-acétate d'éthyle ;
- du chlorure de (R,S)-1-éthyl-4-(éthyloxycarbonyl-formylamino-méthyl)-4-S-nitrosomercapto-pipéridinium ;
- du (R,S)-(1-benzyl-4-S-nitrosomercapto-pipéridin-4-yl)-formylamino-acétate d'éthyle ;
- du chlorure de (R,S)-1-benzyl-4-(éthyloxycarbonyl-formylamino-méthyl)-4-S-nitrosomercapto-pipéridinium ;
- du (R,S)-acétylamino-(1-méthyl-4-S-nitrosomercapto-pipéridin-4-yl)-acétate de méthyle ;
- du chlorure de (R,S)-4-(acétylamino-méthyloxycarbonyl-méthyl)-1-méthyl-4-S-nitrosomercapto-pipéridinium ;
- du (R,S)-(1-méthyl-4-S-nitrosomercapto-pipéridin-4-yl)-[(pyridin-3-carbonyl)-amino]-acétate de méthyle ;
- du (R,S)-benzènesulfonylamino-(1-méthyl-4-S-nitrosomercapto-pipéridin-4-yl)-acétate de méthyle ;
- du chlorure de (R,S)-4-(benzènesulfonylamino-méthoxycarbonyl-méthyl)-1-méthyl-4-S-nitrosomercapto-pipéridinium ;
- du (R,S)-formylamino-(1-méthyl-4-S-nitrosomercapto-pipéridin-4-yl)-acétate de méthyle ;
- du (R,S)-acétylamino-(4-nitrosomercapto-tetrahydro-thiopyran-4-yl)-acétate d'éthyle ; et
- du (R,S)-benzènesulfonylamino-(4-nitrosomercapto-tetrahydro-thiopyran-4-yl)-acétate d'éthyle ;
ou les énantiomères ou les solvates de ceux-ci.

7. - Composé selon la revendication 6 sélectionné dans le groupe constitué :
- du (R)-benzènesulfonylamino-(1-méthyl-4-S-nitrosomercapto-pipéridin-4-yl)-acétate d'éthyle ;
- du (S)-benzènesulfonylamino-(1-méthyl-4-S-nitrosomercapto-pipéridin-4-yl)-acétate d'éthyle ;
- du chlorure de (S)-4-(benzènesulfonylamino-éthoxycarbonyl-méthyl)-1-méthyl-4-S-nitrosomercapto-pipéridinium ; et
- du chlorure de (R)-4-(benzènesulfonylamino-éthoxycarbonyl-méthyl)-1-méthyl-4-S-nitrosomercapto-pipéridinium.

8. - Procédé de synthèse d'un composé de formule (I) tel que défini dans l'une quelconque des revendications 1 à 7 comprenant la nitrosation de la fonctionnalité thiol d'un composé de formule (III) 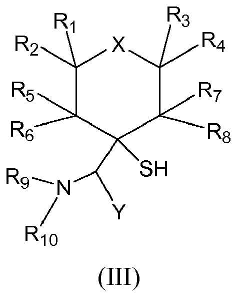 dans laquelle
R₁, R₂, R₃, R₄, R₅, R₆, R₇, R₈, R₉, R₁₀, Y et X sont tels que défini dans la revendication 1.

9. - Procédé selon la revendication 8 comprenant les étapes suivantes :
a) la suppression du groupe Z dans un composé de formule (IV) 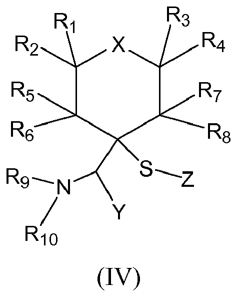 dans laquelle
R₁, R₂, R₃, R₄, R₅, R₆, R₇, R₈, R₉, R₁₀, Y et X sont tels que défini dans la revendication 1, et Z est sélectionné dans le groupe constitué d'un alkyle non substitué, d'un alcényle, d'un aralkyle en C₇-C₂₀, -Si(R')₃, - C(=O)R', dans lequel R' est sélectionné dans le groupe constitué d'un alkyle, d'un alcényle, d'un alcynyle, d'un aryle et d'un aralkyle ;
pour obtenir un composé de formule (III) tel que défini dans la revendication 8 ; et
b) la nitrosation de la fonctionnalité thiol dudit composé de formule (III) obtenu à l'étape a).

10. - Procédé selon l'une quelconque des revendications 8 et 9, dans lequel au moins un des composés de formule (III) et/ou de formule (IV) sont soumis à une séparation chirale.

11. - Composé de formule (III) 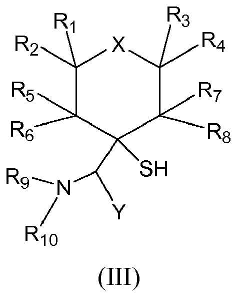 dans laquelle
R₁, R₂, R₃, R₄, R₅, R₆, R₇, R₈, R₉, R₁₀, Y et X sont tels que défini dans la revendication 1 ;
ou ses diastéréoisomères, énantiomères, sels ou les solvates de ceux-ci.

12. - Composé de formule (IV) 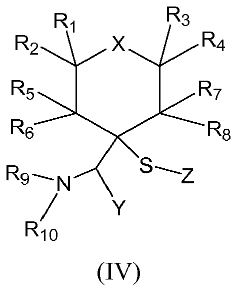 dans laquelle
R₁, R₂, R₃, R₄, R₅, R₆, R₇, R₈, R₉, R₁₀, Y et X sont tels que défini dans la revendication 1, et Z est sélectionné dans le groupe constitué d'un alkyle non substitué, d'un alcényle substitué ou non substitué, d'un aralkyle en C₇-C₂₀, de - Si(R')₃, de -C(=O)R', dans lequel R' est sélectionné dans le groupe constitué d'un alkyle substitué ou non substitué, d'un alcényle substitué ou non substitué, d'un alcynyle substitué ou non substitué, d'un aryle substitué ou non substitué et un aralkyle substitué ou non substitué ;
ou ses diastéréoisomères, énantiomères, sels ou les solvates de ceux-ci.

13. - Composé tel que défini dans l'une quelconque des revendications 1 à 7 destiné à être utilisé en tant que médicament.

14. - Composition pharmaceutique comprenant un composé tel que défini dans l'une quelconque des revendications 1 à 7 et un vecteur pharmaceutiquement acceptable.

15. - Composé tel que défini dans l'une quelconque des revendications 1 à 7 destiné à être utilisé dans le traitement et/ou la prophylaxie d'affections médiées par l'NO.

16. - Composé tel que défini dans l'une quelconque des revendications 1 à 7 destiné à être utilisé dans le traitement et/ou la prophylaxie d'une affection sélectionnée dans le groupe constitué de la dysfonction plaquettaire, les maladies endocrines, métaboliques, cardiovasculaires, inflammatoires, génito-urinaires, digestives, dermatologiques, neuronales, associées au système nerveux central, neurodégénératives, les troubles mentaux, les troubles cognitifs, la maladie ophtalmologique, les affections respiratoires et infectieuses ; l'hypertension, la thrombose, les processus thromboemboliques, une inflammation vasculaire ou faisant suite à un traumatisme, les complications associées au diabète, à une ischémie-reperfusion, à une endartérectomie carotididienne et à un pontage coronarien, aux rejets de greffe, dans les interventions coronariennes percutanées, en tant que modulateur du tonus vasculaire, le syndrome de détresse respiratoire du nouveau-né, l'asthme, l'hypertension pulmonaire, la dysplasie bronchopulmonaire, la fibrose kystique, les maladies infectieuses provoquées par les bactéries, les virus et les protozoaires, la leishmaniose, la trypanosomiase, l'inhibition de la réplication du virus du SIDA, les maladies motrices du tube digestif, la maladie intestinale inflammatoire, l'achalasie, les maladies de la vésicule biliaire et du sphincter d'Oddi, dans la cholangiopancréaotographie, l'insuffisance hépatique, la fibrose hépatique, la cirrhose du foie, l'hypertension portale, les maladies de l'appareil génito-urinaire et la dysfonction érectile, la pré-éclampsie, l'hyperplasie de l'endomètre, la prolifération des muscles lisses de l'utérus, les tumeurs du myomètre, les ulcères cutanés, les ulcères associés au diabète, le glaucome, la maladie d'Alzheimer, la douleur et les fibromyalgies.
